# EUROPEAN PATENT APPLICATION

(11) **EP 1 283 255 A1**
(43) Date of publication of application: **12.02.2003**
(21) Application number: 01926026.4
(22) Date of filing: 27.04.2001
(51) Int. Cl.: C12N 1/15, C12N 1/19, C12N 5/00, C12N 7/00, C12N 15/00, C12P 21/02, C12Q 1/68, C07K 14/47, C07K 16/18

(54) **MYOCARDIAL CELL PROLIFERATION-ASSOCIATED GENES**

(30) Priority: 27.04.2000 JP 2000126741
(71) Applicant: KYOWA HAKKO KOGYO CO., LTD., Chiyoda-ku, Tokyo 100-8185 (JP)
(72) Inventor: YAMADA, Yoji, Machida-shi, Tokyo 194-8533 (JP); SEKINE, Susumu, Machida-shi, Tokyo 194-8533 (JP); KIKUCHI, Yasuhiro, Kyowa Hakko Kogyo Co. Ltd., Tokyo 100-8185 (JP); SAKURADA, Kazuhiro, Machida-shi, Tokyo 194-8533 (JP)
(74) Representative: Grünecker, Kinkeldey, Stockmair & Schwanhäusser Anwaltssozietät
(86) International application number: JP0103700
(87) International publication number: WO01083705

(57) **Abstract**

Genes showing different expression levels between fetal heart and adult heart were obtained. Thus, proteins useful in searching for therapeutic agents for repairing tissues injured by myocardial degeneration, DNAs encoding these proteins, antibodies recognizing these proteins, and methods of using the same are provided.

## Description

### Technical Field

The present invention relates to DNAs (e.g., cDNAs) that are complementary to mRNAs whose expression levels vary between fetal heart and adult heart, and which were obtained by subtraction and differential hybridization, as well as proteins encoded by the DNAs. The present invention also relates to antibodies against the proteins, methods for detecting the proteins and DNAs, and diagnostic and therapeutic agents, which comprise such DNAs, proteins, or antibodies, for various heart diseases caused by myocardial degeneration, such as hypercardia and cardiac failure.

### Background Art

The heart is differentiated earliest among organs, at a very early stage of ontogeny. Immediately after differentiation, the heart starts to spontaneous beat. Even after differentiation, the myocardial cell maintains its proliferation potential and actively divides and proliferates during the fetal period. Specifically, a feature of the myocardial cell during this period is the occurrence of mitotic division despite the presence of many contraction filament bundles in the cytoplasm.

Most other somatic cells lose their division potential after the formation of specific cytoplasmic structures through differentiation. However, this general rule does not apply to myocardial cells.

After birth, the proliferation potential of myocardial cell decreases rapidly. Thus, the growth of heart is achieved by physiological auxesis, wherein the volume of individual myocardial cells increases. It is considered that postnatal myocardial cells have no ability to regenerate.

When myocardial cells necrose due to cardiac infarction, myocarditis, aging, etc., remaining myocardial cells adapt themselves to the situation not by cell division but by cell auxesis. Cardiomegaly occurring immediately after birth is a physiological adaptation; conversely, cardiomegaly occurring after necrosis of myocardial cells is combined with hyperplasia of coexisting cardiac fibroblast cells and interstitial fibrosis, and results in impaired diastolic function of the heart followed by impaired systolic function, which ultimately leads to cardiac failure. Symptomatic treatments, such as that reducing blood pressure and load of the volume, using agents enhancing cardiac contractile force and vasodilators, and that reducing blood volume using diuretics have been conducted as methods to treat cardiac failure caused by cardiac infarction and such. Prognosis is unfavorable in serious cardiac failure, and the heart transplantation is the only radical therapy. However, there are problems associated with transplant, including the shortage of organ donors, difficulty of brain death diagnosis, rejection, rising medical cost and such. Thus, heart transplantation has not established as a general therapy.

Cardiac failure may be treated or prevented by conferring proliferation potential to adult myocardial cells. The molecular mechanism associated with the loss of proliferation capacity of myocardial cells after birth remains to be clarified. However, molecules that are highly expressed in fetal or adult myocardial cells are presumed to be associated with the suppression of proliferation of myocardial cells, due to the different properties between fetus and adult.

Proteins whose expression levels differ between fetal heart and adult heart, include the following:

Proteins known to be more highly expressed in fetal heart than in adult heart include PCNA (proliferating cell nuclear antigen), Rb (retinoblastoma), Cyc (cyclin) D1, CycD3, and Cdk (cyclin D-dependent kinase) 4, which are involved in DNA replication or cell cycle (Am. J. Physiol., 271, H2183-H2189 (1996)). On the other hand, proteins known to be more highly expressed in adult heart than in fetal heart include Gax (Growth arrest-specific homeobox) (Am. J. Physiol., 271, H2183-H2189 (1996)).

However, for example, in spite of the fact that multiple nuclei were observed in adult myocardial cell of transgenic mice wherein forced expression of cyclin D1 was induced in a myocardial cell-specific manner, expression of only adult-specific contractile proteins was detected and no marked increase of the cell count was observed (J. Clinical Investigation, 99, 2644-2654 (1997)). Finally, proliferation potential has not yet successfully been conferred to adult myocardial cells using those proteins alone.

Accordingly, identification of new factors whose expression levels differ between fetal heart and adult heart and that may be associated with myocardial cell proliferation is required.

### Disclosure of the Invention

The elucidation of the molecular mechanism for the postnatal loss of proliferation potential of myocardial cells, which possess proliferation potential during fetal period, may clarify the onset mechanism and therapeutic targets of various heart diseases caused by myocardial necrosis, and may further enable the development of a method for regenerating myocardial cells. The objectives of the present invention are: to obtain genes whose expression levels differ between fetal heart and adult heart; and to provide proteins useful in screening therapeutic agents capable of healing tissues damaged by myocardial necrosis, DNAs encoding the proteins, and antibodies recognizing the proteins, as well as uses thereof.

The present inventors persistently researched to achieve the above-mentioned objectives, and obtained following results. The inventors constructed a subtracted library enriched with genes highly expressed in the fetal heart by subtracting mRNAs extracted from the heart of an 8-week-old rat from a cDNA library constructed using, as a template, mRNAs derived from the heart of a 16-day-old fetal rat. During the construction of the subtracted library, genes whose expression levels are low are equalized and the population of vectors without insert fragment increases. Therefore, differential hybridization for each clone in the subtracted library was carried out to obtain many clones of genes whose expression levels differ between fetal heart and adult heart. The resulting clones comprised known genes whose expression levels had not previously been known to differ between fetal heart and adult heart, and novel genes as well as genes whose expression levels had been known to differ between fetal heart and adult heart. The difference of the gene expression levels was verified for these genes by Northern hybridization. Further, the present inventors identified peptides encoded by the genes and completed the present invention.

Hereinafter, a gene whose expression level differs between fetal heart and adult heart is referred to as a myocardial cell proliferation-associated gene.

The present invention provides:
(1) a DNA comprising the nucleotide sequence selected from the group consisting of the nucleotide sequences represented by SEQ ID NOs: 21, 23, 25, 27, and 30;
(2) a DNA of a gene that hybridizes, under stringent conditions, to a DNA consisting of the nucleotide sequence represented by SEQ ID NO: 21 or 27, and whose expression level varies between fetal heart and adult heart;
(3) a DNA of a gene that hybridizes under stringent conditions to a DNA consisting of the nucleotide sequence represented by SEQ ID NO: 23, 25 or 30, having a 90% or higher homology to the DNA, and whose expression level differs between fetal heart and adult heart;
(4) a DNA comprising a sequence that is identical to 5 to 60 consecutive nucleotide residues of the nucleotide sequence selected from the group consisting of the nucleotide sequences represented by SEQ ID NOs: 21, 23, 25, 27 and 30;
(5) a DNA comprising a sequence complementary to the DNA according to (4);
(6) a method for detecting mRNA corresponding to a gene whose expression level varies between fetal heart and adult heart using the DNA according to any one of (1) to (5);
(7) a diagnostic agent for heart diseases caused by myocardial degeneration, which agent comprises the DNA according to any one of (1) to (5);
(8) a method for detecting a causative gene of a heart disease caused by myocardial degeneration using the DNA according to any one of (1) to (5);
(9) a method of screening for a substance suppressing or enhancing transcription or translation of a gene whose expression level varies between fetal heart and adult heart using the DNA according to any one of (1) to (5);
(10) a method of screening for a therapeutic agent of a heart disease caused by myocardial degeneration using the DNA according to any one of (1) to (5);
(11) a therapeutic agent for a heart disease caused by myocardial degeneration, wherein the agent comprises the DNA according to any one of (1) to (5);
(12) a recombinant viral vector comprising the DNA according to any one of (1) to (5);
(13) a recombinant viral vector comprising an RNA having a sequence homologous to the sense strand of the DNA according to any one of (1) to (5);
(14) a DNA having the nucleotide sequence selected from the group consisting of the nucleotide sequences represented by SEQ ID NOs: 19, 32, and 37;
(15) a DNA of a gene hybridizing under stringent conditions to the DNA according to (14), and whose expression level varies between fetal heart and adult heart;
(16) a DNA comprising a sequence that is identical to 5 to 60 consecutive nucleotide residues of the nucleotide sequence selected from the group consisting of the nucleotide sequences represented by SEQ ID NOs: 19, 32, and 37;
(17) a DNA comprising a sequence complementary to the DNA of (16);
(18) a diagnostic agent for a heart disease caused by myocardial degeneration, wherein the agent comprises the DNA according to any one of (14) to (16);
(19) a method for detecting a causative gene of a heart disease caused by myocardial degeneration using the DNA according to any one of (14) to (16);
(20) a method of screening for a substance suppressing or enhancing transcription or translation of a gene whose expression level varies between fetal heart and adult heart using the DNA according to any one of (14) to (16);
(21) a method of screening for a therapeutic agent of a heart disease caused by myocardial degeneration using the DNA according to any one of (14) to (16);
(22) a method for detecting mRNA corresponding to a gene whose expression level varies between fetal heart and adult heart using a DNA having the nucleotide sequence selected from the group consisting of the nucleotide sequences represented by SEQ ID NOs: 1, 3, 5, 7, 9, 11, 13, 15, 17, 33, and 35;
(23) a diagnostic agent for a heart disease caused by myocardial degeneration, wherein the agent comprises a DNA comprising the nucleotide sequence selected from the group consisting of the nucleotide sequences represented by SEQ ID NOs : 1, 3, 5, 7, 9, 11, 13, 15, 17, 33, and 35;
(24) a method of screening for a substance suppressing or enhancing transcription or translation of a gene whose expression level varies between fetal heart and adult heart using a DNA comprising the nucleotide sequence selected from the group consisting of the nucleotide sequences represented by SEQ ID NOs: 1, 3, 5, 7, 9, 11, 13, 15, 17, 33, and 35;
(25) a method of screening for a therapeutic agent of a heart disease caused by myocardial degeneration using a DNA comprising the nucleotide sequence selected from the group consisting of the nucleotide sequences represented by SEQ ID NOs: 1, 3, 5, 7, 9, 11, 13, 15, 17, 33, and 35;
(26) a therapeutic agent for a heart disease caused by myocardial degeneration, wherein the agent comprises a DNA comprising the nucleotide sequence selected from the group consisting of the nucleotide sequences represented by SEQ ID NOs: 1, 3, 5, 7, 9, 11, 13, 15, 17, 33, and 35;
(27) a recombinant viral vector comprising a DNA having the nucleotide sequence selected from the group consisting of the nucleotide sequences represented by SEQ ID NOs: 1, 3, 5, 7, 9, 11, 13, 15, 17, 33, and 35;
(28) a recombinant viral vector comprising an RNA having a sequence homologous to the sense strand of a DNA comprising the nucleotide sequence selected from the group consisting of the nucleotide sequences represented by SEQ ID NOs: 1, 3, 5, 7, 9, 11, 13, 15, 17, 33, and 35;
(29) a protein comprising the amino acid sequence selected from the group consisting of the amino acid sequences represented by SEQ ID NOs: 22, 24, 26, 28, and 31;
(30) a protein having an amino acid sequence wherein one or more amino acids are deleted, substituted or added in the amino acid sequence selected from the group consisting of the amino acid sequences represented by SEQ ID NOs: 22, 24, 26, and 28, and which has an activity related to the healing of a heart disease caused by myocardial degeneration;
(31) a DNA encoding the protein according to (29) or (30);
(32) a recombinant DNA that is obtained by inserting the DNA according to any one of (1) to (4), and (31) into a vector;
(33) a transformant obtained by introducing the recombinant DNA according to (32) into a host cell;
(34) a method for producing the protein, comprising the steps of culturing the transformant according to (33), producing and accumulating the protein according to (29) or (30) in the culture, and recovering the protein from the culture;
(35) a therapeutic agent for a heart disease caused by myocardial degeneration, which agent comprises the protein according to (29) or (30);
(36) a method of screening for a therapeutic agent for a heart disease caused by myocardial degeneration comprising the steps of culturing the transformant according to (33), and screening the agent using the obtained culture;
(37) a method of screening for a therapeutic agent for a heart disease caused by myocardial degeneration using the protein according to (29) or (30);
(38) a recombinant viral vector associated with the production of the protein according to (29) or (30);
(39) a therapeutic agent for a heart disease caused by myocardial degeneration, wherein the agent comprises the recombinant viral vector according to (38);
(40) an antibody recognizing the protein according to (29) or (30);
(41) an immunological method for detecting the protein of (29) or (30) using the antibody according to (40);
(42) a method of screening for a therapeutic agent for a heart disease caused by myocardial degeneration using the antibody according to (40);
(43) a method of screening for a substance suppressing or enhancing transcription or translation of a gene whose expression level varies between fetal heart and adult heart using the antibody according to (40);
(44) a diagnostic agent for a heart disease caused by myocardial degeneration, wherein the agent comprises the antibody according to (40);
(45) a therapeutic agent for a heart disease caused by myocardial degeneration, wherein the agent comprises the antibody according to (40);
(46) a drug delivery method for delivering to a cardiac lesion a fusion antibody in which the antibody according to (40) is bound to an agent selected from the group consisting of a radioisotope, a protein, and a low-molecular-weight compound;
(47) an antibody recognizing a protein comprising the amino acid sequence represented by SEQ ID NO: 20 or 38;
(48) a method of screening for a therapeutic agent for a heart disease caused by myocardial degeneration using the antibody according to (47);
(49) a method of screening for a substance suppressing transcription or translation of a gene whose expression level varies between fetal heart and adult heart using the antibody according to (47);
(50) a diagnostic agent for a heart disease caused by myocardial degeneration, wherein the agent comprises the antibody according to (47);
(51) a therapeutic agent for a heart disease caused by myocardial degeneration, wherein the agent comprises the antibody according to (47);
(52) a drug delivery method for delivering to a cardiac lesion a fusion antibody in which the antibody according to (47) is bound to an agent selected from the group consisting of a radioisotope, a protein, and a low-molecular-weight compound;
(53) a recombinant viral vector associated with the production of a protein comprising the amino acid sequence selected from the group consisting of the amino acid sequences represented by SEQ ID NOs: 2, 4, 6, 8, 10, 12, 14, 16, 18, 34, and 36;
(54) a therapeutic agent for a heart disease caused by myocardial degeneration, which agent comprises the recombinant viral vector according to (53);
(55) a method of screening for a therapeutic agent for a heart disease caused by myocardial degeneration using an antibody that recognizes a protein comprising the amino acid sequence selected from the group consisting of the amino acid sequences represented by SEQ ID NOs: 2, 4, 6, 8, 10, 12, 14, 16, 18, 34, and 36;
(56) a method of screening for a substance suppressing or enhancing transcription or translation of a gene whose expression level varies between fetal heart and adult heart using an antibody that recognizes a protein comprising the amino acid sequence selected from the group consisting of the amino acid sequences represented by SEQ ID NOs: 2, 4, 6, 8, 10, 12, 14, 16, 18, 34, and 36;
(57) a diagnostic agent for a heart disease caused by myocardial degeneration, wherein the agent comprises an antibody that recognizes a protein comprising the amino acid sequence selected from the group consisting of the amino acid sequences represented by SEQ ID NOs: 2, 4, 6, 8, 10, 12, 14, 16, 18, 34, and 36;
(58) a therapeutic agent for a heart disease caused by myocardial degeneration, wherein the agent comprises an antibody that recognizes a protein comprising the amino acid sequence selected from the group consisting of the amino acid sequences of SEQ ID NOs: 2, 4, 6, 8, 10, 12, 14, 16, 18, 34, and 36; and
(59) a drug delivery method for delivering to a cardiac lesion a fusion antibody in which an antibody recognizing a protein comprising the amino acid sequence selected from the group consisting of the amino acid sequences represented by SEQ ID NOs: 2, 4, 6, 8, 10, 12, 14, 16, 18, 34, and 36, is bound to an agent selected from the group consisting of a radioisotope, a protein, and a low-molecular-weight compound.

The present invention is described below in detail.

The DNAs of the present invention are DNAs of genes whose expression levels vary between fetal heart and adult heart, and include, for example, a DNA having the nucleotide sequence selected from the group consisting of the nucleotide sequences represented by SEQ ID NOs: 21, 23, 25, 27, and 30; and a DNA hybridizing, under stringent conditions, to any of the above DNA and whose expression level varies between fetal heart and adult heart.

The above-mentioned DNA that hybridizes to the nucleotide sequence selected from the group consisting of the nucleotide sequences represented by SEQ ID NOs: 21, 23, 25, 27, and 30, under stringent conditions, refers to DNAs that can be obtained by colony hybridization, plaque hybridization, Southern blot hybridization or the like using, as a probe, a DNA having the nucleotide sequence selected from the group consisting of the nucleotide sequences of SEQ ID NOs: 21, 23, 25, 27, and 30. Specifically, such DNAs include DNAs that can be identified by immobilizing DNAs derived from bacterial colony or phage plaque on a filter, carrying out hybridization with a labeled-DNA probe in the presence of 0.7 to 1.0 M sodium chloride at 65°C, and washing the filter with a solution of 0.1 to 2 x SSC (1 x SSC solution contains 150 mM sodium chloride and 15 mM sodium citrate) at 65°C.

The hybridization can be performed according to the methods described in "Molecular Cloning, A Laboratory Manual, Second Edition, Cold Spring Harbor Laboratory Press (1989)" (hereinafter abbreviated as "Molecular Cloning 2nd Ed."), "Current Protocols in Molecular Biology, John Wiley & Sons (1987-1997)" (hereinafter abbreviated as "Current Protocols in Molecular Biology"), "DNA cloning 1: Core Techniques, A Practical Approach, Second Edition, Oxford University (1995)," etc. Specifically, the hybridizable DNA includes a DNA having at least 60% or higher homology, preferably 80% or higher homology, more preferably 90% or higher homology to the nucleotide sequence selected from the group consisting of the nucleotide sequences of SEQ ID NOs: 21, 23, 25, 27, and 30.

Further, the DNAs of the present invention also include oligonucleotides and antisense oligonucleotides having a partial nucleotide sequence of DNAs of the present invention. Said oligonucleotides include, for example, oligonucleotides having the same sequence as the nucleotide sequence of 5 to 60 consecutive residues, preferably 10 to 40 consecutive residues of the nucleotide sequence selected from the group consisting of the nucleotide sequences of SEQ ID NOs: 21, 23, 25, 27, and 30. And such antisense oligonucleotides include, for example, antisense oligonucleotides complementary to the oligonucleotides.

The proteins of the present invention include proteins having an activity associated with heart diseases caused by myocardial degeneration. Specifically, said, proteins include proteins having the amino acid sequence selected from the group consisting of the amino acid sequences of SEQ ID NOs: 22, 24, 26, 28, and 31; and proteins having an amino acid sequence wherein one or more amino acids are deleted, substituted, or added in the amino acid sequence selected from the group consisting of the amino acid sequences of SEQ ID NOs: 22, 24, 26, and 28, and having an activity associated with the healing of heart diseases caused by myocardial degeneration.

The proteins, having an amino acid sequence wherein one or several amino acids are deleted, substituted, or added in the amino acid sequence selected from the group consisting of the amino acid sequences of SEQ ID NOs: 22, 24, 26, and 28, and having an activity associated with the healing of heart diseases caused by myocardial degeneration, can be prepared following the methods described in Molecular Cloning 2nd Ed.; Current Protocols in Molecular Biology; Nucleic Acids Research, 10, 6487 (1982); Proc. Natl. Acad. Sci., USA, 79, 6409 (1982); Gene, 34, 315 (1985); Nucleic Acids Research, 13, 4431 (1985); Proc. Natl. Acad. Sci., USA, 82, 488 (1985), etc.

### 1. Preparation of myocardial cell proliferation-associated genes

### (1) Preparation of subtracted cDNA library from rat heart and selection of cDNAs from the library by differential hybridization:

DNAs of myocardial cell proliferation-associated genes are prepared as follows:

First, a cDNA library is prepared from the heart of 16-day-old fetal rat by subtracting mRNAs from the heart of 8-week-old rat. Then, differential hybridization is carried out for cDNA clones of the subtracted cDNA library using RNAs from the heart of either the a 16-day-old fetal rat or an 8-week-old rat as probes. Myocardial cell proliferation-associated genes can be obtained by selecting cDNA clones whose expression levels vary between the heart of the 16-day-old fetal rat and that of the 8-week-old rat.

Subtraction is a method for selecting cDNAs of genes whose expression level vary in a control group, wherein single-stranded cDNAs are prepared from mRNAs that are extracted from tissues or cells of a certain state, the cDNAs are hybridized to mRNAs from cells of the control group, and cDNAs hybridizing to the mRNAs are subtracted.

### (1)-1. Preparation of subtracted cDNA library

There are several methods for preparing subtracted cDNA library. In the present invention, a method wherein, first, a cDNA library is prepared by an usual method from the heart of a 16-day-old fetal rat, and the cDNAs are converted into single-stranded DNAs using helper phage, followed by subtraction (Proc. Natl. Acad. Sci. USA, 88, 825 (1991)) was used. The subtraction is performed by a method wherein the cDNAs are hybridized to biotinylated mRNAs from the heart of an 8-week-old rat, streptavidin is bound to the hybridized biotinylated mRNA-cDNA complex, and are extracted with phenol.

### (1)-1-A. Preparation of cDNA library from the heart of a 16-day-old fetal rat

The guanidine thiocyanate-cesium trifluoroacetate method (Methods in Enzymol., 154, 3 (1987) can be exemplified as a method for preparing total RNA from rat heart.

An mRNA generally contains a poly (A) tail at the 3' end. Thus, a poly (A) + RNA can be prepared from total RNA by methods such as the oligo (dT)-immobilized cellulose column method (Molecular Cloning, 2nd Ed.).

Alternatively, mRNA can be also prepared using a kit, such as the Fast Track mRNA Isolation Kit (Invitrogen), the Quick Prep mRNA Purification Kit (Amersham Pharmacia Biotech), or the like.

Methods for constructing a cDNA library from mRNA include those described in Molecular Cloning 2nd Ed.; Current Protocols in Molecular Biology; DNA cloning 1: Core Techniques, A Practical Approach, Second Edition, Oxford University Press (1995); etc. Methods using commercially available kits, such as Superscript Plasmid System for cDNA Synthesis and Plasmid Cloning (Life Technologies) , and ZAP-cDNA Synthesis Kit (Stratagene), are also included.

Cloning vectors include vectors that can replicate in *E. coli* to a high copy number, which have marker genes for transformation, such as the ampicillin resistance gene and kanamycin resistance gene, as well as a multi-cloning site for cDNA insertion, and which can be converted to a single-stranded DNA by a simple method. Said cloning vectors include phagemid vectors that contain a replication signal IG (intergenic space) derived from an M13 phage, and which are plasmids that can be converted to single-stranded DNA phages by helper phage infection. Specifically, said vectors include pBluescript SK(-), pBluescriptII KS (+), pBS (-) , pBC(+) (all of them are from Stratagene) ; pUC118 (TaKaRa Shuzo), etc. The cloning vectors also include λ phage vectors that can be converted to phagemids by *in vivo* excision using helper phages. Specific examples are: λ ZAPII, ZAP Express (both are from Stratagene) , etc. The above-mentioned *in vivo* excision, method for converting to a single-stranded DNA phage, and method for purifying single-stranded DNA from phage in the culture supernatant can be performed according to the directions set forth in the manual provided with the respective commercially available vectors.

Any *E. coli* can be used to introduce a vector containing an insert cDNA, so long as it can express the introduced gene. Specifically, such *E. coli* includes *Escherlchia coli* XL1-Blue MRF' (Stratagene; Strategies, 5, 81 (1992)), *Escherichia coli* C600 (Genetics, 39, 440 (1954)), *Escherichia coli* Y1088 (Science, 222, 778 (1983)), *Escherichia coli* Y1090 (Science, 222, 778 (1983)), *Escherichia coli* NM522 (J. Mol. Biol., 166, 1 (1983)), *Escherichia coli* K802 (J. Mol. Biol., 16, 118 (1966)) *Escherichia coli* JM105 (Gene, 38, 275 (1985)), etc.

In the subtraction, cDNAs are used for the hybridization with mRNAs from the heart of an 8-week-old rat, and the type of phagemid determines which of the two strands of the phagemid is converted to a single-stranded DNA. Thus, to prepare a cDNA library, the procedure of cDNA preparation and the orientation of the insert DNA in the vector have to be designed so that every cDNA clone generates an antisense strand (the strand having the nucleotide sequence complementary to the actual mRNA) as the single-stranded DNA.

For example, as described in the manual of ZAP cDNA synthesis kit from Stratagene, cDNA synthesis with reverse transcriptase is performed using an oligo (dT) primer having an *Xho*I site at the 5' end and dNTP containing 5-methyl dCTP, instead of dCTP, as a substrate (which prohibits the synthesized cDNA from internal digestion with *Xho*I). *Eco*RI adapters are added to each end of the synthesized cDNA, and then, the resulting DNA is digested with *Xho*I. The digested cDNA is inserted into the *Eco*RI/*Xho*I site of vector λZAPII. According to this method, the *Eco*RI site of the cDNA always corresponds to the 5' end and the *Xho*I site to the 3' end to place the insert in a fixed orientation within the vector.

The cDNA library obtained by the above described method is converted to phagemid vector pBluescript SK(-) by *in vivo* excision. Then, single-stranded DNA comprising an antisense strand of the cDNA can be provided by infecting helper phage to the phagemid.

### (1)-1-B. Subtraction using mRNA from the heart of an 8-week-old rat

Using the cDNA library of the phagemid vector prepared in (1)-1-A, single-stranded DNA phages are released into the culture medium via helper phage infection. The single-stranded cDNAs are recovered and purified from the culture medium. When λ phage vectors are used, the same procedure as described above are used after the conversion of the vectors into phagemids by *in vivo* excision (Molecular Cloning 2nd Ed.). The purification of single-stranded DNAs can be performed according to the method described in Molecular Cloning 2nd Ed.

Specific procedures, reagent compositions and reaction conditions described in Genes to Cells, 3, 459 (1998) can be used in the subtraction. After the biotinylation of the mRNAs prepared from the heart of an 8-week-old rat by the method described in (1)-1-A with PHOTOPROBE biotin (Vector Laboratories), and such, they are hybridized to the above-mentioned single-stranded cDNAs from the heart of 16-day-old fetal rat. After hybridization, the solution is reacted with streptavidin, which tightly binds to biotin, to increase the hydrophobicity. Then, extraction is carried out by adding phenol thereto. Non-hybridized cDNAs are separated into the aqueous layer, and cDNAs that hybridized to the biotinylated mRNAs are extracted into the phenol layer.

### (1)-1-C. Construction of a cDNA library after subtraction

The subtracted cDNAs prepared in (1)-1-B are converted to double-stranded DNA using an appropriate primer, which has a nucleotide sequence complementary to the nucleotide sequence of the vector portion, and DNA polymerase, such as BcaBEST (TaKaRa Shuzo) or Klenow fragment, to reconverted the cDNAs to a cDNA library by introducing them to *E. coli*. A preferred method for introducing DNA into *E. coli* is electroporation due to its high transformation efficiency.

### (1)-2. Differential hybridization

Complementary DNAs, which correspond to genes whose expression levels are elevated in the heart of a 16-day-old fetal rat, are enriched in the subtracted cDNA library prepared in (1)-1. However, not all of the cDNA clones in the library correspond to genes associated with myocardial cell proliferation. In order to select cDNAs of myocardial cell proliferation-associated genes, the expression levels of mRNAs from the heart are compared between a 16-day-old fetal rat and an 8-week-old rat by Northern hybridization (Molecular Cloning 2nd Ed.) using respective cDNA clones as a probe or by RT (reverse-transcribed)-PCR (PCR Protocols, Academic Press (1990)) using primers based on the nucleotide sequence of the cDNA clones. Then, cDNAs of myocardial cell proliferation-associated genes are selected as cDNAs whose mRNA expression eve is higher in either of the two rat hearts. Alternatively, differential hybridization described below enables the inclusive and efficient selection of cDNA clones whose expression levels is higher in either of the two hearts.

First, the subtracted cDNA library provided by the method described in (1)-1 is diluted to a concentration which enables separation of respective colonies. Then, the dilution is plated on an agar medium for cultivation, the colonies are isolated, and each isolated colony is cultured in a liquid culture medium under the same condition. cDNA is amplified by PCR using cloning vector-specific oligonucleotide primers and, as a template, cDNA comprised in *E. coli* of the culture liquid. Then, equal aliquots of the reaction solution are respectively spotted onto two sheets of nylon membrane. Following the denaturation and neutralization of the DNAs on the nylon membranes by the method described in "Molecular Cloning 2nd Ed.", the DNAs are immobilized on the nylon membranes by ultraviolet-light irradiation. One of the two membranes is hybridized with total mRNA from the heart of a 16-day-old fetal rat as a probe, and the other with total mRNA from the heart from an 8-week-old rat. Hybridization signal intensity of respective clones is compared to select clones whose expression levels vary between the heart of an 8-week-old rat and that of a 16-day-old fetal rat. The colonies corresponding to the selected clones are isolated and separately cultured on a 96-well plate. After the culture media is aliquoted as reaction solution by an automatic micro-aliquoter for 96-well plate, such as Hydra96 (Robbins Scientific), PCR is performed. The procedure enables the easy and rapid preparation of two sheets of identical membranes on which the same amount of DNAs of many clones are blotted. In addition, the spots clearly correspond to the original clones.

As the probe, labeled cDNA prepared from total mRNA by an usual method for labeling DNA probe using reverse transcriptase and random primer can be used. However, as compared to DNA probes, RNA probes hybridize more tightly to DNAs on membranes to give intense signals, and thus are preferable. The labeling of the probe can be done using radioisotopes such as ³²P and ³⁵S or nonradioactive substances such as digoxigenin (DIG) and biotin. In terms of safety, nonradioactive substances are more preferable.

The respective RNA probes derived from the heart of a 16-day-old fetal rat and that of an 8-week-old rat are hybridized to the DNAs on the membranes prepared above, and then probes that hybridized to the DNA of respective colonies are detected. Detection of a hybridized probe is performed by any suitable method adapted to the type of the used label. Highly sensitive and quantitative detection methods include, for example, the use of a radioisotope as a label; autoradiography wherein an X-ray film or imaging plate is directly exposed to the signal; and the use of DIG as a label, wherein alkaline phosphatase-labeled anti-DIG antibody is bound according to the DIG system users' guide (Roche), and then reacted with a substrate that gives alkaline phosphatase-mediated light emission, such as CSPD to expose an X-ray film.

For example, the ratio of mRNA molecules corresponding to a gene whose expression in the heart is higher in either a 16-day-old fetal rat or an 8-week old rat is expected to be higher in either of the probes. Thus, when an equal amount of DNA is blotted on the membranes, more probes bind to a cDNA spot corresponding to the gene. Namely, a cDNA corresponding to a gene whose expression level varies between the heart of a 16-day-old fetal rat and that of an 8-week-old rat, can be selected by comparing the intensities of hybridization signals of spots on two membranes, that are blotted with the same cDNA clone.

Rat cDNAs obtained as described above include those having the nucleotide sequences of SEQ ID NOs: 1, 3, 5, 7, 9, 11, 13, 15, 17, 19, 21, 23, 25, 27, 29, 32, 33, 35, and 37.

### (2) Nucleotide sequence analysis of the DNAs:

The nucleotide sequences of cDNAs selected by the above-mentioned method corresponding to genes of which expression levels vary between a 16-day-old fetal rat and an 8-week-old rat, can be determined by commonly used methods for nucleotide sequence analysis, for example, the dideoxy method of Sanger et al. (Proc. Natl. Acad. Sci., USA, 74, 5463 (1977)) or using a DNA sequencer such as 373A DNA sequencer (Perkin Elmer).

Then, novelty of nucleotide sequence determined .by the above-mentioned method can be confirmed by homology search against nucleotide sequence databases, such as GenBank, EMBL, and DDBJ, using a homology search program such as Blast.

### (3) Preparation of full-length cDNAs:

When a DNA obtained by the above-described method is a partial cDNA, for example, a cDNA fragment further extending to the 5' direction, as. compared with the partial cDNA, can be obtained by 5'-RACE, wherein PCR is carried out using primers based on the nucleotide sequence of the cDNA clone (Proc. Natl. Acad. Sci. USA, 85, 8998 (1988)). Further, a full-length cDNA can be obtained by assembling the cDNA fragment with the original partial cDNA.

Full-length cDNAs of myocardial cell proliferation-associated genes that can be obtained according to the above described method include, for example, DNAs having the nucleotide sequences of SEQ ID NOs: 21, 23, 25, 27, and 30.

Furthermore, once a full-length cDNA nucleotide sequence is revealed as described above, a DNA of interest can be obtained by PCR, using as a template the cDNA or cDNA library synthesized from mRNA and primers prepared based on the nucleotide sequence of the full-length cDNA. Alternatively, a DNA of interest can be prepared by chemical synthesis, using a DNA synthesizer, based on the determined nucleotide sequence of the full-length cDNA. Exemplary DNA synthesizers include model 392 from Perkin Elmer, which utilizes the phosphoramidite method.

### (4) Isolation of corresponding genes of human:

Genes corresponding to the rat genes obtained as described above, whose expression levels vary between the hearts of fetal rat and adult rat, are also expected to exist in human. In general, proteins having the same function are highly homologous to each other, even when the proteins originate from different animal species, and further, the nucleotide sequences of genes encoding the proteins tend to exhibit high homology to each other. Thus, human cDNAs of interest may be obtained from a human heart cDNA library by hybridization under slightly stringent conditions using the rat cDNAs as probes. Such slightly stringent conditions can be determined according to the following method.

Although depending on the degree of homology between human cDNA and rat cDNA, slightly stringent conditions can be determined as follows. Human chromosomal DNA is digested with restriction enzymes, and then Southern blotting on the digested DNA is carried out using rat cDNA as a probe under several hybridization conditions with varying degrees of stringency. The most stringent condition of those conditions giving clear hybridization bands is determined as the slightly stringent condition. Specifically, when hybridization buffer without formamide is used, the hybridization is carried out in a hybridization buffer with fixed salt concentration of 1 M changing the hybridization temperature gradually from 68 to 42°C. Membrane wash is carried out using 2 x SSC containing 0.5% SDS at the same temperature as used in the hybridization. When a hybridization buffer containing formamide is used, hybridization is carried out at fixed temperature (42°C) and salt concentration (6x SSC) whereas the formamide concentration is gradually changed within a concentration ranging from 50% to 0%. Membrane wash is carried out using 6 x SSC containing 0.5% SDS at 50°C.

Further, nucleotide sequence novelty and homology search is performed by the same method as described in (2) with respect to the nucleotide sequence of rat cDNA obtained in (1) or (3). The search is carried out to select nucleotide sequences of human cDNAs that exhibit 60% homology, preferably 80% or higher homology to the whole protein-coding region of the nucleotide sequence of a rat cDNA. A human cDNA exhibiting high homology is expected to be a cDNA of a human counterpart of a rat gene obtained in (1) or (3). Further, the human cDNA can be amplified by RT-PCR using primers corresponding to the nucleotide sequences of the 5' end and 3' end of the human cDNA and, as a template, RNAs extracted from human cells or tissues, preferably heart tissue or cells derived from the heart. In some cases, the nucleotide sequence of a human cDNA found in a database may be only partial sequence or EST, but even in such cases, a full-length human counterpart cDNA of the rat cDNA can be obtained by the same method as described in (3).

Furthermore, the nucleotide sequence of the obtained human cDNA can be analyzed by the same method as described in (2) to determine the amino acid sequence of human protein encoded by the cDNA.

### (5) Isolation of genomic genes:

A rat or human genomic DNA corresponding to the gene of the present invention can be obtained by plaque hybridization and such, by screening a genomic DNA library prepared using chromosomal DNA isolated from rat or human cells or tissues using the rat or human cDNA obtained in (1) or (4) as a probe, following the method described in Molecular Cloning, 2nd Ed. The exon/intron organization of the genomic gene can be clarified by comparing the nucleotide sequence of genomic DNA to that of the cDNA. Furthermore, the nucleotide sequence of the genomic region responsible for transcriptional regulation, including the promoter and such, of a gene of this invention can be determined using the 5' end of the cDNA as a probe. Said sequences are useful for analyzing the regulatory mechanism involved in the transcription of the gene of the present invention. Moreover, clones wherein a gene of the present invention on the chromosome has been inactivated or substituted with an arbitrary sequence can be created by a technique of homologous recombination (e.g., Nature, 324, 34-38 (1987); Cell, 51, 503-512 (1987)).

### (6) Preparation of oligonucleotides:

Based on the sequence information of a DNA of the present invention, an oligonucleotide or an antisense oligonucleotide having a partial sequence of the DNA of the present invention can be prepared by usual methods or on DNA synthesizer.

The oligonucleotide or antisense oligonucleotide includes, for example, a sense primer corresponding to the nucleotide sequence of the 5' end of a nucleotide sequence of an mRNA to be detected, or an antisense primer corresponding to the nucleotide sequence of the 3' end thereof. However, nucleotides corresponding to uracil of mRNA are thymidine in an oligonucleotide primer. A preferred pair of sense primer and antisense primer include oligonucleotides having 5 to 60 nucleotides whose melting temperatures (Tm) and numbers of nucleotides are not extremely different from each other.

Furthermore, derivatives of the oligonucleotides (hereinafter referred to as "oligonucleotide derivatives") can be also used as oligonucleotides of the present invention.

The oligonucleotide derivatives include, oligonucleotide derivatives wherein phosphodiester bond is converted to phosphorothioate bond; oligonucleotide derivatives wherein phosphodiester bond is converted to N3'-P5' phosphoramidate bond; oligonucleotide derivatives wherein ribose-phosphodiester bond is converted to peptide-nucleotide bond; oligonucleotide derivatives wherein uracil is substituted with C-5 propynyluracil; oligonucleotide derivatives wherein uracil is substituted with C-5 thiazole uracil; oligonucleotide derivatives wherein uracil is substituted with C-5 propynylcytosine; oligonucleotide derivatives wherein cytosine is substituted with phenoxazine-modified cytosine; oligonucleotide derivatives wherein ribose is substituted with 2'-O-propylribose; and oligonucleotide derivatives wherein ribose is substituted with 2'-methoxyethoxyribose (Cell Technology, 16, 1463 (1997)).

### 2. Production of myocardial cell proliferation-associated proteins

As required, a DNA fragment with a suitable size containing the coding region for a protein can be prepared based on a full-length cDNA.

A recombinant expression vector to express the protein is constructed by inserting the DNA fragment or the full-length cDNA downstream of the promoter in the expression vector.

The recombinant expression vector is introduced into a host cell that is compatible with the expression vector.

All types of cells can be used as host cells so long as they can express a DNA of interest. Examples include bacterial cells belonging to the genus *Escherichia,* the genus *Serratia*, the genus *Corynebacterium,* the genus *Brevibacterium,* the genus *Pseudomonas,* the genus *Bacillus,* the genus *Microbacterium*, etc.; yeasts belonging to the genus *Kluyveromyces,* the genus *Saccharomyces*, the genus *Shizosaccharomyces*, the genus *Trichosporon,* the genus *Schwanniomyces*, etc.; animal cells; and insect cells.

An expression vector capable of replicating autonomously or being integrated into the chromosome of the host cell and containing a promoter at a suitable position where a DNA of a myocardial cell proliferation-associated gene can be transcribed is used for an expression vector.

When a bacterial cell is used as the host cell, as the recombinant expression vectors for a myocardial cell proliferation-associated gene, a recombinant expression vector that is capable of replicating autonomously in the bacterial cell and contain a promoter, a ribosome-binding sequence, a DNA of the myocardial cell proliferation-associated gene; and a transcription terminator sequence is preferably used. The vector may further contain genes that regulate the promoter.

Examples of such expression vectors include pBTrp2, pBTac1, pBTac2 (all the vectors are commercially available from Boehringer-Mannheim) ; pKK233-2 (Amersham Pharmacia Biotech) ; pSE280 (Invitrogen) ; pGEMEX-1 (Promega) ; pQE-8 (QIAGEN) ; pKYP10 (Unexamined Published Japanese Patent Application (JP-A) Sho 58-110600) ; pKYP200 (Agricultural Biological Chemistry, 48, 669 (1984)); pLSA1 (Agric. Biol. Chem., 53, 277 (1989)); pGEL1 (Proc. Natl. Acad. Sci. USA, 82, 4306 (1985)); pBluescript II SK(-) (Stratagene); pGEX (Amersham Pharmacia Biotech); pET-3 (Novagen); pTerm2 (USP 4686191; USP 4939094; USP 5160735); pSupex; pUB110; pTP5; pC194; pEG400 (J. Bacteriol., 172, 2392 (1990)); etc.

It is preferred to use an expression vector wherein the distance of the Shine-Dalgarno sequence that is a ribosome binding sequence, and the initiation codon is appropriately adjusted (e.g., 6 to 18 nucleotides).

Any promoter can be used, so long as it directs the expression in the host cell. Such promoters include, for example, trp promoter (Ptrp), lac promoter (Plac), P_{L} promoter, P_{R} promoter, and T7 promoter derived from *E. coli* or phage; SPO1 promoter; SPO2 promoter; penP promoter; etc. Artificially designed and modified promoters, such as Ptrp x 2 (a promoter wherein two Ptrp promoter units are connected in tandem) , tac promoter, letI promoter (Gene, 44, 29 (1986)), lacT7 promoter, etc., can be also used.

The production efficiency of a protein of interest can be improved by replacing the the protein-coding nucleotide sequence of a DNA of myocardial cell proliferation-associated gene of the present invention so with a codon that optimizes expression in particular host cell.

A transcription terminator sequence is not essential for the expression of a DNA of myocardial cell proliferation-associated gene of the present invention; however, it is preferable to arrange a transcription terminator sequence immediately downstream of the structural gene.

Host cells to be used for the present invention include microorganisms belonging to the genus *Escherichia*, the genus *Serratia*, the genus *Corynebacterium*, the genus *Brevibacterium*, the genus *Pseudomonas*, the genus *Bacillus,* the genus *Microbacterium*, etc.; specifically, for example, *Escherichia coli* XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No.49, *Escherichia coli* W3110, *Escherichia coli* NY49, *Bacillus subtilis, Bacillus amyloliquefaciens, Brevibacterium ammoniagenes, Brevibacterium immariophilum* ATCC14068, *Brevibacterium saccharolyticum* ATCC14066, *Corynebacterium glutamicum* ATCC13032, Corynebacterium *glutamicum* ATCC14067, *Corynebacterium glutamicum* ATCC13869, *Corynebacterium acetoacidophilum* ATCC13870, *Microbacterium ammoniaphilum* ATCC15354, *Pseudomonas* sp. D-0110, etc.

Any method for introducing a recombinant expression vector into host cells can be used, so long as it ensures the introduction of a DNA into the above-mentioned host cell. Such methods include, for example, a method utilizing calcium ion (Proc. Natl. Acad. Sci. USA, 69, 2110 (1972)), the protoplast method (JP-A Sho 63-248394), and methods described in the literature (Gene, 17, 107 (1982) ; Molecular & General Genetics, 168, 111 (1979)).

When a yeast cell is used as the host cell, suitable expression vectors include, for example, YEp13 (ATCC37115), YEp24 (ATCC37051), YCp50 (ATCC37419), pHS19, and pHS15.

Any promoter can be used, so long as it can direct the expression in yeast. Such promoters include, for example, PHO5 promoter, PGK promoter, GAP promoter, ADH promoter, gall promoter, gal10 promoter, heat-shock protein promoter, MFα1 promoter, and CUP1 promoter.

The host cells that can be used in the present invention include *Saccharomyces cerevisiae, Schizosaccharomyces pombe, Kluyveromyces lactis, Trichosporon pullulans, Schwanniomyces alluvius*, etc.

Any method for introducing a recombinant expression vector into yeast host cells can be used, so long as it ensures the introduction of a DNA into yeast. Such methods include, for example, electroporation (Methods. in Enzymol., 194, 182 (1990)), the spheroplast method (Proc. Natl. Acad. Sci. USA, 75, 1929 (1978)), the lithium acetate method (J. Bacteriol., 153, 163 (1983)), and the method described in Proc. Natl. Acad. Sci. USA, 75, 1929 (1978).

When an animal cell is used as the host cell, suitable expression vectors include, for example, pcDNAI (Invitrogen), pcDM8 (Invitrogen), pAGE107 (JP-A Hei 3-22979; Cytotechnology, 3, 133 (1990)), pAS3-3 (JP-A Hei 2-227075), pCDM8 (Nature, 329, 840 (1987)), pcDNAI/Amp (Invitrogen), pREP4 (Invitrogen), pAGE103 (J. Biochem., 101, 1307 (1987)), pAGE210, etc.

Any promoter can be used, so long as it directs the expression in animal cells. Such promoters include, for example, the promoter of the IE (immediate early) gene of cytomegalovirus (human CMV), SV 40 early promoter, retroviral promoter, metallothionein promoter, heat-shock protein promoter, and SRα promoter. Further, the enhancer of the IE gene of human CMV may be used in combination with its promoter.

The host cells to be used in the present invention include Namalwa cell, a human cell line; COS cell, derived from monkey; CHO cell, derived from Chinese hamster; HBT5637 (JP-A Sho 63-299); etc.

Any method for introducing a recombinant expression vector into animal host cells can be used as long as it ensures the introduction of a DNA into animal cells. Such methods include, for example, electroporation (Cytotechnology, 3, 133 (1990)), the calcium phosphate method (JP-A Hei 2-227075), and the lipofection method (Proc. Natl. Acad. Sci. USA, 84, 7413 (1987); Virology, 52, 456 (1973)). Preparation and cultivation of a transformant can be carried out according to the method described in JP-A Hei 2-227075 or JP-A Hei 2-257891.

When an insect cell is used as the host cell, the protein can be expressed, for example, by the method as described in "Baculovirus Expression Vectors, A Laboratory Manual"; "Current Protocols in Molecular Biology, supp. 1-38 (1987-1997)"; Bio/Technology, 6, 47 (1988), or the like.

Specifically, a recombinant gene transfer vector and baculovirus are co-introduced into insect cells to release recombinant viruses into the supernatant of insect cell culture. Then, another batch of insect cells are infected with the recombinant viruses to express the protein.

Suitable gene transfer vectors include, for example, pVL1392, pVL1393, pBlueBacIII (all of these are from Invitrogen), etc.

Baculoviruses that can be used in the present invention include, for example, *Autographa californica,* a nuclear polyhedrosis virus that is infectious to insects belonging to the family of armyworm.

Insect cell that can be used in the present invention include Sf9 and Sf21 both of which are ovarian cell lines from *Spodoptera frugiperda* (Baculovirus Expression Vectors, A Laboratory Manual, W. H. Freeman and. Company, New York, (1992)); and High5 (Invitrogen) that is an ovarian cell line from *Trichoplusia ni*; etc.

Methods for co-introducing the above-mentioned recombinant gene transfer vector and baculovirus into insect cells to prepare recombinant viruses include, for example, the calcium phosphate method (JP-A Hei 2-227075) and the lipofection method (Proc. Natl. Acad. Sci. USA, 84, 7413 (1987)).

To express a gene, besides direct expression, a protein can be produced and secreted, or expressed as a fusion protein according to methods described in Molecular Cloning 2nd Ed., etc.

Proteins conjugated with sugars or sugar chains can be obtained by expressing the proteins in yeast, animal cells, or insect cells.

A myocardial cell proliferation-associated protein can be produced by culturing, in a culture medium, a transformant harboring a recombinant DNA that is inserted with a DNA of a myocardial cell proliferation-associated gene; expressing and accumulating the myocardial cell proliferation-associated protein in the culture; and recovering the protein from the culture.

A transformant for the production of a myocardial cell proliferation-associated protein of the present invention can be cultured in a culture medium according to usual methods for culturing host cells (i.e., transformants).

When the transformant of the present invention is a prokaryotic host cell, such as *E. coli*, or a eukaryotic host cell, such as yeast, culture medium used for culturing the transformant may be any natural or synthetic culture medium containing carbon sources, nitrogen sources, inorganic substances, and others that are assimilated by the host cell (i.e., transformant) and which ensure efficient culture of the transformant.

Any carbon source that is assimilated by the host cell can be used, including glucose, fructose, sucrose, and molasses containing these sugars; carbohydrate, such as starch and starch hydrolysate; organic acids, such as acetic acid and propionic acid; and alcohols, such as ethanol and propanol.

Any nitrogen source can be used, including ammonia, various ammonium salts of inorganic or organic acids, such as ammonium chloride, ammonium sulfate, ammonium acetate, ammonium phosphate; other nitrogen-containing compounds; peptone; meat extract;, yeast extract; corn steep liquor; casein hydrolysate; soybean cake and soybean cake hydrolysate; and various fermenting microbial cells and digests thereof.

Any inorganic substance can be used, including potassium dihydrogenphosphate, dipotassium hydrogenphosphate, magnesium phosphate, magnesium sulfate, sodium chloride, ferrous sulfate, manganese sulfate, copper sulfate, calcium carbonate, etc.

Culturing is carried out under an aerobic condition by shaking culture, stirring culture with deep aeration, or the like. Preferred temperature for the culture ranges from 15 to 40°C. Typical culture period ranges from 16 hours to 7 days. The pH of culture medium is maintained within 3.0 to 9.0. The pH is adjusted by inorganic or organic acid, alkaline solution, urea, calcium carbonate, ammonia, or the like.

If required, an antibiotic, such as ampicillin or tetracycline, may be added to the culture medium during culturing.

To culture a transformant containing an expression vector with an inducible promoter, an inducer may be added to the culture medium if required. For example, to culture a transformant containing an expression vector with a lac promoter, isopropyl-β-D-thiogalactopyranoside (IPTG) or its equivalent may be added to the culture medium. To culture a transformant containing an expression vector with a trp promoter, indole acrylic acid (IAA) or its equivalent may be added to the culture medium.

When an animal cell is used as the host cell to provide a transformant, the culture medium to be used for the transformant includes the commonly used RPMI1640 (The Journal of the American Medical Association, 199, 519 (1967)), Eagle's MEM (Science, 122, 501 (1952)), Dulbecco's modified MEM (Virology, 8, 396 (1959)), 199 culture medium (Proceeding of the Society for the Biological Medicine, 73, 1 (1950)), these culture media supplemented with fetal calf serum, etc.

Culturing is typically carried out at pH 6 to 8, at 30 to 40°C, under an atmosphere of 5% carbon dioxide for 1 to 7 days.

If required, antibiotics, such as kanamycin or penicillin, may be added to the culture medium during culturing.

When an insect cell is used as the host cell to provide a transformant, culture medium to be used for the transformant includes commonly used TNM-FH (Pharmingen) ; Sf-900 II SFM (Life Technologies); ExCell400 and ExCell405 (both are from JRH Biosciences); Grace's Insect Medium (Grace, T.C.C., Nature, 195, 788 (1962)); etc.

Culturing is typically carried out at pH 6 to 7, at 25 to 30°C for 1 to 5 days.

If required, antibiotics, such as gentamycin, may be added to the culture medium during culturing.

The myocardial cell proliferation-associated protein can be isolated and purified from the culture of a transformant according to usual methods for protein isolation and purification.

For example, when the protein is produced in a soluble form in cells, the cells are harvested by centrifugation after culturing; suspended in aqueous buffer; and then, cell-free extract is prepared by disrupting the cells with a sonicator, French press, Manton Gaulin. homogenizer, DYNO-MILL, etc. The cell-free extract is centrifuged, and then, a purified preparation of the protein can be obtained from the obtained supernatant by commonly used methods for protein isolation and purification, including techniques such as solvent extraction, salting-out with ammonium sulfate or the like, desalting, precipitation with organic solvents, anion-exchange chromatography using a resin such as diethylaminoethyl (DEAE)-Sepharose and DIAION HPA-75 (Mitsubishi Chemical), cation-exchange chromatography using resin such as S-Sepharose FF (Amersham Pharmacia Biotech), hydrophobic chromatography using resin such as butyl-Sepharose and phenyl-Sepharose, gel filtration with molecule sieve, affinity chromatography, chromatofocusing, electrophoresis such as isoelectric focusing, etc. These techniques can be used either alone or in combination.

When the protein is produced as an insoluble matter in cells, the cells are harvested, crushed, centrifuged, and then, the protein insoluble matter can be recovered as a precipitated fraction.

The recovered protein insoluble matter is solubilized with a protein denaturant.

The resulting solution is diluted or dialyzed to decrease the concentration of the protein denaturant in the solution. Thus, the protein refolds into the normal conformation. Then, a purified preparation of the protein can be obtained by the same protein isolation and purification method described above.

When a protein or glycosylated form thereof is secreted extracellularly, the protein or glycosylated form thereof can be recovered from culture supernatant. Specifically, the supernatant is separated from the culture by techniques, such as centrifugation; and then, a purified preparation of the protein can be obtained from the supernatant by the protein isolation and purification method described above.

The proteins that can be obtained following the method described above include, for example, proteins having the amino acid sequences of SEQ ID NOs: 22, 24, 26, 28, and 31.

Alternatively, a protein of the present invention can also be produced by chemical synthesis methods, such as the Fmoc method (fluorenylmethyloxycarbonyl method) and tBoc method (t-butyloxycarbonyl method). Further, the protein can be synthesized by peptide synthesizer, for example, those from Advanced ChemTech (USA), Perkin-Elmer, Amersham Pharmacia Biotech, Protein Technology Instrument (USA), Synthecell-Vega (USA) ; PerSeptive (USA), Shimazu, etc.

### 3. Preparation of antibodies specifically recognizing myocardial cell proliferation-associated proteins:

Together with an appropriate adjuvant (e.g., complete Freund's adjuvant, aluminum hydroxide gel, Bordetella pertussis vaccine, etc.), a purified preparation of a full-length protein of the present invention or a partial fragment thereof, or a synthetic peptide having a partial amino acid sequence of a protein of the present invention is administered as an antigen subcutaneously, intravenously or intraperitoneally to a nonhuman mammal, such as rabbit, goat, rat, mouse and hamster, at a dose of about 50 to 100 µg/animal. When a peptide is used as the antigen, it is preferable to use the peptide that is covalently linked to a carrier protein, such as keyhole limpet haemocyanin or bovine thyroglobulin. The antigen peptide can be synthesized in a peptide synthesizer.

The antigen is given 3 to 10 times in total at 1- or 2-week intervals after the first administration. The blood is collected from the venous plexus of the eyeground 3 to 7 days after each administration to examine whether the serum is reactive to the antigen used for immunization by enzyme immunoassay (Enzyme Immunoassay (ELISA): Igakushoin (1976); Antibodies-A Laboratory Manual, Cold Spring Harbor Laboratory (1988)), or the like.

When a serum from a nonhuman mammal shows a sufficient antibody titer against the antigen used for immunization, the nonhuman mammal can be used as a source of the serum or antibody-producing cells.

Polyclonal antibodies can be purified from the serum.

Monoclonal antibodies can be obtained from a hybridoma prepared by fusing an antibody-producing cells with myeloma cells derived from a nonhuman mammal; culturing the hybridoma or transplanting the hybridoma to an animal to develop an ascite carcinoma; and isolating and purifying the antibody from the culture medium or the ascites.

Antibody-producing cells to be used in the present invention include those derived from spleen, lymph node, and peripheral blood. Antibody-producing cells from spleen are particularly preferable.

Preferred myeloma cells to be used in the present invention include mouse cell lines such as P3-X63Ag8-U1 (P3-U1) (Current Topics in Microbiology and Immunology, 18, 1 (1978)), P3-NS1/1-Ag41 (NS-1) (European J. Immunology, 6, 511 (1976)), SP2/O-Ag14(SP-2) (Nature, 276, 269 (1978)), P3-X63-Ag8653(653) (J. Immunology, 123, 1548 (1979)), P3-X63-Ag8 (X63) (Nature, 256, 495 (1975)), all of which are mouse (BALB/c-derived) myeloma cell lines that are resistant to 8-azaguanine.

Hybridoma cells can be prepared by the following method:

Antibody-producing cells and myeloma cells are combined and suspended in HAT culture medium (culture medium containing hypoxanthine, thymidine, and aminopterin). Then, the cells are cultured for 7 to 14 days. After cultivation, an aliquot of the culture supernatant is used for assays such as enzyme immunoassay to select hybridomas that produce antibodies reactive to the antigen but not to proteins without the antigen. Subsequently, hybridomas are cloned by the limiting-dilution method. Finally, hybridoma cells constantly showing high antibody titers by enzyme immunoassay are selected as monoclonal antibody-producing hybridomas.

Methods for isolating and purifying polyclonal antibodies or monoclonal antibodies include: centrifugal separation, ammonium sulfate precipitation, caprilic acid precipitation, and column chromatography using DEAE-Sepharose column, anion-exchange column, protein-A column, protein-G column, gel filtration column, etc. These methods can be used either alone or in combination.

### 4. Preparation of recombinant viral vectors producing myocardial cell proliferation-associated proteins:

A method for preparing a recombinant viral vector to produce a myocardial cell proliferation-associated protein of the present invention in specific human tissues is described in detail below.

Based on a full-length cDNA corresponding to a myocardial cell proliferation-associated gene, a DNA fragment of a suitable size containing the coding region of the protein is prepared if necessary.

A recombinant viral vector is then constructed by inserting the DNA fragment or the full-length cDNA downstream of a promoter in the viral vector.

When an RNA viral vector is used as the vector, the recombinant virus can be created by preparing an RNA fragment homologous to the full-length cDNA for the cardiac muscle proliferation-associated gene and inserting it downstream of the promoter in the virus vector. The RNA fragment may be selected from double-stranded strand, or alternatively may be either sense strand or antisense strand depending on the type of the viral vector. For example, where a retroviral vector is used, an RNA homologous to the sense strand is selected. When a sense viral vector is used, an RNA homologous to the antisense strand is selected.

The recombinant viral vector is introduced into a packaging cell compatible with the vector.

The packaging cells can be any of cells supplying proteins which are required for virus packaging and which are deficient in the recombinant viral vector wherein at least one of the genes encoding the proteins is deleted. For example, human kidney-derived HEK293 cell, mouse fibroblast cell NIH3T3, or the like may be used. Proteins to be supplied by the packaging cell include: retrovirus-derived gag, pol, and env when using a retroviral vector, murine; gag, pol, env, vpr, vpu, vif, tat, rev, and nef derived from HIV virus when using a lentiviral vector; adenoviral E1A and E1B, in case of an adenoviral vector; Rep (p5, p19, p40) and Vp (Cap) , when using an adeno-associated virus.

Viral vectors that are used in the present invention include those that produce recombinant viruses in the above-mentioned packaging cells and have a promoter at a position suitable for the transcription of a DNA of myocardial cell proliferation-associated gene in the target cells. Plasmid vectors that can be used in the present invention include MFG (Proc. Natl. Acad. Sci. USA, 92, 6733-6737 (1995)), pBabePuro (Nucleic Acids Res., 18, 3587-3596 (1990)), LL-CG, CL-CG, CS-CG, CLG (Journal of Virology, 72, 8150-8157 (1998)), pAdex1 (Nucleic Acids Res., 23, 3816-3821 (1995)), etc. Any promoter can be used so long as it directs the expression in human tissues, including, for example, the promoter of IE (immediate early) gene of CMV (human CMV), SV 40 early promoter, retroviral promoter, metallothionein promoter, heat-shock protein promoter, and SRα promoter. Further, an enhancer of IE gene of human CMV may be used along with the promoter.

Methods for introducing the recombinant viral vector into the packaging cells include, for example, the calcium phosphate method (JP-A Hei 2-227075) and lipofection method (Proc. Natl. Acad. Sci. USA, 84, 7413 (1987)).

### 5. Detection of mRNA of myocardial cell proliferation-associated genes:

A method for detecting mRNA of a myocardial cell proliferation-associated gene using a DNA of the gene of the present invention is described below.

DNAs that can be used in the following method include, for example, DNAs having the nucleotide sequences of SEQ ID NOs: 1, 3, 5, 7, 9, 11, 13, 15, 17, 19, 21, 23, 25, 27, 30, 32, 33, 35, and 37 and DNA fragments thereof.

Methods for detecting changes in the expression level or conformation of mRNA of a myocardial cell proliferation-associated gene include, for example, (1) Northern blotting, (2) *in situ* hybridization, (3) quantitative PCR, (4) differential hybridization,

### (5) DNA chip method, and (6) RNase protection assay.

Samples that can be used for the analysis by the above-mentioned method include mRNA or total RNA (hereinafter mRNA and total RNA are collectively referred to as "sample-derived RNA") obtained from biological samples such as heart tissues, serum, and saliva from heart disease patients or healthy persons; or samples of primary culture cells of cells that are obtained from the biological samples and are cultured in an appropriate culture medium in a test tube. In addition, paraffin or cryostat sections prepared from tissues obtained from the biological samples can also be used.

In Northern blotting, changes in the expression level or conformation of mRNA of a myocardial cell proliferation-associated gene can be detected by isolating sample-derived RNA by gel electrophoresis, transferring the isolated RNA onto a supporting material such as a nylon membrane, carrying out hybridization using a labeled probe prepared from a DNA of the present invention, washing the nylon membrane and detecting the band specific to the mRNA of the myocardial cell proliferation-associated gene. When hybridization is carried out, incubation should be performed under conditions ensuring the formation of a stable hybrid of the probe and mRNA of a gene associated with myocardial cell proliferation in the sample-derived RNA. Highly stringent conditions are preferable for the steps of hybridization and washing, in order to prevent false-positive reactions. Such conditions can be determined by considering various factors, such as temperature, ionic strength, nucleotide composition, length of probe, and formamide concentration. These factors are described, for example, in Molecular Cloning 2nd Ed.

The labeled probe to be used in Northern blotting can be prepared, for example, by incorporating a radioisotope, biotin, fluorescent group, chemiluminescent group, or the like, into a DNA of the present invention or an oligonucleotide designed based on the sequence of the DNA by a known method (nick-translation, random priming or kinasing). The amount of bound labeled probes reflects the mRNA expression level of a myocardial cell proliferation-associated gene. Thus, the level can be determined by quantifying the amount of bound labeled probes. Furthermore, conformational changes of mRNA of a myocardial cell proliferation-associated gene can be detected by analyzing the binding site of the labeled probe.

In situ hybridization is a method for detecting the mRNA expression level of a myocardial cell proliferation-associated gene, which comprises the steps of hybridization and washing using the above-mentioned labeled probe and paraffin or cryostat sections of tissues obtained from a living body. It is preferred to carry out the steps of hybridization and washing under highly stringent conditions to prevent false-positive reactions during *in situ* hybridization. The conditions can be determined by considering various factors such as temperature, ionic strength, nucleotide composition, length of probe, and formamide concentration. These factors are described, for example, in Current Protocols in Molecular Biology.

Methods for detecting mRNA of a myocardial cell proliferation-associated gene with, such as quantitative PCR, differential hybridization, and DNA-chip method, may comprise the step of synthesizing cDNA from sample-derived RNA using an oligo dT primer or random primer and reverse transcriptase (hereinafter the cDNA is referred to as "sample-derived cDNA"). When the sample-derived RNA is mRNA, both of the above-mentioned primers are usable, whereas the oligo dT primer is used in case of the sample-derived RNA as total RNA.

In quantitative PCR, DNA fragments derived from mRNA of a myocardial cell proliferation-associated gene are amplified by PCR using a sample-derived cDNA as a template and primers designed based on the nucleotide sequence of a DNA of the present invention. The amount of amplified DNA fragments reflects the mRNA expression level of the myocardial cell proliferation-associated gene. Thus, the mRNA level can be quantified by using a DNA encoding actin, G3PDH (glyceraldehyde 3-phosphate dehydrogenase), or the like as an internal control. Further, conformational changes of mRNA of a myocardial cell proliferation-associated gene can be detected by separating the .amplified DNA fragments by gel electrophoresis. According to this detection method, it is preferred to use primers that are suitable for specific and efficient amplification of the target sequence. Such suitable primers can be designed by considering conditions where neither inter- nor intra-primer base pairing is formed, and where the primers specifically bind to the target cDNAs at a certain annealing temperature and dissociate from the target cDNAs by denaturation. The quantification of amplified DNA fragments must be carried out within a logarithmic phase of the amplification during PCR. A phase of PCR can be identified by recovering DNA fragments amplified in each reaction cycle and quantitatively analyzing them by gel electrophoresis.

Differential hybridization (Trends in Genetics, 7, 314-317 (1991)) and the DNA chip method (Genome Research, 6, 639-645 (1996)) are methods for detecting differences in expression levels of mRNA of a myocardial cell proliferation-associated gene, which comprise the step of hybridization and washing on a filter or a base, such as glass slide or silicon, on which a DNA of the present invention has been immobilized, using a sample-derived cDNA as a probe. According to either method, the differences in expression level of mRNA of the myocardial cell proliferation-associated gene between control and target samples can be accurately detected by immobilizing actin gene, G3PDH gene, or the like, as an internal control on the filter or base. Alternatively, the expression level of mRNA of a myocardial cell proliferation-associated gene can be accurately quantified by synthesizing labeled cDNA probes from RNA either derived from control sample or target sample using different labeled dNTPs, and then hybridizing these probes simultaneously on the filter or base.

RNase protection assay can be carried out by the following procedure. First, a promoter sequence, such as T7 promoter or SP6 promoter, is linked to the 3' end of a DNA of the present invention. A labeled antisense RNA is synthesized by *in vitro* transcription system that uses RNA polymerase and labeled rNTP. The labeled antisense RNA is annealed to sample-derived RNA. The resulting RNA-RNA hybrid is digested with RNase, and then the RNA protected fragment is detected as a band after gel electrophoresis. The expression level of mRNA corresponding to a myocardial cell proliferation-associated gene can be quantified by measuring the intensity of the obtained band.

### 6. Detection of causative genes of heart diseases:

A method for detecting causative genes of heart diseases caused by myocardial degeneration wherein DNAs of the myocardial cell proliferation-associated genes of the present invention are used is described below.

DNAs to be used in the method include, for example, DNAs having the nucleotide sequences of SEQ ID NOs: 1, 3, 5, 7, 9, 11, 13, 15, 17, 19, 21, 23, 25, 27, 30, 32, 33, 35 and 37, and DNA fragments thereof.

The most apparent test for evaluating the presence or absence of a causative mutation of a heart disease, which is located within the locus of a myocardial cell proliferation-associated gene, is a direct comparison of the gene between a control group and a heart disease patient.

Specifically, human biological samples, such as heart tissue, serum and saliva, are collected from a heart disease patient and a healthy person. Alternatively, samples are prepared from primary culture cells established from the biological samples. DNAs are extracted from the biological samples or primary culture cell-derived samples (hereinafter the DNA is referred to as "sample-derived DNA") The sample-derived DNA can be used directly as the sample DNA. Alternatively, DNAs corresponding to a myocardial cell proliferation-associated gene amplified from the sample-derived DNA using primers designed based on the nucleotide sequence of a DNA of the present invention can also be used as the sample DNA. Alternatively, DNA fragments, comprising a myocardial cell proliferation-associated gene amplified by PCR using sample-derived cDNA as a template and primers designed based on the nucleotide sequence of a DNA of the present invention, can also be used as the sample DNA.

A hetero-duplex formed by the hybridization of a DNA strand containing the wild-type allele to a DNA strand containing the mutant allele can be detected as a method to determine the presence of causative mutation of a heart disease in a DNA of a myocardial cell proliferation-associated gene.

Methods for detecting a hetero-duplex include: (1) polyacrylamide gel electrophoresis (Trends Genet., 7, 5 (1991)) ; (2) hetero-duplex detection method by single-strand conformation polymorphism analysis (Genomics, 16, 325-332 (1993)); (3) the method of chemical cleavage of mismatches (CCM; Human Genetics (1996), Tom Strachan and Andrew P. Read, BIOS Scientific Publishers Limited); (4) the method of enzymatic cleavage of mismatches (Nature Genetics, 9, 103-104 (1996)); and (5) denaturing gradient gel electrophoresis (Mutat. Res., 288, 103-112 (1993)); etc.

According to the hetero-duplex detection method by polyacrylamide gel electrophoresis, a DNA of a myocardial cell proliferation-associated gene is amplified as a fragment shorter than 200 bp using a sample-derived DNA or sample-derived cDNA as a template, and primers designed based on the nucleotide sequence of SEQ ID NO: 1, 3, 5, 7, 9, 11, 13, 15, 17, 19, 21, 23, 25, 27, 30, 32, 33, 35 or 37; and then the DNA fragment is subjected to polyacrylamide gel electrophoresis. When a hetero-duplex is formed due to a mutation in the DNA of the myocardial cell proliferation-associated gene, the mobility of the duplex in the gel is lower than that of the homo-duplex without mutations. Thus, such a hetero-duplex can be detected as an extra band. A special gel (Hydro-link, MDE, etc.) gives a higher resolution. Insertions, deletions, and most of the single-nucleotide substitutions can be detected according to the method when the DNA fragment is shorter than 200 bp. It is preferred to carry out the hetero-duplex analysis on a single sheet of gel in combination with a single-strand conformation polymorphism analysis as described below.

According to the single-strand conformation polymorphism analysis (SSCP analysis), a DNA of a myocardial cell proliferation-associated gene is amplified as a fragment shorter than 200 bp using a sample-derived DNA or sample-derived cDNA as a template and primers designed based on the nucleotide sequence of the DNA of the present invention; and then the amplified DNA is electrophoresed on a non-denaturing polyacrylamide gel after denaturation. The amplified DNA of the myocardial cell proliferation-associated gene can be detected as a band by labeling the primers with a radioisotope or fluorescent dye before DNA amplification. Alternatively, unlabeled amplified products can be visualized by silver-staining. Fragments having mutated nucleotide sequences can be detected based on the difference in electrophoretic mobility by the co-electrophoresis of a control DNA to discriminate the difference of the electrophoretic pattern of the wild type and that of the mutant.

According to the method of chemical cleavage of mismatches (CCM), a DNA fragment of a myocardial cell proliferation-associated gene is amplified using a sample-derived DNA or sample-derived cDNA as a template, and primers designed based on the nucleotide sequence of SEQ ID NO: 1, 3, 5, 7, 9, 11, 13, 15, 17, 19, 21, 23, 25, 27, 30, 32, 33, 35 or 37. Mutations in the nucleotide sequence can be detected by hybridizing the amplified DNA fragment with a labeled DNA that has been prepared by incorporating a radioisotope or fluorescent dye into a DNA of the present invention, and digesting one of the DNA strands at the mismatched position by osmium-tetroxide treatment. The method of CCM is one of the most sensitive detection methods, and is applicable to sample of kilobase-length.

A mismatch can be digested enzymatically by the combined use of RNaseA and, instead of the use of osmium tetroxide described above, another enzyme such as T4 phage resolvase or endonuclease VII that are associated with the repair of intracellular mismatches.

According to denaturing gradient gel electrophoresis (DGGE), a DNA fragment of a myocardial cell proliferation-associated gene is amplified using a sample-derived DNA or sample-derived cDNA as a template, and primers designed based on the nucleotide sequence of SEQ ID NO: 1, 3, 5, 7, 9, 11, 13, 15, 17, 19, 21, 23, 25, 27, 30, 32, 33, 35 or 37. Then, the amplified DNA fragment is electrophoresed. on a gel with a concentration gradient of a chemical denaturant or alternatively a temperature gradient. The amplified DNA fragment migrates to a position in the gel where the DNA is denatured to single-stranded chains, and the DNA no longer migrates after denaturation. Mutations can be detected due to the differences in the mobility of the amplified DNA in the gel, depending on the presence of mutations in the DNA of the myocardial cell proliferation-associated gene. The addition of a poly(G:C) tail to primers improve the detection sensitivity.

An alternative method for detecting causative genes of heart diseases includes the protein truncation test (PTT method; Genomics, 20, 1-4 (1994)). According to the test, a frame-shift mutation, splice-site mutation and nonsense mutation, all of which may result in protein deficiency, can be specifically detected. In the PTT method, a specific primer, wherein a T7 promoter sequence and eukaryotic translation initiation sequence are linked to the 5' end of a DNA having the nucleotide sequence of SEQ ID NO: 1, 3, 5, 7, 9, 11, 13, 15, 17, 19, 21, 23, 25, 27, 30, 33 or 35, is used; next, cDNA is prepared from a sample-derived RNA by reverse transcription-PCR (RT-PCR) using the primer. Proteins can be produced by in vitro transcription and translation using the cDNA. Then, the protein is electrophoresed on a gel. When the position of the protein after electrophoresis corresponds to that of a full-length protein, no mutation resulting in protein deficiency exist in the gene. On the other hand, when the protein is deleted, such a protein migrates to a position which corresponds to a shorter protein than the full-length protein by electrophoresis. Furthermore, the position of protein migration reflects the degree of deletion.

Primers designed based on the nucleotide sequence of a DNA of the present invention can be used in order to determine the nucleotide sequences of sample-derived DNA and sample-derived cDNA. The presence of causative mutations of a heart disease in the sample-derived DNA or sample-derived cDNA can be assessed based on the determined nucleotide sequence.

Mutations located outside the coding region of a myocardial cell proliferation-associated gene may be detected by analyzing non-coding regions such as regions in the vicinity of the gene, introns thereof, and regulatory sequence thereof. Heart diseases caused by mutations in the non-coding regions can be detected by the same method as described above.

A gene, which has been suggested to have a mutation in the non-coding region by the method as described above, can be cloned using, as a hybridization probe, a DNA having the nucleotide sequence of SEQ ID NO: 1, 3, 5, 7, 9, 11, 13, 15, 17, 19, 21, 23, 25, 27, 30, 32, 33, 35 or 37. The mutation in the non-coding region can be found according to any one of the above-mentioned methods.

An identified mutation can be analyzed according to the statistical method described in Handbook of Human Genetics Linkage (The John Hopkins University Press, Baltimore (1994)) to identify the mutation as SNP (Single nucleotide polymorphism) linked to a heart disease. Furthermore, a causative gene of a heart disease may be identified by preparing DNAs from a family whose members have anamneses associated with the heart disease according to the method described above and detecting mutations therein.

### 7. Methods for predicting the onset and prognosis of heart disease using DNA of myocardial cell proliferation-associated gene:

DNAs used in the method include, for example, DNAs having the nucleotide sequences of SEQ ID NOs: 1, 3, 5, 7, 9, 11, 13, 15, 17, 19, 21, 23, 25, 27, 30, 32, 33, 35 and 37, and DNA fragments thereof.

The cause of a heart disease can be identified by detecting gene mutations in any tissues from a human individual. For example, when a mutation exists in the germ line, an individual who has inherited the mutation may have a tendency to be affected with heart disease. The mutation can be detected by testing DNA from any of tissues of the individual. For example, a heart disease can be diagnosed by extracting DNA from cells of collected human blood and detecting gene mutations using the DNA. Alternatively, prenatal diagnosis for a heart disease can be performed by collecting fetal cells, placental cells or amniotic cells, extracting DNA from the cells and detecting gene mutations using the DNA.

Further, the type of heart disease can be diagnosed by preparing DNA from a living tissue from lesions of a patient who has developed a heart disease and detecting alterations in genes. The diagnosis may be useful for selecting drugs to be administered. The DNA of the living tissue can be prepared by taking a tissue of the lesion isolated from the peripheral normal tissues, treating it with trypsin or the like, culturing the cells obtained in an appropriate culture medium, and extracting chromosomal DNA and mRNA from the cultured cells.

DNA obtained from human sample by any one of the above-mentioned methods for the purpose of diagnosing a disease is hereinafter referred to as "diagnostic sample-derived DNA". Further, cDNA synthesized from RNA which is obtained from human sample by any one of the above-mentioned methods for the purpose of diagnosing a disease is referred to as "diagnostic sample-derived cDNA".

Using DNA of a myocardial cell proliferation-associated gene and diagnostic sample-derived DNA or diagnostic sample-derived cDNA, a heart disease can be diagnosed according to the methods as described above for detecting a causative gene of heart diseases.

Further, methods for diagnosing heart diseases using DNA of a myocardial cell proliferation-associated gene and diagnostic sample-derived DNA or diagnostic sample-derived cDNA include: (1) the method comprising the detection of restriction enzyme sites; (2) the method using an allele-specific oligonucleotide probe (ASO: allele specific oligonucleotide hybridization); (3) PCR using allele-specific oligonucleotide (ARMS: amplification refractory mutation system); (4) oligonucleotide ligation assay (OLA); (5) PCR-PHFA method (PCR-preferential homoduplex formation assay); and (6) method using an oligo DNA array (Protein, Nucleic Acid and Enzyme, 43, 2004-2011 (1998)).

The detection of restriction enzyme sites can be achieved by the following method. When a restriction enzyme site is lost or newly generated due to a single nucleotide alteration, mutation can be simply detected by a procedure which comprises amplifying the diagnostic sample-derived DNA or the diagnostic sample-derived cDNA by PCR using primers designed based on the nucleotide sequence of a DNA of a myocardial cell proliferation-associated gene of the present invention, digesting with restriction-enzyme and comparing the obtained restriction fragments of the DNA with those of a normal person. However, a single-nucleotide alteration is a rare event. Thus, for diagnostic purposes, oligonucleotide probes are designed based on the sequence information of a DNA of a myocardial cell proliferation-associated gene of the present invention in conjunction with the information of the separately identified mutations. Then the oligonucleotide probes are immobilized on a filter and subjected to hybridization to detect the mutations by reverse-dot blotting.

The method using allele-specific oligonucleotide probes (ASO) is characterized by hybridization of a short synthetic DNA probe to only a full-matched nucleotide sequence. Thus, single-nucleotide mutations can be detected readily by this method. Specifically, the oligonucleotide probe is designed based on the nucleotide sequence of a DNA of the present invention and on identified nucleotide mutations. The oligonucleotide probe is immobilized on a filter. Hybridization is carried out using a probe prepared from diagnostic sample-derived DNA or diagnostic sample-derived cDNA by PCR using labeled dNTP and primers designed based on the sequence of the DNA of the present invention. Such reverse-dot blotting is often used for this method with ASO.

According to the reverse-dot blotting, oligonucleotides, which have been designed based on the nucleotide sequence of a DNA of the present invention and on the information of mutations, are synthesized directly on a base, such as glass slide and silicon, as a DNA chip (i.e., a high-density array). Then, a small amount of diagnostic sample-derived DNA or diagnostic sample-derived cDNA is reacted on the DNA chip. This mutation-detection method more simply detects various mutations and thus is suitable for large scale diagnosis.

Nucleotide mutations can also be detected by the following oligonucleotide ligation assay (OLA). OLA is described below in detail.

Two types of oligonucleotides, each of which consist of about 20 nucleotide residues, are prepared. Based on the nucleotide sequence of a DNA of the present invention, the oligonucleotides are designed so as to hybridize to the 5'-end and 3'-end sides of the mutated site, respectively. A DNA fragment of a myocardial cell proliferation-associated gene is amplified by PCR using diagnostic sample-derived DNA or diagnostic sample-derived cDNA as a template and primers designed based on the nucleotide sequence of the DNA of the myocardial cell proliferation-associated gene of the present invention. The above-mentioned two types of oligonucleotides are hybridized to the amplified DNA fragment. After hybridization, the two oligonucleotides are ligated to each other with DNA ligase. For. example, one of the two oligonucleotides is biotinylated and the other is labeled with a different labeling substance, such as digoxigenin, to rapidly detect the ligation reaction. OLA requires neither electrophoresis nor centrifugation. Thus, OLA is a mutation-detection method suitable for effective diagnosis of many samples in a short term.

Alternatively, the following PCR-PHFA method allows convenient and quantitative detection of a small quantity of DNA derived from a mutant gene.

PCR-PHFA method comprises: gene amplification (PCR), liquid-phase hybridization with a very high specificity, and ED-PCR (enzymatic detection of PCR product) which detects PCR products by the same procedure as in ELISA.

An amplification product labeled at both ends is prepared by PCR using a primer pair, wherein one is labeled with dinitrophenyl (DNP) and the other with biotin-labeled, and a DNA of the present invention as a template. The labeled product is combined with an excess amount of 20 to 100 fold of non-labeled amplification product that is obtained using a pair of non-labeled primers, which nucleotide sequences are the same as the labeled primers, and the diagnostic sample-derived DNA or diagnostic sample-derived cDNA as a template. The mixture is heat-denatured and cooled under a mild temperature gradient of about 1°C/5-10 minutes to preferentially form hybrids consisting of perfectly complementary strands. The reconstituted labeled DNA is trapped and adsorbed on a streptavidin-immobilized well via biotin. An enzyme-conjugated anti-DNP antibody is bound to the DNA *via* DNP to detected the resultant complex by coloring reaction with the enzyme. When no gene having the same sequence as that of the labeled DNA exists in the sample, double-stranded DNAs are preferentially reconstituted from the original pair of labeled DNAs and as a result the color is developed. On the other hand, when genes having the same nucleotide sequence are present, the amount of reconstituted labeled DNA markedly reduces due to the random replacement of the complementary strands which finally result in a marked decrease of color development. This method enables detection and quantification of known mutations and polymorphic genes.

### 8. Immunological method for detecting or quantifying myocardial cell proliferation-associated protein using antibody specifically recognizing the myocardial cell proliferation-associated protein:

Immunological methods for detecting and quantifying a myocardial cell proliferation-associated protein intracellulary or extracellulary expressed by microorganisms, animals, insects or tissues using an antibody (polyclonal or monoclonal antibody) that specifically recognizes the myocardial cell proliferation-associated protein of the present invention include fluorescent antibody method; enzyme immunoassay (ELISA); radioimmunoassay (RIA); immunohistochemistry (ABC method, CSA method, etc.), such as immunohistological staining and immunocytological staining; Western blotting; dot blotting; immunoprecipitation; sandwich ELISA (Monoclonal Antibody - Experimental Manual, Kodansha Scientific (1987); The second series of lectures on biochemical experiments Vol. 5, Immunobiochemical Experiments, Tokyo Kagaku Dozin (1986)).

The fluorescent antibody method comprises the steps of reacting an antibody of the present invention with a microorganism, animal cell, insect cell or tissue intracellulary or extracellulary expressing a myocardial cell proliferation-associated protein, further reacting thereto an anti-mouse IgG antibody or a fragment thereof labeled with a fluorescent substance such as fluorescein isothiocyanate (FITC) and assaying the fluorescent dye in flow cytometer.

The enzyme immunoassay (ELISA) comprises the steps of reacting an antibody of the present invention with a microorganism, animal cell, insect cell or tissue intracellulary or extracellulary expressing a myocardial cell proliferation-associated protein, further reacting thereto an anti-mouse IgG antibody labeled with an enzyme such as peroxidase and biotin or a labeled fragment thereof and assaying the color development with photospectrometer.

The radioimmunoassay (RIA) comprises the steps of reacting an antibody of the present invention with a microorganism, animal cell, insect cell or tissue intracellulary or extracellulary expressing a myocardial cell proliferation-associated protein, further reacting thereto an anti-mouse IgG antibody or a fragment thereof labeled with a radioisotope and measuring the radioactivity with scintillation counter, etc.

Immunohistochemistry such as immunohistological staining and immunocytological staining comprises the steps of reacting an antibody of the present invention with a microorganism, animal cell, insect cell or tissue intracellulary or extracellulary expressing a myocardial cell proliferation-associated protein, further reacting thereto an anti-mouse IgG antibody or a fragment thereof labeled with a fluorescent material such as FITC or an enzyme such as peroxidase and biotin, and observing the label under a microscope.

Western blotting comprises the steps of fractionating extract from microorganism, animal cell, insect cell or tissue intracellulary or extracellulary expressing a myocardial cell proliferation-associated protein by SDS-polyacrylamide gel electrophoresis (Antibodies - A Laboratory Manual, Cold Spring Harbor Laboratory, (1988)), transferring the protein from the gel onto a PVDF membrane or nitrocellulose membrane, reacting an antibody of the present invention with the membrane, further reacting thereto an anti-mouse IgG antibody or a fragment thereof labeled with a fluorescent substance such as FITC or an enzyme such as peroxidase and biotin and verifying the label.

Dot blotting comprises the steps of blotting an extract from microorganism, animal cell, insect cell or tissue intracellulary or extracellulary expressing a myocardial cell proliferation-associated protein onto a nitrocellulose membrane, reacting an antibody of the present invention with the membrane, further reacting thereto an anti-mouse IgG antibody or a fragment thereof labeled with a fluorescent substance such as FITC or an enzyme such as peroxidase and biotin and verifying the label.

Immunoprecipitation comprises the steps of reacting an antibody of the present invention with an extract of microorganism, animal cell, insect cell or tissue intracellulary or extracellulary expressing a myocardial cell proliferation-associated protein, adding thereto a carrier that specifically binds to immunoglobulin, such as protein G-Sepharose and precipitating the resulting antigen-antibody complex.

The sandwich ELISA comprises the steps of previously immobilizing one of two antibodies that specifically recognizes a myocardial cell proliferation-associated protein on a plate, wherein each of the antibodies recognizes a separate epitope of the same antigen, labeling the other antibody with a fluorescent substance such as FITC or an enzyme such as peroxidase and biotin, reacting an extract of microorganisms, animal cells, insect cells or tissues intracellulary or extracellulary expressing the protein of this invention with the antibody immobilized plate, reacting the labeled antibody thereto and detecting the labeled substance bound thereto.

### 9. Diagnosis for heart diseases using antibody specifically recognizing myocardial proliferation-associated protein:

The determination of the alterations in the expression level and conformation of a myocardial cell proliferation-associated protein expressed in human biological samples and human primary culture cells is useful in determining the risk of future onset of a heart disease as well as the cause of a heart disease already developed.

Methods for diagnosing the disease by detecting the expression level and conformational alterations of a myocardial cell proliferation-associated protein include the above-mentioned fluorescent antibody method, enzyme immunoassay (ELISA), radioimmunoassay (RIA) , immunohistochemistry such as immunohistological staining and immunocytological staining (ABC method, CSA method, etc.), Western blotting, dot blotting, immunoprecipitation, sandwich ELISA, etc.

The samples to be used in the diagnosis by the above-mentioned methods include biological samples *per se,* such as heart tissue from patient's lesions, blood, serum, urine, feces, and saliva; as well as cells and cell extracts obtained from the biological samples. In addition to these, paraffin or cryostat sections of tissues obtained from the biological samples may be used.

### 10. Screening of therapeutic agents for heart diseases using myocardial cell proliferation-associated protein, DNA encoding the protein or antibody recognizing the protein:

DNAs to be used in the screening method include, for example, DNA having the nucleotide sequences of SEQ ID NOs: 1, 3, 5, 7, 9, 11, 13, 15, 17, 19, 21, 23, 25, 27, 30, 32, 33, 35 and 37. Proteins to be used in the screening method include, for example, a protein having the amino acid sequence selected from the group consisting of the amino acid sequences of SEQ ID NOs: 22, 24, 26, 28 and 31; or a protein having an amino acid sequence wherein one or several amino acids are deleted, substituted, or added in the amino acid sequence of SEQ ID NO: 22, 24, 26 or 28, and having an activity associated with the healing of a heart disease caused by myocardial degeneration. Antibodies to be used in the screening method include antibodies that recognize the proteins.

Microorganisms, animal cells, and insect cells, which are transformed by introducing a DNA of a myocardial cell proliferation-associated gene of the present invention and produce the myocardial cell proliferation-associated protein or a partial polypeptide of the protein, as well as purified myocardial cell proliferation-associated proteins and purified polypeptides, are all useful for screening agents specifically acting on the myocardial cell proliferation-associated protein.

Such agents obtained by the screening may be useful for treating heart diseases.

One example of the above-mentioned screening methods comprises the step of selecting a target compound specifically binding to microorganisms, animal cells, and insect cells, which are transformed to produce a myocardial cell proliferation-associated protein of the present invention or a partial polypeptide of the protein (hereinafter the transformant is referred to as "transformant for screening"). The specific target compound can be detected by comparing its binding pattern to a non-transformed microorganism, animal cell, or insect cell used as a control. Alternatively, the target compound can be selected by a competitive screening using, as an index, the inhibition of binding between the "transformant for screening" and a compound or protein that binds specifically to the "transformant for screening."

The purified myocardial cell proliferation-associated protein of the present invention or the purified partial polypeptide of the protein can be used to select target compounds which specifically bind to the myocardial cell proliferation-associated protein. The target compound can be quantified by the above-mentioned immunological method, using an antibody that specifically recognizes the myocardial cell proliferation-associated protein of the present invention. Alternatively, a target.compound can be selected by competitive screening using, as an index, the inhibition of binding between a myocardial cell proliferation-associated protein or a myocardial cell proliferation-associated polypeptide and another target compound that binds to the protein or polypeptide.

Another example of the above-mentioned screening methods comprises the steps of synthesizing many partial peptides of a myocardial cell proliferation-associated protein on plastic pins or a solid-phase support in high density and then efficiently selecting compounds or proteins selectively binding to the peptides (WO 84/03564).

An expression-controlling agent, which enhances or suppresses the expression of mRNA of a myocardial cell proliferation-associated gene or a myocardial cell proliferation-associated protein in cells of a cardiac cell line, is also effective to treat heart diseases.

Substances suppressing or enhancing transcription or translation of a myocardial cell proliferation-associated gene can be screened by adding various test compounds to cells of a cardiac cell line and detecting changes in the mRNA expression level of the myocardial cell proliferation-associated gene using a DNA of the myocardial cell proliferation-associated gene of the present invention. Such changes in the mRNA expression level of a myocardial cell proliferation-associated gene can be detected by the above-mentioned PCR, Northern blotting, or RNase protection assay.

Alternatively, substances suppressing or enhancing transcription or translation of a myocardial cell proliferation-associated gene can be screened by adding various test compounds to cells of a cardiac cell line and detecting changes in the expression level of the myocardial cell proliferation-associated protein using an antibody specifically recognizing the myocardial cell proliferation-associated protein of the present invention. Such changes in the expression level of the myocardial cell proliferation-associated protein can be detected by the above-mentioned fluorescent antibody method, enzyme immunoassay (ELISA), radioimmunoassay (RIA), and immunohistochemical staining such as immunohistological staining and immunocytological staining (ABC method, CSA method, etc.), Western blotting, dot blotting, immunoprecipitation, and sandwich ELISA.

The compounds obtained by the above-mentioned methods can be assessed for their therapeutic effects on heart diseases by administering the compounds as therapeutic agents to a heart disease model animals, such as a model rat for cardiac infarction, and measuring cardiac action potential, cardiac rate, or the like, of the animal.

### 11. Method for delivering drugs to the heart in a specific manner using antibody specifically recognizing myocardial proliferation-associated protein (drug delivery method):

Any antibody can be used in the method of drug delivery, so long as it can specifically recognize a myocardial cell proliferation-associated protein of the present invention; humanized antibodies are particularly preferred.

The humanized antibodies include human chimeric antibodies, human CDRs (Complementary Determining Region; hereinafter abbreviated as "CDR"), grafted antibodies, etc.

The term "human chimeric antibody" refers to an antibody consisting of the variable region of an antibody heavy chain (hereinafter, the heavy chain and variable region are referred to as "H chain" and "V region", respectively; and thus the variable region of a heavy chain is referred to as "HV" or "VH") and antibody light chain (hereinafter, the light chain is referred to as "L chain"; and thus the variable region of light chain is referred to as "LV" or "VL"), both of which are derived from a nonhuman animal, and the constant region of a human antibody heavy chain (hereinafter, the constant region is referred to as "C region"; and thus the constant region of antibody heavy chain is referred to as "CH") and human antibody light chain (hereinafter also referred to as "CL"). Suitable nonhuman animals include mouse, rat, hamster, rabbit, and others, so long as they can be utilized to prepare hybridomas producing monoclonal antibodies.

A human chimeric antibody of the present invention can be produced by isolating cDNAs encoding VH and VL from a hybridoma producing a monoclonal antibody, which can bind to a myocardial cell proliferation-associated protein of the present invention and neutralize the activity of the protein; constructing a human chimeric antibody expression vector by inserting the respective cDNAs into a host cell expression vector containing genes encoding human antibody CH and human antibody CL; introducing the constructed expression vector into host cells; and expressing the antibody.

Any CH of human immunoglobulins (hereinafter abbreviated as "hIg") can be used for a human chimeric antibody of the present invention. However, those belonging to the hIgG class are preferable, and further those of any subclasses of hIgG1, hIgG2, hIgG3, and hIgG4 belonging to the hIgG class may be used. On the other hand, any CL may be used for the human chimeric antibody as long as they belong to the hIg, and those belonging to the κ class and λ class may be used.

The human CDR grafted antibody refers to an antibody constructed by grafting the amino acid sequences of CDRs in VH and VL of an antibody from a nonhuman animal into appropriate positions of the VH and VL of a human antibody.

A human CDR grafted antibody of the present invention can be produced by constructing cDNAs encoding V regions wherein the CDR sequences of VH and VL of an human antibody is substituted with VH and VL CDR sequences from a nonhuman antibody that is reactive to a myocardial cell proliferation-associated protein of the present invention, and which can bind to the myocardial cell proliferation-associated protein to neutralize the activity of the protein; inserting the respective cDNAs into expression vectors containing genes encoding the human antibody CH and CL; introducing the constructed expression vectors of human CDR grafted antibody into host cells; and expressing the antibody.

The CH for a human CDR grafted antibody of the present invention can be derived from any antibody belonging to the hIg. However, those belonging to the hIgG class are preferable, and further those of any subclass of hIgG1, hIgG2, hIgG3, and hIgG4 belonging to the hIgG class may be used. Further, a CL to construct the human CDR grafted antibody can be derived from any antibody belonging to the hIg class, and those belonging to the κ class and λ class may be used.

The human antibody originally referred to a natural antibody existing in human body. However, antibodies obtained from a phage library of human antibodies and from transgenic animals producing human antibodies created according to advanced techniques of genetic engineering, cell engineering and developmental engineering are also encompassed by the term.

An antibody existing in human body can be obtained., for example, by the following method.

Human peripheral blood lymphocytes are isolated and immortalized by infecting EB virus, or the like. The cells are then cloned. The resulting lymphocytes producing an antibody of interest are cultured. The antibody can be obtained from the culture.

A phage library of human antibody is a library wherein antibody genes prepared from human B cells are inserted into the phage genome and antibody fragments such as Fab and single-chain antibody are displayed on the phage particles. Phages expressing antibody fragments with the desired antigen binding activity can be recovered from the library using, as an index, the binding activity of the phage to a base immobilized with the antigen. Further, the antibody fragment can be converted into a complete human antibody by genetic engineering.

The human antibody-producing transgenic animal refers to an animal wherein human antibody genes are integrated in the cells thereof. Specifically, a human antibody-producing transgenic animal can be created by introducing human antibody genes into mouse ES cells, transplanting the ES cells into early embryos from another mouse individual, and developing the embryos. Methods for preparing human antibodies from a human antibody-producing transgenic animal include a method which comprises the step of preparing hybridomas producing a human antibody by conventional hybridoma preparation methods for a nonhuman mammal, culturing the hybridomas to produce and accumulate the human antibody in the culture.

Antibody fragments include Fab, Fab', F(ab')₂, single-chain antibody, disulfide-stabilized variable region fragment (dsFv), peptide containing CDR.

Fab is an antibody fragment with a molecular weight of about 50,000 having antigen-binding activity wherein the N-terminal half (nearly half) of the H chain and the entire L chain are bridged by a disulfide bond, which is one of the fragments provided by treating IgG with a proteolytic enzyme, papain (cleavage at amino acid residue 224 of the H chain).

The Fab of the present invention can be obtained by treating an antibody that specifically reacts to a protein of the present invention with the proteolytic enzyme papain. Alternatively, the Fab can be obtained by inserting DNAs encoding Fab of an antibody into an expression vector, introducing the vector into a host cell, and expressing the DNAs.

F(ab')₂ is an antibody fragment with a molecular weight of about 100,000 and having antigen-binding activity. It is one of the fragments provided by treating IgG with a proteolytic enzyme, pepsin (cleavage at amino acid residue 234 of the H chain) which fragment is slightly larger than two Fabs bridged together by a disulfide bond at the hinge region.

The F(ab')₂ of the present invention can be obtained by treating an antibody that specifically reacts to a protein of the present invention with the proteolytic enzyme pepsin. Alternatively, the F(ab')₂ can be obtained by inserting DNAs encoding F(ab')₂ of an antibody into an expression vector, introducing the vector into the host, and expressing the DNAs.

Fab' is an antibody fragment with a molecular weight of about 50,000 and having an antigen binding activity. It is provided by cleaving the disulfide bond in the hinge region of the above-mentioned F(ab')₂.

The Fab' of the present invention can be obtained by treating an antibody that specifically reacts to a protein of the present invention with a reducing agent dithiothreitol. Alternatively, the Fab' can be obtained by inserting DNAs encoding Fab' fragment of an antibody into an expression vector, introducing the vector into the host, and expressing the DNAs.

A single-chain antibody (hereinafter also referred as "scFv") is a VH-P-VL or VL-P-VH polypeptide provided by linking a single VH and a single VL together *via* an appropriate peptide linker (hereinafter referred as "P"). The VH and VL of the scFv to be used in the present invention can be derived from an antibody that specifically reacts to a protein of the present invention, for example, a humanized antibody or human antibody.

The single-chain antibody of the present invention can be obtained by the following method.

The single-chain antibody can be obtained by preparing cDNAs encoding VH and VL of an antibody that specifically reacts to a protein of the present invention, constructing DNAs encoding the single-chain antibody, inserting the DNAs into an expression vector, introducing the vector into the host, and expressing the DNAs.

A disulfide-stabilized variable-region fragment (hereinafter also referred as "dsFv") is an antibody fragment consisting of VH and VL, each of which has a cysteine residue substituted for the original amino acid residue. The two polypeptides are connected together at the cysteines which form a disulfide bond. The amino acid residues to be replaced with cysteine can be selected based on the predicted antibody conformation according to the method of Reiter et al. (Protein Engineering, 7, 697 (1994)).

The VH and VL of dsFv to be used in the present invention can be derived from an antibody that specifically reacts to a protein of the present invention, for example, a humanized antibody or human antibody.

The disulfide-stabilized variable-region fragment (dsFv) of the present invention can be obtained by the following method.

The dsFv can be obtained by preparing cDNAs encoding VH and VL of an antibody that specifically reacts to a protein of the present invention, constructing DNAs encoding the dsFv, inserting the DNAs into an expression vector, introducing the vector into the host, and expressing the DNAs.

A peptide containing CDR can be produced by a method of chemical synthesis such as Fmoc method and tBoc method.

Fusion antibodies described below, which are prepared from an antibody of the present invention, can be used in drug delivery wherein agents or proteins are delivered specifically to the heart lesions.

The fusion antibody is an antibody wherein agents such as radioisotope, protein, low-molecular-weight compound are chemically linked or linked by genetic engineering to an antibody that specifically reacts to a protein of the present invention, for example, a humanized antibody, a human antibody or an antibody fragment thereof.

The fusion antibody of the present invention can be produced by chemically linking or linking by genetic engineering an agent such as radioisotope, protein, low-molecular-weight compound to the N-terminal or C-terminal end of H or L chain of an antibody that specifically reacts to a protein of the present invention or alternatively an antibody fragment thereof, an appropriate substituent, side chain, or sugar chain in the antibody or antibody fragment.

The radioisotopes to be used for the fusion antibody include ¹³¹I and ¹²⁵I. Antibodies and antibody fragments can be labeled with the radioisotopes, for example, by chloramine T method, or the like.

The low-molecular-weight compounds used for the fusion antibody of the present invention include alkylating agents, such as nitrogen mustard and cyclophosphamide; antimetabolites, such as 5-fluorouracil and methotrexate; antibiotics, such as daunomycin, bleomycin, mitomycin C, daunorubicin and doxorubicin; plant alkaloids, such as vincristine, vinblastine and vindesine; anticancer agents, such as tamoxifen; hormones, such as dexamethasone (Clinical Oncology (Ed. Japanese Association of Clinical Oncology (1996) Cancer and Chemotherapy)) ; steroid drugs, such as hydrocortisone and prednisone; non-steroidal drugs, such as aspirin and indomethacin; immunomodulators, such as aurothiomalate and penicillamine; immunosuppressants, such as cyclophosphamide and azathioprine; and anti-inflammatory drugs, such as chlorpheniramine maleate and antihistamic agents, such as clemastine (Inflammation and anti-inflammatory treatment (1982) Ishiyaku Pub., Inc.).

The low-molecular-weight agents can be linked to the above-mentioned antibodies by usual methods. For example, daunomycin can be linked to an antibody by linking the amino groups of daunomycin to the antibody *via* glutaraldehyde or, alternatively, by linking the amino group of daunomycin to the carboxyl group of the antibody *via* water-soluble carbodiimide.

Preferred proteins for the fusion antibody include cytokines which activate immune cells and growth-regulating factors for the vascular endothelium, vascular smooth muscle, or the like. Examples of such proteins include human interleukin 2, human granulocyte-macrophage colony stimulating factor, human macrophage colony stimulating factor, human interleukin 12, fibroblast growth factor-2 (FGF-2) and platelet-derived growth factor (PDGF).

A fusion antibody with the protein can be prepared by the following method.

A DNA encoding the fusion antibody is constructed by ligating cDNAs encoding an antibody or an antibody fragment thereof to a cDNA encoding the protein. The fusion antibody can be obtained by inserting the DNA into a prokaryotic or eukaryotic expression vector, introducing the vector into the host prokaryote or eukaryote, and expressing the DNA.

### 12. Agents for gene therapy containing DNA of myocardial cell proliferation-associated gene:

An agent for gene therapy utilizing a viral vector containing a DNA of a myocardial cell proliferation-associated gene of the present invention can be prepared by mixing a recombinant viral vector prepared in Section 4 with a base used for gene therapy agents (Nature Genet., 8, 42 (1994)).

A base for the present gene therapy agents can be any base that is commonly used for injection, including distilled water; salt solutions, such as solution of sodium chloride and mixed solution comprising an inorganic salt and sodium chloride; solution of a sugar, such as mannitol, lactose, dextran and glucose; solution of an amino acid, such as glycine and arginine; mixed solution of organic acid solution or salt solution and glucose solution. Further, according to conventional methods, the base can be combined with an adjuvant, such as osmoregulator, pH modifier, vegetable oil such as sesame oil and soy bean oil, detergent such as lecithin and non-ionic detergent, to prepare them as a solution, suspension or dispersion for injection. By powdering, freeze-drying, or the like, these injections can be prepared as preparations to be dissolved at the time of use. When the agent for gene therapy of the present invention is a liquid, it can be used directly for the treatment. When it is a solid, it is dissolved, as required, in a sterilized base described above immediately before gene therapy. Methods for administering a gene therapy agent of the present invention include local administration method, wherein the agent is delivered from patient's coronary artery to the heart.

A viral vector can be delivered to the heart lesions via gene transfer by applying the method of liposome delivery, a method of direct in vivo gene transfer.

A viral vector can be prepared by combining an appropriately-sized DNA of a myocardial cell proliferation-associated gene of the present invention with a polylysine-conjugated specific antibody to the adenoviral hexon protein, and binding the resulting complex with the adenoviral vector. The viral vector is thus stabilized and reaches the target cells. The vector is incorporated via an endosome into the cell and disassembled in the cell, which allows efficient expression of the gene.

A DNA of a myocardial cell proliferation-associated gene can be delivered to the lesions by a non-viral method of gene transfer.

Such non-viral methods of gene transfer known to those skilled in the art include the calcium phosphate co-precipitation method (Virology, 52, 456-467 (1973); Science, 209, 1414-1422 (1980)), microinjection (Proc. Natl. Acad. Sci. USA, 77, 5399-5403 (1980); Proc. Natl. Acad. Sci. USA, 77, 7380-7384 (1980); Cell, 27, 223-231 (1981) ; Nature, 294, 92-94 (1981)), membrane fusion-mediated transfer using liposome (Proc. Natl. Acad. Sci. USA, 84, 7413-7417 (1987); Biochemistry, 28, 9508-9514 (1989); J. Biol. Chem., 264, 12126-12129 (1989); Hum. Gene Ther., 3, 267-275, (1992); Science, 249, 1285-1288 (1990); Circulation, 83, 2007-2011 (1992)), direct DNA incorporation and receptor-mediated DNA transfer method (Science, 247, 1465-1468 (1990); J. Biol. Chem., 266, 14338-14342 (1991); Proc. Natl. Acad. Sci. USA, 87, 3655-3659 (1991); J. Biol. Chem., 264, 16985-16987 (1989); BioTechniques, 11, 474-485 (1991); Proc. Natl. Acad. Sci. USA, 87, 3410-3414 (1990); Proc. Natl. Acad. Sci. USA, 88, 4255-4259 (1991); Proc. Natl. Acad. Sci. USA, 87, 4033-4037 (1990); Proc. Natl. Acad. Sci. USA, 88, 8850-8854 (1991); Hum. Gene Ther., 3, 147-154 (1991)).

Local incorporation and expression of a gene by tissues has been reported for a tumor treatment involving direct administration of a liposome preparation into the target tissue according to the membrane fusion-mediated transfer method that utilizes liposome (Hum. Gene Ther. 3, 399-410 (1992)). Thus, a similar effect may be expected for heart lesions. The technique of direct DNA incorporation is preferable to direct delivery of a DNA to the heart lesions. A receptor-mediated DNA transfer can be carried out, for example, with a protein ligand conjugated with the DNA (which is normally present as a covalently-closed supercoiled plasmid) via polylysine. The ligand is selected depending on the corresponding ligand receptor expressed on the surface of a target cell or cells in the tissue. Exemplary combinations of the receptor and ligand include, the combination of endothelin (ET)-1 receptor and ET-1. If desired, such a ligand-DNA conjugate can be injected directly to the blood vessel to reach a target tissue where the complex is bound to the receptor and internalized to the cells. To prevent intracellular DNA degradation, adenoviruses are co-infected with the DNA to disrupt the function of endosomes.

### 13. Therapeutic agents for heart diseases containing myocardial cell proliferation-associated protein:

A myocardial cell proliferation-associated protein of the present invention can be used for reconstructing the cardiac structure and function in various heart diseases caused by myocardial degeneration.

A therapeutic agent for heart diseases, which contains a myocardial cell proliferation-associated protein of the present invention, may comprises the protein alone as the active ingredient. Typically, it is preferable to provide it as a pharmaceutical composition that is prepared by mixing the protein with one or more pharmaceutically acceptable carriers by appropriate methods, which are well known to one skilled in the art of pharmaceutics.

Preferred routes of administration are those that are most effective for the therapy, and include oral administration and parenteral administration such as intraoral, tracheobronchial, intrarectal, subcutaneous, intramuscular, and intravenous administrations. For a protein preparation, intravenous administration is preferred.

The dosage forms of the present agents include nebula, capsule, tablet, granule, syrup, emulsion, suppository, injection, ointment, tape, etc.

Preparations suitable for oral administration include emulsion, syrup, capsule, tablet, powder, granule, etc. For example, liquid preparations such as emulsion and syrup can be prepared using, as an additive, water; sugars such as sucrose, sorbitol and fructose; glycols such as polyethylene glycol and propylene glycol; oils such as sesame oil, olive oil and soy bean oil; preservative such as p-hydroxybenzoic acid esters; flavors such as strawberry flavor and peppermint. Capsules, tablets, powders and granules can be produced using, as an additive, excipient such as lactose, glucose, sucrose and mannitol; disintegrator such as starch and sodium alginate; lubricant such as magnesium stearate and talc; binder such as polyvinyl alcohol, hydroxypropylcellulose, and gelatin; detergent such as fatty acid ester; and plasticizer such as glycerin.

Preparations suitable for parenteral administration include injection, suppository and nebula. An injection can be prepared using a carrier comprising salt solution, glucose solution, or a mixture thereof. A powder injection can be prepared by freeze-drying a protein of the present invention according to conventional methods and adding sodium chloride thereto. A suppository can be prepared using a carrier such as cacao butter, hydrogenated oil, and carboxylic acid.

Further, a nebula can be prepared from a protein of the present invention with or without a carrier or the like that has no irritating effect on recipient's oral and airway mucous membrane and allows dispersion of the protein of the present invention as a fine particle to enhance the absorption thereof.

Specific examples of such carriers are lactose and glycerin. Preparations such as aerosol and dry powder can be provided depending on the properties of the carriers and the protein of the present invention to be used. Further, the illustrated additives for the oral dosage forms can also be added as additives in these parenteral dosage forms.

While the dosage and administration frequency depend on the type of disease to be treated, method of administration, period of treatment, age, weight, etc., typically it is within the range of 10 µg/kg/day to 8 mg/kg/day for an adult individual.

### 14. Therapeutic agents for heart diseases containing antibody specifically recognizing myocardial cell proliferation-associated protein:

An antibody that specifically recognizes a myocardial cell proliferation-associated protein of the present invention can be used without any modification for treating heart diseases, etc.

A therapeutic agent containing an antibody that specifically recognizes a myocardial cell proliferation-associated protein of the present invention, may comprises the antibody alone as the active ingredient. Typically, it is preferable to provide it as a pharmaceutical, prepared by mixing the antibody with one or more pharmaceutically acceptable carriers by an appropriate method that is well known to one skilled in the art of pharmaceutics. The preparation and administration of the therapeutic agent can be carried out in the same way as for the therapeutic agent containing a myocardial cell proliferation-associated protein described above in Section 13.

The present invention is illustrated in detail below with reference to Examples.

### Brief Description of the Drawings

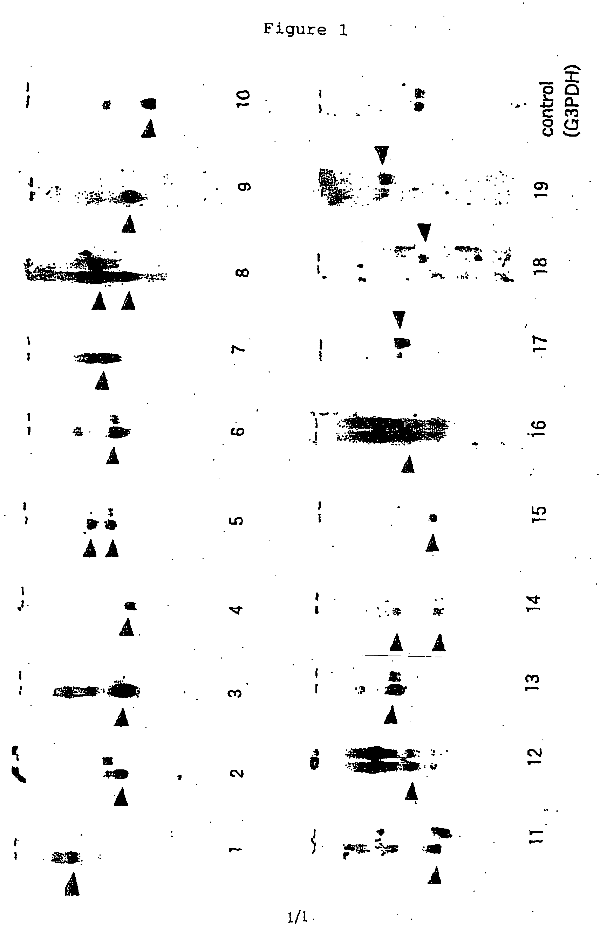

Figure 1 shows the results of Northern analysis for genes whose expression levels differ between the fetal and adult hearts. Panels 1 to 19 show the changes of the expression levels of RHDH-009, -063, -068, -098, -099, -231, -249, -274, -286, -057, -185, -226, -235, -239, -279-1, -309, -100, -140 and -093 between the fetal and adult hearts, respectively. In each blot, the left lane contained 12 µg total RNA from the heart of a 16-day-old fetal rat, and the right lane 12 µg total RNA from. the heart of an 8-week-old rat.

### Best Mode for Carrying out the Invention

### Example 1. Preparation of cDNA library from the heart of 16-day-old fetal rat

Heart was excised from a fetus of Wistar rat on the 16th day of pregnancy (Japan SLC), and total RNA was prepared therefrom by the guanidine thiocyanate-cesium trifluoroacetate method (Methods in Enzymology, 154, 3 (1987)). mRNA was obtained as poly(A)⁺ RNA by passing the total RNA through an oligo(dT) cellulose column (Collaborative Research). From the obtained mRNA, a cDNA library, which contained 1.0 x 10⁶ independent plaques, was prepared using ZAP-cDNA synthesis kit (ZAP-cDNA Synthesis Kit, Stratagene). The preparation of the cDNA library was conducted following the method described in the manual attached to the kit. In this cDNA library, a cDNA is inserted in λ phage vector λ ZAPII (Stratagene) at the *Xh*oI/*Eco*RI site so that the 5' end of the cDNA is ligated to the *Eco*RI site.

### Example 2. Preparation of subtracted library

### (1) Preparation of single-stranded DNA

Along with helper phage ExAssist (Stratagene), the cDNA library (in the form of λ phage) from rat heart on the 16th day of fetal period prepared in Example 1, was transfected to a host cell, *Escerichia coli* XL1-Blue MRF' (Stratagene). The portion of phagemid pBluescript SK(-) containing a cDNA was excised as a single-stranded DNA phage from the vector by *in vivo* excision. The single-stranded DNA phage was released to the culture supernatant. The *in vivo* excision was performed according to the manual from Stratagene. 700 µl of the culture supernatant (titer: 1.8 x 10⁵ cfu/µl) was added to 7 ml of 10 mM MgSO₄ solution containing 1.8 x 10¹⁰ cells of *Escherichia coli* SORL (Stratagene), an ExAssist-resistant host cell. The mixture was cultured at 37°C for 15 minutes, and then combined with 200 ml of 2 x YT culture medium (1.6% bactotryptone, 1% yeast extract). The mixture was cultured at 37°C for 45 minutes with shaking and the single-stranded DNA phages containing cDNA were infected to the bacterial cells. To the mixture, ampicillin was added at a final concentration of 50 µg/ml, and then culturing was continued at 37°C for one hour with shaking to proliferate phage-infected *E. coli* cells alone. The cell count was measured by absorbance at a wavelength of 600 nm. Since the cell count was 8.0 x 10¹⁰, helper phage R408 (Stratagene) was added to the culture at a multiplicity of infection (m.o.i.) of 10 (7.7 x 10¹¹ pfu), and the culture was incubated at 37°C for 7 hours with shaking. Again, the single-stranded DNAs were released to the supernatant. The culture liquid was transferred into a sterilized tube and centrifuged at 10,000 rpm at 4°C for 10 minutes. Only supernatants containing phages were recovered by transferring them into a fresh sterilized tube. After re-centrifugation under the same condition, the supernatant was filtered through a sterilizing filter with a pore size of 0.22 mm (Millipore) to completely remove cells. 20 ml of 10 x buffer (100 mM Tris-HCl (pH 7.5), 100 mM magnesium chloride) and 140 units of deoxyribonuclease I (Nippon Gene) were added to the supernatant. The mixture was incubated at 37°C for 30 minutes, and then 1/4 volume of 20% polyethylene glycol (molecular weight = 6000)-2.5 M sodium chloride was added thereto. The resulting mixture was mixed well and allowed to stand still at room temperature for 20 minutes. The mixture was centrifuged at 10,000 rpm at 4°C for 10 minutes to precipitate the phages. After completely removing the supernatant, the phage precipitated was suspended in 400 µl of TE (10 mM Tris-HCl (pH8.0), 1 mM EDTA (pH8.0)). 4 µl of 10% SDS and 625 µg (25 µl) of proteinase K were added to the suspension. Then, the suspension was incubated at 42°C for one hour. After phenol extraction, phenol-chloroform extraction, and chloroform extraction, the aqueous layer was subjected to ethanol precipitation, which gave 75.0 µg of the single-stranded DNA (vector pBluescript SK(-)) derived from the cDNA library from the heart of 16-day-old fetal rat.

### (2) Biotinylation of RNA

Poly(A)⁺ RNA was prepared from the heart of an 8-week-old rat by the method described in Example 1. 10 µg of the RNA and distilled water were combined in a test tube. The total volume of the solution was 20 µl. 30 µl of 1 µg/µl PHOTOPROBE biotin (Vector Laboratories) was added to the solution in dark. The test tube was uncapped and placed on ice, and then the RNA was biotinylated by irradiation with mercury-lamp light from a height of about 10 cm for 20 minutes.

50 µl solution of 100 mM Tris-HCl (pH 9.5) and 1 mM EDTA (pH 8.0) was added to the reaction solution. 100 µl of water-saturated butanol was added to the mixed solution and then the mixture was stirred vigorously. After the mixture was centrifuged at 14,000 rpm at 4°C for 5 minutes, the upper butanol layer was removed. The same treatment was repeated three times in total. 100 µl of chloroform was added to the aqueous layer and the mixture was stirred vigorously. After the mixture was centrifuged at 14,000 rpm at 4°C for 5 minutes, the aqueous layer was transferred into a fresh test tube. The treatment was repeated again, and then the RNA was ethanol-precipitated. The recovered RNA precipitate was dissolved in 20 µl of distilled water and the biotinylation treatment was repeated. The biotinylated RNA was stored at -80°C at the ethanol-precipitated state for later hybridization.

### (3) Subtraction

12.5 µl of 2 x reaction buffer (80% formamide, 100 mM HEPES (pH 7.5), 2 mM EDTA (pH 8.0), 0.2% SDS), 2.5 µl of 2.5 M sodium chloride, and 0.5 µg (1 µl) of poly(A) (Amersham Pharmacia Biotech) were added to 0.5 µg (1 µl) of the single-stranded DNA from the cDNA library from the heart of 16-day-old fetal rat prepared in (1). Further an aliquot (corresponding to 10 µg of RNA) of the biotinylated RNA prepared in (2), dissolved in 5 µl of distilled water, was added thereto. After heating at 65°C for 10 minutes, the mixture was incubated at 42°C for 2 nights for hybridization.

After the hybridization reaction, 400 µl of buffer (500 mM sodium chloride, 50 mM HEPES (pH 7.5), 2 mM EDTA (pH 8.0)) was added to the reaction solution and then 10 µg (5 µl) of streptavidin (Life Technologies) was added thereto. The mixture was incubated at room temperature for 5 minutes. The complex of streptavidin and biotinylated RNA-cDNA hybrid was removed from the water layer by phenol-chloroform extraction. Again, 10 µg of streptavidin was added to the aqueous layer and the mixture was incubated at room temperature for 5 minutes. After conducting phenol-chloroform extraction twice, the sample was treated with chloroform extraction. The aqueous layer was recovered, and then filtered through a unit-filter ultra-free C3 plus TK (Millipore) to adsorb the cDNA on the filter. After washing, the cDNA was eluted from the filter with 30 µl of 1/10 TE (1 mM Tris-HCl (pH 8.0), 0.1 mM EDTA (pH 8.0)) to concentrate and desalt the cDNA. The treatment with the filter was carried out according to the manual from Millipore.

### (4) Synthesis and introduction into E. coli of double-stranded DNA

14 µl of distilled water and 1 µl of primer extension primer (2 µg/µl) having the nucleotide sequence of SEQ ID NO: 42 were added to a 15-µl aliquot from the 30 µl of subtracted single-stranded DNA obtained as described above. The mixture was heated at 65°C for 10 minutes. After annealing of the primer to the single-stranded DNA by allowing the mixture to stand still at room temperature for 5 minutes, 5 µl of 10x reaction buffer (which was attached to BcaBEST Dideoxy Sequencing Kit; Takara Shuzo), 10 µl of 1 mM dNTP mixture, 0.5 µl of 3 µg/µl single-stranded DNA binding protein (USB), 2 µl of 2 units/µl BcaBEST DNA polymerase (Takara Shuzo), and 2.5 µl of distilled water were added thereto. Double-stranded DNA was synthesized by incubating the mixture at 65°C for one hour. 60 µl of distilled water was added to the reaction solution and the solution was subjected to phenol-chloroform extraction, followed by chloroform extraction. The solution was concentrated with unit-filter ultra-free C3 plus TK by the same method as in (3), and finally the double-stranded DNA was dissolved in 20 µl of TE. A 1/5 aliquot of the double-stranded DNA was introduce into *E. coli* XL-1 Blue MRF' by electroporation to prepare a cDNA library (subtracted cDNA library).

### Example 3. Differential hybridization

### (1) Preparation of array filter

Colonies were formed on LB-Ap agar medium using the subtracted cDNA library prepared in (4) of Example 2. 9,600 colonies thereof were inoculated on 103 plates with 96 wells each of which contained 100 µl of LB-Ap culture medium. Each colony was inoculated in a single well of the 96-well plates and cultured at 37°C, and then 75 µl of 50% glycerol was added thereto. The cultures were stored at -80°C (this culture liquid for storage is called "glycerol stock").

Using a 96-pin replicator, the bacteria were inoculated again from the glycerol stocks to wells of 96-well plates, which contained 100 µl of LB-Ap culture medium in each well. The bacteria were grown at 37°C overnight by allowing them to stand still. 20-µl aliquots of following reaction solution were added to 96-well PCR plates using automatic dispenser Hydra96, and then trace amounts of the liquids of overnight culture containing *E. coli* were added thereto. The PCR solution contained 2 µl of 10x reaction buffer (attached to ExTaq), 2 µl of 2.5 mM dNTP, 1 µl of 10 µM T3 HT primer (SEQ ID NO: 39), 1 µl of 10 µM T7 primer (SEQ ID NO: 40), 13.8 µl of distilled water and 0.2 µl of Taq DNA polymerase ExTaq (Takara Shuzo). PCR was performed in a thermal cycler and the profile of thermal cycling was: preheating at 94°C for 5 minutes; 30 cycles of denaturation at 94°C for 1 minute, annealing at 64°C for 1 minute, and extension at 72°C for 1 minute. The reaction solutions were stored at 4°C. The T3 HT primer and T7 primer were designed based on the specific vector sequences flanking the cDNA insert in order to amplify the cDNA portion.

A 0.5-µl aliquot of each reaction solution was spotted onto NYLON TRANSFER MEMBRANE Hybond N⁺ (Amersham Pharmacia Biotech). The spots were arranged into a lattice-shaped configuration in the same way as on the 96-well plate (12 spots by 8 spots) . 384 colonies, which corresponded to four 96-well plates, were spotted onto one sheet of nylon membrane into a lattice-shaped configuration (24 spots by 16 spots). The PCR solution derived from a single colony was spotted onto two sheets of membrane at corresponding positions to obtain the DNA-spotted membranes in duplicate. The membranes on which the reaction solutions had been spotted were air-dried at room temperature, and then placed on paper filters, which had been soaked in denaturation solution (0.5 M NaOH, 1.5 M sodium chloride). The membranes were allowed to stand still at room temperature for 10 minutes. After the DNA was denatured, the membranes were transferred onto paper filters, which had been soaked in neutralization solution (1.0 M Tris-HCl (pH7.5), 1.5 M sodium chloride) and allowed to stand still at room temperature for 10 minutes. The membranes (array filter) were washed in a square dish filled with sufficient amount of 2x SSC (0.3 M sodium chloride, 30 mM sodium citrate) containing 0.5% SDS.

### (2) Probe labeling

Using Label IT Digoxin Nucleic Acid Labeling Kit (hereinafter referred to as "Label IT Kit"; Mirus), digoxigenin (DIG)-labeled probes were prepared from poly(A)⁺ RNAs obtained from the heart of a 16-day-old fetal rat and the heart from an 8-week old rat prepared in Examples 1 and 2(2). The labeling was carried out according to the manual attached to the kit.

### (3) Hybridization

The methods of hybridization and detection of the hybridized spots, as well as the reagents used therein, were in accordance with the DIG system users' guide from Roche.

The membranes prepared in (1) were placed in a hybridization bag, and then 20 ml of a hybridization buffer (5x SSC, 0.1% N-lauroylsarcosine, 0.02% SDS, 2% blocking reagent (Roche), 50% formamide) , which had been preheated at 50°C, was added thereto. The pre-hybridization was carried out at 50°C for 4 hours. 1/10 volume of denaturation buffer (attached to the Label IT Kit; Mirus) was added to the probe prepared in (2) and incubated at room temperature for 5 minutes. Then 1/10 volume of neutralization buffer (attached to the Label IT Kit; Mirus) was also added thereto. The denatured probe was combined with the hybridization buffer and the mixture was added to the hybridization bag containing the membranes after the pre-hybridization. The bag was incubated for hybridization at 50°C overnight with shaking so that the filters move in the bag (about 12 rpm). As noted above, the membranes were prepared in duplicate in (1) and thus comprise the same DNA spots. One of the two was hybridized with the probe from the heart of the 16-day old fetal rat, and the other with the probe from the heart of the 8-week-old rat.

### (4) Spot detection

The membranes were taken from the hybridization bag, and then washed with 2x SSC-0.1% SDS at 68°C for 10 minutes. The membranes were washed again with fresh washing solution under the same condition. Washing was further repeated twice with 0.1x SSC-0.1% SDS at 68°C for 15 minutes. Then, the membranes were treated with DIG luminescence detection kit (comprising alkaline phosphatase-conjugated anti-DIG antibody and chemiluminescence substrate CSPD (Roche)). X-ray films Hyper Film ECL (Amersham Pharmacia Biotech) were exposed to the luminescent light on the membranes. The films were then developed. The period of exposure was controlled so to reach a similar background level with the probe from the heart of the 16-day old fetal rat and with that of the 8-week-old rat. Clones which exhibited more intense hybridization signals with the probe from the 8-week-old rat heart than with the probe from the heart of the 16-day-old fetal rat, and clones which exhibited more intense hybridization signals with the probe from the heart of the 16-day-old fetal rat than with the probe from the 8-week-old rat heart, were selected. The number of the selected clones was 316 in total. The clones were assigned based on the addresses on the array. Plasmid DNAs of the respective clones were prepared from the cultures obtained from the glycerol stocks prepared in (1) of Example 3.

### Example 4. Analysis of each clone

### (1) Determination of nucleotide sequences

The nucleotide sequences of cDNAs of the 316 clones selected by differential hybridization in Example 3 were determined with a DNA sequencer. These nucleotide sequences were searched for homology against the nucleotide sequences in databases GenBank, EMBL and GeneSeq (Derwent) using analysis program BlastN. The analysis result demonstrated that known genes, genes encoding slow-fiber troponin I, non-muscle myosin alkali light chain, vimentin and elongation 1α, were contained among genes that were expressed at higher levels in the fetal heart than in the adult heart.

Next, the entire nucleotide sequences of cDNAs were also determined for the genes whose expression levels were verified to be higher in the heart of the 16-day-old fetal rat as compared with those in the heart of the 8-week-old rat and the genes whose expression levels were verified to be higher in the heart of the 8-week-old rat as compared with those in the heart of the 16-day-old fetal rat by Northern hybridization described below in (2). The amino acid sequences of the proteins encoded by the cDNAs were deduced from the determined nucleotide sequences. Further, these amino acid sequences were also searched for homology against the amino acid sequences in databases SwissProt, PIR, GenPept, TREMBL and GeneSeq using analytical program Blast.

### (2) Analysis of differences in expression levels by Northern hybridization

Clones that seemed to be interesting were selected from the 316 clones isolated in (1). These clones were selected so as to mainly including those having novel nucleotide sequences. Each of the genes was analyzed by Northern hybridization to compare their expression levels in the heart between the 16-day-old fetal rat and the 8-week-old rat.

### (2)-1 RNA transfer onto membrane

Distilled water was added to 12 µg of total RNA obtained from the heart of 16-day-old fetal rat or heart from 8-week-old rat by the same method as in Example 1 to a total volume of 3.5 µl. 1.5 µl of 10x MOPS buffer (80 mM sodium acetate, 197 mM MOPS, 10 mM EDTA (pH 8.0)), 2 µl of 35% formaldehyde solution (Nacalai Tesque), and 5 µl of deionized formamide were added to the RNA solution. After heating at 65°C for 5 minutes, the mixture was cooled rapidly on ice. The whole quantity was used for electrophoresis on a 1% agarose gel containing 1x MOPS and 2% formaldehyde. After the electrophoresis, the RNA on the gel was transferred onto NYLON TRANSFER MEMBRANE Hybond N⁺ (Amersham Pharmacia Biotech) by capillary transfer using 20x SSC (3 M sodium chloride, 0.3 M sodium citrate). After the transfer, the RNA was immobilized on the membrane by ultraviolet irradiation in a Cross-Linker Optimal Link (Funakoshi).

### (2)-2 Probe labeling

The selected clones were double-digestion with *Apa*I and *Pst*I to cut the insert DNA fragments out. The fragments were purified with QIAEX II Gel Extraction Kit (Qiagen) according to the method described in the manual attached to the kit. The DNA fragments were labeled by DIG-High Prime (Roche) using the purified DNA fragments as templates. The labeled DNAs were used as probes. The labeling was conducted in accordance with the manual attached to the kit.

### (2)-3 Hybridization and autoradiography

Methods of hybridization and for detecting the hybridized spots as well as the reagents used were in accordance with the DIG system users' guide from Roche.

The membrane prepared in (1) was placed in a hybridization bag, and then hybridization buffer containing SDS at a high concentration (5x SSC, 0.1% lauroylsarcosyl, 7% SDS, 50 mM sodium phosphate buffer (pH7.0), 50% formamide, 2% blocking reagent (Roche)) preheated at 50°C was added thereto. The bag was incubated at 50°C for several hours or longer for prehybridization. The probe prepared in (2) was denatured by heating at 95°C for 5 minutes and then was cooled rapidly. The denatured probe was mixed with the hybridization buffer, and the mixture was added to the hybridization bag containing the membranes on which pre-hybridization had been terminated. The bag was incubated for hybridization at 50°C overnight. The membrane was taken from the hybridization bag, and then washed with 2x SSC-0.1% SDS at 68°C for 10 minutes. The membrane was washed again with fresh washing solution under the same condition. Further, washing was repeated twice with 0.1x SSC-0.1% SDS at 68°C for 15 minutes. Then, the membrane was treated with DIG luminescence detection kit, which contains alkaline phosphatase-conjugated anti-DIG antibody and chemiluminescence substrate CSPD (Roche). Then X-ray film Hyper Film ECL (Amersham Pharmacia Biotech) was exposed to the luminescent light on the membrane for autoradiography.

16 clones whose expression levels were higher in the heart of the 16-day-old fetal rat than in the heart of the 8-week-old rat were obtained: RHDH-009, RHDH-063, RHDH-068, RHDH-098, RHDH-099, RHDH-231, RHDH-249, RHDH-274, RHDH-286, RHDH-057, RHDH-185, RHDH-226, RHDH-235, RHDH-239, RHDH-279, and RHDH-309. Three clones whose expression levels were higher in the heart of the 8-week-old rat than in the heart of the 16-day-old fetal rat were obtained: RHDH-100, RHDH-140 and RHDH-093. These clones are described below.

### (3) Known genes whose expression levels are higher in the heart of the 16-day-old fetal rat than in the heart of the 8-week-old rat

The nucleotide sequence of RHDH-009 was identical with that of rat insulin-like growth factor II (IGF-II) [Accession: X13101] (SEQ ID NO: 1). The amino acid sequence encoded by the gene is shown in SEQ ID NO: 2. IGF-II has been known as a cell-growth factor, but little is known about its physiological function. The result of Northern blotting carried out on the hearts of the 16-day-old fetal rat and the 8-week-old rat is shown in panel 1 of Figure 1.

The nucleotide sequence of RHDH-063 (SEQ ID NO: 3) exhibited a high (76%) homology to that of the gene encoding a presumptive human secretory protein [Interantional Patent application: WO 99/3126]. Accordingly, RHDH-063 was estimated to be the rat orthologue thereof. The amino acid sequence encoded by the nucleotide sequence of SEQ ID NO: 3 is shown in SEQ ID NO: 4. The protein encoded by the gene exhibited no marked homology to other known proteins, and its function still remains unclear. The result of Northern blotting carried out on the hearts of the 16-day-old fetal rat and the 8-week-old rat is shown in panel 2 of Figure 1.

The nucleotide sequence of RHDH-068 was identical with that of rat melanocyte-specific expression gene 1 (*msg1*) [Accession: AF104399] (SEQ ID NO: 5). The amino acid sequence encoded by the gene is shown in SEQ ID NO: 6. The *msg1* gene has been suggested to participate in cellular pigmentation [Proc. Natl. Acad. Sci. USA, 93, 12298-12303 (1996)]. However, its function in heart remains to be confessed. The result of Northern blotting carried out on the hearts of the 16-day-old fetal rat and the 8-week-old rat is shown in panel 3 of Figure 1.

The nucleotide sequence of RHDH-098 exhibited high homology to that of mouse *c-abl* [Accession: L10656] (SEQ ID NO: 7). The amino acid sequence encoded by the gene is shown in SEQ ID NO: 8. The product of *c-abl* gene has a tyrosine kinase activity. The result of Northern blotting carried out on the hearts of the 16-day-old fetal rat and the 8-week-old rat is shown in panel 4 of Figure 1.

The nucleotide sequence of RHDH-099 was identical with that of rat non-neuronal enolase [Accession: X02610] (SEQ ID NO: 9). The amino acid sequence encoded by the gene is shown in SEQ ID NO: 10. The non-neuronal enolase gene encodes an enzyme member of the glycolytic system. However, the existence of the difference in the expression level of this gene between the fetal and adult hearts was unknown. The result of Northern blotting carried out on the hearts of the 16-day-old fetal rat and the 8-week-old rat is shown in panel 5 of Figure 1.

The nucleotide sequence of RHDH-231 was identical with that of rat receptor-linked tyrosine phosphatase (PTP-P1) [Accession: L19180] (SEQ ID NO: 11). The amino acid sequence encoded by the gene is shown in SEQ ID NO: 12. The product of this gene has a tyrosine phosphatase activity. The result of Northern blotting carried out on the hearts of the 16-day-old fetal rat and the 8-week-old rat is shown in panel 6 of Figure 1.

The nucleotide sequence of RHDH-249 was identical with that of rat *TSC-22* [Accession: L25785] (SEQ ID NO: 13). The amino acid sequence encoded by the gene is shown in SEQ ID NO: 14. It has been reported that the expression of the rat *TSC-22* gene is induced by TGF-β [J. Biol. Chem., 267, 10219 (1992)]. However, its function still remains unclear. The result of Northern blotting carried out on the hearts of the 16-day-old fetal rat and the 8-week-old rat is shown in panel 7 of Figure 1.

The nucleotide sequence of RHDH-274 was identical with that of rat *SH3p8* [Accession: AB008161] (SEQ ID NO: 15). The amino acid sequence encoded by the gene is shown in SEQ ID NO: 16. The product of the SH3p8 gene has a Src homology 3 (SH3) domain and an activity to bind to synaptojanin or dynamin I [Proc. Natl. Acad. Sci. USA, 94, 8569-8574 (1997)]. The result of Northern blotting carried out on the hearts of the 16-day-old fetal rat and the 8-week-old rat is shown in panel 8 of Figure 1.

The nucleotide sequence of RHDH-286 (SEQ ID NO: 17) exhibited high (91%) homology to that of mouse retinoic acid-response protein (MK) [Accession: M35833]. Accordingly, RHDH-286 was estimated to be the rat orthologue of the mouse MK gene. The amino acid sequence encoded by the nucleotide sequence of SEQ ID NO: 17 is shown in SEQ ID NO: 18. It has been known that the expression of the MK gene product is induced at early stages during differentiation of embryonic tumor cells by retinoic acid [Biochem. Biophys. Res. Commun., 151, 1312-1318 (1988)]. The result of Northern blotting carried out on the hearts of the 16-day-old fetal rat and the 8-week-old rat is shown in panel 9 of Figure 1.

### (4) Novel genes with higher expression level in the heart of the 16-day-old fetal rat than in the heart of the 8-week-old rat

The nucleotide sequence of RHDH-057 is shown in SEQ ID NO: 19. According to the result of homology analysis, no identical sequence identical to this nucleotide sequence could be found, and thus it was concluded to be a novel sequence. The sequence of RHDH-057 was partially shared by ESTs in UniGene Rn.7790, but no sequence completely covering the nucleotide sequence of RHDH-057 could be found. The amino acid sequence encoded by RHDH-057 is shown in SEQ ID NO: 20. The result of Northern blotting carried out on the hearts of the 16-day-old fetal rat and the 8-week-old rat is shown in panel 10 of Figure 1.

The nucleotide sequence of RHDH-185 is shown in SEQ ID NO: 21. According to the result of homology analysis, no known genes were found to have a sequence identical to this nucleotide sequence, and thus it was concluded to be a novel sequence. The sequence of RHDH-185 was partially shared by ESTs in UniGene Rn.12591; however, no sequence was found that completely covered the nucleotide sequence of RHDH-185. The *E. coli* XL1-Blue MRF'/pRHDH185 strain that contains the plasmid pRHDH-185 containing the cDNA RHDH-185 has been deposited, under the Budapest Treaty, in the following international depositary authority under the accession number FERM BP-7081 on March 10, 2000: International Patent Organism Depositary, National Institute of Advanced Industrial Science and Technology (AIST), Independent Administrative Institution: Chuo 6, 1-1-1 Higashi, Tsukuba, Ibaraki, Japan (Previous Name: The National Institute of Bioscience and Human-Technology, The Agency of.Industrial Science and Technology: 1-1-3 Higashi, Tsukuba, Ibaraki, Japan). An ORF of 109 amino acids was found in the nucleotide sequence of RHDH-185. The amino acid sequence is shown in SEQ ID NO: 22. The result of Northern blotting carried out on the hearts of the 16-day-old fetal rat and the 8-week-old rat is shown in panel 11 of Figure 1.

The nucleotide sequence of RHDH-226 is shown in SEQ ID NO: 23. According to the result of homology analysis, no known genes were found to have a sequence identical to this nucleotide sequence, and thus, it was concluded to be a novel sequence. The sequence of RHDH-226 was partially shared by ESTs in UniGene Rn.7270; however, no sequence was found that completely covered the nucleotide sequence of RHDH-226. The *E. coli* XL1-Blue MRF'/pRHDH226 strain that contains the plasmid pRHDH-226 containing the cDNA RHDH-226 has been deposited, under the Budapest Treaty, in the following international depositary authority under the accession number FERM BP-7082 on March 10, 2000: International Patent Organism Depositary, National Institute of Advanced Industrial Science and Technology (AIST), Independent Administrative Institution: Chuo 6, 1-1-1 Higashi, Tsukuba, Ibaraki, Japan (Previous Name: The National Institute of Bioscience and Human-Technology, The Agency of Industrial Science and Technology: 1-1-3 Higashi, Tsukuba, Ibaraki, Japan).

An ORF of 376 amino acids was found in the nucleotide sequence of RHDH-226. The amino acid sequence is shown in SEQ ID NO: 24. Sequences in databases were searched for homology to this amino acid sequence. The result showed that the region of residues 1 to 273 in the amino acid sequence of SEQ ID NO: 24 exhibited high (88%) homology to the amino acid sequence of a putative human secretory protein disclosed in WO 99/06423. However, no amino acid sequence that corresponds to the amino acid sequence of residues 274 to 376 in SEQ ID NO: 24 was found for the protein shown in WO99/06423. Thus, the protein disclosed in WO 99/06423 seems not to be the human orthologue of the protein comprising the amino acid sequence of SEQ ID NO: 24. Furthermore, no functional information on the protein disclosed in WO 99/06423 is available, except that it is a secretory factor. The result of Northern blotting carried out on the hearts of 16-day-old fetal rat and 8-week-old rat is shown in panel 12 of Figure 1.

The nucleotide sequence of RHDH-235 is shown in SEQ ID NO: 25. According to the result of homology analysis, this sequence exhibited 65% homology to that of the human metastasis-associated gene 1 (*mta1*) [Accession: U35113] and 64% homology to that of rat *mta1*, which is the rat orthologue thereof [Accession: U02522]. An ORF of 513 amino acids is found in the nucleotide sequence of RHDH-235. The amino acid sequence is shown in SEQ ID NO: 26. The amino acid sequence of SEQ ID NO: 26 exhibited 74% homology to the amino acid sequence of the protein encoded by the human *mta1* gene and 73% homology to that encoded by the rat *mta1* gene. Since the amino acid sequence of SEQ ID NO: 26 was not identical to neither that of human *mta1* nor that of rat *mta1*, RHDH-235 was assumed to be a novel gene. The *E. coli* XL1-Blue MRF'/pRHDH235 strain that contains the plasmid pRHDH-235 containing the cDNA RHDH-235 has been deposited, under the Budapest Treaty, in the following international depositary authority under the accession number FERM BP-7083 on March 10, 2000: International Patent Organism Depositary, National Institute of Advanced Industrial Science and Technology (AIST), Independent Administrative Institution: Chuo 6, 1-1-1 Higashi, Tsukuba, Ibaraki, Japan (Previous Name: The National Institute of Bioscience and Human-Technology, The Agency of Industrial Science and Technology: 1-1-3 Higashi, Tsukuba, Ibaraki, Japan). The result of Northern blotting carried out on the hearts of the 16-day-old fetal rat and the 8-week-old rat is shown in panel 13 of Figure 1.

The nucleotide sequence of RHDH-239 is shown in SEQ ID NO: 27. According to the result of homology analysis, no known genes were found to have a sequence identical to this nucleotide sequence, and thus it was concluded to be a novel sequence. The sequence of RHDH-239 was partially shared by ESTs in UniGene Rn.23890, but no sequence was found that completely covered the nucleotide sequence of RHDH-239. The *E*. *coli* XL1-Blue MRF' /pRHDH239 that contains the plasmid pRHDH-239 containing the cDNA RHDH-239 has been deposited, under the Budapest Treaty, in the following international depositary authority under the accession number FERM BP-7084 on March 10, 2000: International Patent Organism Depositary, National Institute of Advanced Industrial Science and Technology (AIST), Independent Administrative Institution: Chuo 6, 1-1-1 Higashi, Tsukuba, Ibaraki, Japan (Previous Name: The National Institute of Bioscience and Human-Technology, The Agency of Industrial Science and Technology: 1-1-3 Higashi, Tsukuba, Ibaraki, Japan). An ORF of 158 amino acids was found in the nucleotide sequence of RHDH-239. The amino acid sequence is shown in SEQ ID NO: 28. The result of Northern blotting carried out on the hearts of the 16-day-old fetal rat and the 8-week-old rat is shown in panel 14 of Figure 1.

The nucleotide sequence of RHDH-279 is shown in SEQ ID NO: 29. According to the result of homology analysis, this sequence exhibited high (92%) homology to that of mouse interferon regulatory factor 3 (*mirf3*) [Accession: U75840]. A comparison of the nucleotide sequence of SEQ ID NO: 29 and the nucleotide sequence of the *mirf3* gene suggested that a part corresponding to the 5' end of full-length cDNA might be missing in this clone. Thus, a cDNA fragment further extending to the 5' direction of the cloned cDNA was amplified and isolated by PCR using primers specific to the vector sequence (SEQ ID NO: 39) and specific to RHDH-279 (SEQ ID NO: 41) and the rat cDNA library from the heart of the 16-day-old fetal rat as a template, which was prepared in Example 1 using λZAPII vector.

The RHDH-279-1 clone was obtained by assembling the cDNA fragment and the RHDH-279 cDNA. The nucleotide sequence of RHDH-279-1 is shown in SEQ ID NO: 30. The *E. coli* XL1-Blue MRF'/pRHDH279-1 that contains the plasmid pRHDH-279-1 containing the cDNA RHDH-279-1 has been deposited, under the Budapest Treaty, in the following international depositary authority under the accession number FERM BP-7085 on March 10, 2000: International Patent Organism Depositary, National Institute of Advanced Industrial Science and Technology (AIST), Independent Administrative Institution: Chuo 6, 1-1-1 Higashi, Tsukuba, Ibaraki, Japan (Previous Name: The National Institute of Bioscience and Human-Technology, The Agency of Industrial Science and Technology: 1-1-3 Higashi, Tsukuba, Ibaraki, Japan) . The sequence segment after the 30th residue in the nucleotide sequence of SEQ ID NO: 30 exhibited high (92%) homology to *mirf3*. However, the segment of residues 1 to 29 of the nucleotide sequence of SEQ ID NO: 30 was quite different from the sequence of *mirf3*.

Thus, the nucleotide sequence of SEQ ID NO: 30 is assumed not to be merely the rat orthologue of *mifr3.* An ORF of 94 amino acids is found in the nucleotide sequence of SEQ ID NO: 30. The amino acid sequence is shown in SEQ ID NO: 31. The result of Northern blotting carried out on the hearts of the 16-day-old fetal rat and the 8-week-old rat is shown in panel 15 of Figure 1.

The nucleotide sequence of RHDH-309 is shown in SEQ ID NO: 32. According to the result of homology analysis, no sequence of known genes identical to this nucleotide sequence was found, and thus it was concluded to be a novel sequence. The sequence of RHDH-309 was partially shared by ESTs in UniGene Rn.1779, but no sequence completely covering to the nucleotide sequence of RHDH-309 could be found. This sequence had no ORF consisting of 100 amino acids or more, and therefore it was assumed to be a noncoding region. The result of Northern blotting carried out on the hearts of the 16-day-old fetal rat and the 8-week-old rat is shown in panel 16 of Figure 1.

### (5) Known genes with higher expression level in the heart of the 8-week-old rat than in the heart of the 16-day-old fetal rat

The nucleotide sequence of RHDH-100 was identical with that of rat protein kinase C receptor [Accession: U03390] (SEQ ID NO: 33).

The amino acid sequence encoded by the gene is shown in SEQ ID NO: 34. The result of Northern blotting carried out on the hearts of the 16-day-old fetal rat and the 8-week-old rat is shown in panel 17 of Figure 1.

The nucleotide sequence of RHDH-140 (SEQ ID NO: 35) exhibited high (92%) homology to that of mouse pigment epithelium-derived factor (PEDF) [Accession: AF017057]. Thus, RHDH-140 was estimated to be the rat orthologue of the mouse PEDF gene. The amino acid sequence encoded by the nucleotide sequence of SEQ ID NO: 35 is shown in SEQ ID NO: 36. The result of Northern blotting carried out on the hearts of the 16-day-old fetal rat and the 8-week-old rat is shown in panel 18 of Figure 1.

### (6) Novel genes with higher expression level in the heart of the 8-week-old rat than in the heart of the 16-day-old fetal rat

The nucleotide sequence of RHDH-093 is shown in SEQ ID NO: 37. According to the result of homology analysis, no known genes were found having a sequence identical to this nucleotide sequence, and thus, it was concluded to be a novel sequence. The sequence of RHDH-093 was partially shared by ESTs in UniGene Rn.16542, but no sequence was found to completely cover the nucleotide sequence of RHDH-093. The amino acid sequence encoded by the nucleotide sequence of RHDH-093 is shown in SEQ ID NO: 38. The result of Northern blotting carried out on the hearts of the 16-day-old fetal rat and the 8-week-old rat is shown in panel 19 of Figure 1.

### Industrial Applicability

Diagnostic agents and therapeutic agents for various heart diseases caused by myocardial necrosis, for example, hypercardia and cardiac failure, are provided.

### "Sequence Listing Free Text"

SEQ ID NO: 39 - Description of artificial sequence: synthetic DNA
SEQ ID NO: 40 - Description of artificial sequence: synthetic DNA
SEQ ID NO: 41 - Description of artificial sequence: synthetic DNA
SEQ ID NO: 42 - Description of artificial sequence: synthetic DNA

## Claims

1. A DNA comprising the nucleotide sequence selected from the group consisting of the nucleotide sequences represented by SEQ ID NOs: 21, 23, 25, 27, and 30.

2. A DNA of a gene that hybridizes, under stringent conditions, to a DNA consisting of the nucleotide sequence represented by SEQ ID NO: 21 or 27, and whose expression level varies between fetal heart and adult heart.

3. A DNA of a gene that hybridizes under stringent conditions to a DNA consisting of the nucleotide sequence represented by SEQ ID NO: 23, 25 or 30, having a 90% or higher homology to the DNA, and whose expression level differs between fetal heart and adult heart.

4. A DNA comprising a sequence that is identical to 5 to 60 consecutive nucleotide residues of the nucleotide sequence selected from the group consisting of the nucleotide sequences represented by SEQ ID NOs: 21, 23, 25, 27 and 30.

5. A DNA comprising a sequence complementary to the DNA according to claim 4.

6. A method for detecting mRNA corresponding to a gene whose expression level varies between fetal heart and adult heart using the DNA according to any one of claims 1 to 5.

7. A diagnostic agent for heart diseases caused by myocardial degeneration, which agent comprises the DNA according to any one of claims 1 to 5.

8. A method for detecting a causative gene of a heart disease caused by myocardial degeneration using the DNA according to any one of claims 1 to 5.

9. A method of screening for a substance suppressing or enhancing transcription or translation of a gene whose expression level varies between fetal heart and adult heart using the DNA according to any one of claims 1 to 5.

10. A method of screening for a therapeutic agent of a heart disease caused by myocardial degeneration using the DNA according to any one of claims 1 to 5.

11. A therapeutic agent for a heart disease caused by myocardial degeneration, wherein the agent comprises the DNA according to any one of claims 1 to 5.

12. A recombinant viral vector comprising the DNA according to any one of claims 1 to 5.

13. A recombinant viral vector comprising an RNA having a sequence homologous to the sense strand of the DNA according to any one of claim 1 to 5.

14. A DNA having the nucleotide sequence selected from the group consisting of the nucleotide sequences represented by SEQ ID NOs: 19, 32, and 37.

15. A DNA of a gene hybridizing under stringent conditions to the DNA according to claim 14, and whose expression level varies between fetal heart and adult heart.

16. A DNA comprising a sequence that is identical to 5 to 60 consecutive nucleotide residues of the nucleotide sequence selected from the group consisting of the nucleotide sequences represented by SEQ ID NOs: 19, 32, and 37.

17. A DNA comprising a sequence complementary to the DNA of claim 16.

18. A diagnostic agent for a heart disease caused by myocardial degeneration, wherein the agent comprises the DNA according to any one of claims 14 to 16.

19. A method for detecting a causative gene of a heart disease caused by myocardial degeneration using the DNA according to any one of claims 14 to 16.

20. A method of screening for a substance suppressing or enhancing transcription or translation of a gene whose expression level varies between fetal heart and adult heart using the DNA according to any one of claims 14 to 16.

21. A method of screening for a therapeutic agent of a heart disease caused by myocardial degeneration using the DNA according to any one of claims 14 to 16.

22. A method for detecting mRNA corresponding to a gene whose expression level varies between fetal heart and adult heart using a DNA having the nucleotide sequence selected from the group consisting of the nucleotide sequences represented by SEQ ID NOs: 1, 3, 5, 7, 9, 11, 13, 15, 17, 33, and 35.

23. A diagnostic agent for a heart disease caused by myocardial degeneration, wherein the agent comprises a DNA comprising the nucleotide sequence selected from the group consisting of the nucleotide sequences represented by SEQ ID NOs: 1, 3, 5, 7, 9, 11, 13, 15, 17, 33, and 35.

24. A method of screening for a substance suppressing or enhancing transcription or translation of a gene whose expression level varies between fetal heart and adult heart using a DNA comprising the nucleotide sequence selected from the group consisting of the nucleotide sequences represented by SEQ ID NOs: 1, 3, 5, 7, 9, 11, 13, 15, 17, 33, and 35.

25. A method of screening for a therapeutic agent of a heart disease caused by myocardial degeneration using a DNA comprising the nucleotide sequence selected from the group consisting of the nucleotide sequences represented by SEQ ID NOs: 1, 3, 5, 7, 9, 11, 13, 15, 17, 33, and 35.

26. A therapeutic agent for a heart disease caused by myocardial degeneration, wherein the agent comprises a DNA comprising the nucleotide sequence selected from the group consisting of the nucleotide sequences represented by SEQ ID NOs: 1, 3, 5, 7, 9, 11, 13, 15, 17, 33, and 35.

27. A recombinant viral vector comprising a DNA having the nucleotide sequence selected from the group consisting of the nucleotide sequences represented by SEQ ID NOs: 1, 3, 5, 7, 9, 11, 13, 15, 17, 33, and 35.

28. A recombinant viral vector comprising an RNA having a sequence homologous to the sense strand of a DNA comprising the nucleotide sequence selected from the group consisting of the nucleotide sequences represented by SEQ ID NOs: 1, 3, 5, 7, 9, 11, 13, 15, 17, 33, and 35.

29. A protein comprising the amino acid sequence selected from the group consisting of the amino acid sequences represented by SEQ ID NOs: 22, 24, 26, 28, and 31.

30. A protein having an amino acid sequence wherein one or more amino acids are deleted, substituted or added in the amino acid sequence selected from the group consisting of the amino acid sequences represented by SEQ ID NOs: 22, 24, 26, and 28, and which has an activity related to the healing of a heart disease caused by myocardial degeneration.

31. A DNA encoding the protein according to claim 29 or 30.

32. A recombinant DNA that is obtained by inserting the DNA according to any one of claims 1 to 4, and 31 into a vector.

33. A transformant obtained by introducing the recombinant DNA according to claim 32 into a host cell.

34. A method for producing the protein, comprising the steps of culturing the transformant according to claim 33, producing and accumulating the protein according to claim 29 or 30 in the culture, and recovering the protein from the culture.

35. A therapeutic agent for a heart disease caused by myocardial degeneration, which agent comprises the protein according to claim 29 or 30.

36. A method of screening for a therapeutic agent for a heart disease caused by myocardial degeneration comprising the steps of culturing the transformant according to claim 33, and screening the agent using the obtained culture.

37. A method of screening for a therapeutic agent for a heart disease caused by myocardial degeneration using the protein according to claim 29 or 30.

38. A recombinant viral vector associated with the production of the protein according to claim 29 or 30.

39. A therapeutic agent for a heart disease caused by myocardial degeneration, wherein the agent comprises the recombinant viral vector according to claim 38.

40. An antibody recognizing the protein according to claim 29 or 30.

41. An immunological method for detecting the protein of claim 29 or 30 using the antibody according to claim 40.

42. A method of screening for a therapeutic agent for a heart disease caused by myocardial degeneration using the antibody according to claim 40.

43. A method of screening for a substance suppressing or enhancing transcription or translation of a gene whose expression level varies between fetal heart and adult heart using the antibody according to claim 40.

44. A diagnostic agent for a heart disease caused by myocardial degeneration, wherein the agent comprises the antibody according to claim 40.

45. A therapeutic agent for a heart disease caused by myocardial degeneration, wherein the agent comprises the antibody according to claim 40.

46. A drug delivery method for delivering to a cardiac lesion a fusion antibody in which the antibody according to claim 40 is bound to an agent selected from the group consisting of a radioisotope, a protein, and a low-molecular-weight compound.

47. An antibody recognizing a protein comprising the amino acid sequence represented by SEQ ID NO: 20 or 38.

48. A method of screening for a therapeutic agent for a heart disease caused by myocardial degeneration using the antibody according to claim 47.

49. A method of screening for a substance suppressing transcription or translation of a gene whose expression level varies between fetal heart and adult heart using the antibody according to claim 47.

50. A diagnostic agent for a heart disease caused by myocardial degeneration, wherein the agent comprises the antibody according to claim 47.

51. A therapeutic agent for a heart disease caused by myocardial degeneration, wherein the agent comprises the antibody according to claim 47.

52. A drug delivery method for delivering to a cardiac lesion a fusion antibody in which the antibody according to claim 47 is bound to an agent selected from the group consisting of a radioisotope, a protein, and a low-molecular-weight compound.

53. A recombinant viral vector associated with the production of a protein comprising the amino acid sequence selected from the group consisting of the amino acid sequences represented by SEQ ID NOs: 2, 4, 6, 8, 10, 12, 14, 16, 18, 34, and 36.

54. A therapeutic agent for a heart disease caused by myocardial degeneration, which agent comprises the recombinant viral vector according to claim 53.

55. A method of screening for a therapeutic agent for a heart disease caused by myocardial degeneration using an antibody that recognizes a protein comprising the amino acid sequence selected from the group consisting of the amino acid sequences represented by SEQ ID NOs: 2, 4, 6, 8, 10, 12, 14, 16, 18, 34, and 36.

56. A method of screening for a substance suppressing or enhancing transcription or translation of a gene whose expression level varies between fetal heart and adult heart using an antibody that recognizes a protein comprising the amino acid sequence selected from the group consisting of the amino acid sequences represented by SEQ ID NOs: 2, 4, 6, 8, 10, 12, 14, 16, 18, 34, and 36.

57. A diagnostic agent for a heart disease caused by myocardial degeneration, wherein the agent comprises an antibody that recognizes a protein comprising the amino acid sequence selected from the group consisting of the amino acid sequences represented by SEQ ID NOs: 2, 4, 6, 8, 10, 12, 14, 16, 18, 34, and 36.

58. A therapeutic agent for a heart disease caused by myocardial degeneration, wherein the agent comprises an antibody that recognizes a protein comprising the amino acid sequence selected from the group consisting of the amino acid sequences of SEQ ID NOs: 2, 4, 6, 8, 10, 12, 14, 16, 18, 34, and 36.

59. A drug delivery method for delivering to a cardiac lesion a fusion antibody in which an antibody recognizing a protein comprising the amino acid sequence selected from the group consisting of the amino acid sequences represented by SEQ ID NOs: 2, 4, 6, 8, 10, 12, 14, 16, 18, 34, and 36, is bound to an agent selected from the group consisting of a radioisotope, a protein, and a low-molecular-weight compound.


SEQUENCE LISTING

<110> KYOWA HAKKO KOGYO CO., LTD.

<120> MYOCARDIAL CELL PROLIFERATION-ASSOCIATED GENES

<130> 11301W01

<140>
<141>

<150> JP2000-126741
<151> 2000-04-27

<160> 42

<170> PatentIn Ver. 2.0

<210> 1
<211> 3532
<212> DNA
<213> Rattus norvegius
<220>
<221> CDS
<222> (101)..(640)
<400> 1
gcaaactgga catttgcttc tcctgtgaga accttccagc cttttcctgt cttcatcctc 60
ttccagcccc agcggcctcc ttatccaact tcaggtacca atg ggg atc cca gtg 115
Met Gly Ile Pro Val

1                5
ggg aag tcg atg ttg gtg ctt ctc atc tct ttg gcc ttc gcc ttg tgc    163
Gly Lys Ser Met Leu Val Leu Leu Ile Ser Leu Ala Phe Ala Leu Cys
                10                15                20
tgc atc gct gct tac cgc ccc agc gag act ctg tgc gga ggg gag ctt    211
Cys Ile Ala Ala Tyr Arg Pro Ser Glu Thr Leu Cys Gly Gly Glu Leu
            25                30                35
gtt gac acg ctt cag ttt gtc tgt tcg gac cgc ggc ttc tac ttc agc    259
Val Asp Thr Leu Gln Phe Val Cys Ser Asp Arg Gly Phe Tyr Phe Ser
        40                45                50
agg cct tca agc cgt gcc aac cgt cgc agc cgt ggc atc gtg gaa gag    307
Arg Pro Ser Ser Arg Ala Asn Arg Arg Ser Arg Gly Ile Val Glu Glu
    55                60                65
tgc tgc ttc cgc agc tgc gac ttg gcc ctc ctg gag aca tac tgt gcc    355
Cys Cys Phe Arg Ser Cys Asp Leu Ala Leu Leu Glu Thr Tyr Cys Ala
70                75                80                85
acc ccc gcc aag tcc gag agg gac gtg tct acc tct cag gcc gta ctt    403
Thr Pro Ala Lys Ser Glu Arg Asp Val Ser Thr Ser Gln Ala Val Leu
                90                95                100
ccg gac gac ttc ccc aga tac ccc gtg ggc aag ttc ttc aaa ttc gac    451
Pro Asp Asp Phe Pro Arg Tyr Pro Val Gly Lys Phe Phe Lys Phe Asp
            105               110               115
acc tgg aga cag tcc gcg gga cgc ctg cgc aga ggc ctg cct gcc ctc    499
Thr Trp Arg Gln Ser Ala Gly Arg Leu Arg Arg Gly Leu Pro Ala Leu
        120               125               130
ctg cgt gcc cgc cgg ggt cgc atg ctt gcc aaa gag ctc gaa gcg ttc    547
Leu Arg Ala Arg Arg Gly Arg Met Leu Ala Lys Glu Leu Glu Ala Phe
    135               140               145
aga gag gcc aag cgc cac cgt ccc ctg atc gtg tta cca ccc aaa gac    595

Arg Glu Ala Lys Arg His Arg Pro Leu Ile Val Leu Pro Pro Lys Asp
150                 155                 160                 165
ccc gcc cac ggg gga gcc tct tcg gag atg tcc agc aac cat cag tgaacc 646
Pro Ala His Gly Gly Ala Ser Ser Glu Met Ser Ser Asn His Gln
                170                 175                 180
aaattatgtg gtaattctgc aatgtagtac catcagtctg tgacctcctc ttgagcaggg 706
acagctccat catgtcccac actaaggtct ctctgctcca cttcccttcc caggtttctc 766
cccacccacc cccatgcccc gcctccccac atcaggctgc tccccttgcc ccacaccatc 826
gggcaagggg atcccagcaa ctcttcaaaa ccaaatttga ttggctctaa acaacccaat 886
tggcaccctc caaattatat atgaacatta aaaaaaaact ttaaagcata tagtcccttt 946
acaacaaatt ggcttaagaa actccataac tgataatcta aaaattaaat aaccaaagaa 1006
attaattggc taaaaacata ctaaaaatta attggcttaa aaacaattgg caaaaatcaa 1066
ataatttggc ccgccccccc cccccttcatc ttctttccat ttagatcttt agtcaaattg 1126
gctcagactt ggatctcaga acccaagaag aaaggaaggg gacccaaaat tttgcaggta 1186
gcatgtcatt gcttcagtgc tctctccttg tcactagtca cttttagcat aatctggctg 1246
tgaacaacaa tagccgccca aactctttct tcactggtca ttccatcaca aatgtcaccc 1306
atgtcaccaa ggggctgggt gaaggaaccc aaggagagga acagaacatg aaaactgaaa 1366
atagaaccta attggcacaa gcccccagtc ccaaaaatct cacttttcat acctactcta 1426
aaaagcacat gattataccc acacgtacat gcacacacac atgcacacag gcatgcatac 1486
acacacacac acacacacac acactattag atgagaacat tgaaatggct gagcaacttc 1546
gattggaacc acattgccca atccaaggcc catcttaaat tccctgagca gtttgcatgg 1606
tttgagctcc tctctgaatc catctagttt ctgctgccag tgtagagtca gtttggccag 1666
ataaggagat ggcactgcca agtgatacat gctacccgag tagcctgacc cctaggtgtg 1726
ctcctgggag gaaagatctg ggggacaacc cctaccccaa gcacacctat gggccatctc 1786
tgtcaatctc ctggggagcc cccacttttt aggggctccc caggagactc acactgatgt 1846
ggggagtgtg ggaagtctgg cggttggagg ggtgggtggg gggcagtggg ggctgggtgg 1906
ggggaaacta tgggtaggaa gtggtcccag agaggtctta ggtggaacag tcaggaggag 1966
gcacaggtca acttgcagaa ttactgaaga atcaggaccc caaattttct gtcaattgat 2026
ctattcccct ctttttatgt ctggggcagg tttttttcctt tttttttttt aatccctcct 2086
tagcttttaa tgcgctcata atcccattcc ctatgtaacg ggggcagcga tcaagtaatg 2146

aatgcatcaa gccatcaata ccagcgagag ccagtaacac cggctagagc catcaacacc 2206
ggcttccacc atgtcctgct cccaaccatt tatcaacctt tttttttttt ttatctgtct 2266
ctatcgcttg gcctgagttg ggagtggagt ctctgtgggg tgctggccac gcacccacag 2326
agaaataaaa ggaattgaga aggccgctac ctggcctgac ttctggggac agtggctggt 2386
ccccagaagt tctgaggagt ggagggggcg tggggcagtg tcccctcagg tgttaggaag 2446
gtgctcggag gccacaaaga tggggcccca gctggccctg ccagttgggg gggaagggga 2506
tgtagatgta agactagaga ggttccatca ggcgggagca agtggctgcc ttctgagcac 2566
ttgggggagg tcctccccgt gcccctcagt gtcatcttgc ccactcctca gcaccccatc 2626
ttaccctcag gaggtctgga gctctacaga cctcctgggg gcaaggtggg gtgaggcctg 2686
gagctgggga agcgaggagg ctttaaagcc ttcagagcca ggagaactgt gtacatgggg 2746
ttgtctgggc cctggggccc gagggtctgg tgagccgtag cagccactcc acggtgccta 2806
ggactgcggc ggggaacagg gcggctggag gtttacctca cccccacttc tgcttccagt 2866
gcagtccccc tgcccaacag tcctactagt aatctagagg cctgaggctt ctgggcccag 2926
gtgacaggac tggcaccacc ctgggggcgg tgtgtgtcag ccagccatgg cacagagggt 2986
tctcagcaag tgcctaaaga atgggccatt tggaacattg gacagaaact caaagagtaa 3046
attgttataa ttggagaata tgaattggcc tggtacccaa aatatctcga ggcaccctaa 3106
attacctgcc catttgactg gacatccacc cagtgttaat atgcctcgtg ggatgggtgt 3166
tttcaggggc atttgctgac catcctctgt gtccccagat ttgcagttct ccccatcata 3226
ggtcaccctg atgcaggcac ctccctggcc tcccatgcct agtgtggccc tccatcttgt 3286
tttgtctctt ccctactgtc ttcggtggga tcccctcttg ggtcccccaa tttgtcatcc 3346
tgtgaagact tcccacgcgt cgaatgccat atgtcacctg tgccactgcc catgtcatcc 3406
agcagtggcc ccgggtattt gccccaactc agtcctttta acatgcattt tctggcaaaa 3466
tccaaagctt gggttttgtt tttaacctgt taacgcttgc aaacctaata aagcattcaa 3526
aatact 3532
<210> 2
<211> 180
<212> PRT
<213> Rattus norvegicus
<400> 2
Met Gly Ile Pro Val Gly Lys Ser Met Leu Val Leu Leu Ile Ser Leu

1                   5                   10                  15

Ala Phe Ala Leu Cys Cys Ile Ala Ala Tyr Arg Pro Ser Glu Thr Leu

                20                  25                  30

Cys Gly Gly Glu Leu Val Asp Thr Leu Gln Phe Val Cys Ser Asp Arg

            35                  40                  45

Gly Phe Tyr Phe Ser Arg Pro Ser Ser Arg Ala Asn Arg Arg Ser Arg

    50                  55                  60

Gly Ile Val Glu Glu Cys Cys Phe Arg Ser Cys Asp Leu Ala Leu Leu

65                  70                  75                  80

Glu Thr Tyr Cys Ala Thr Pro Ala Lys Ser Glu Arg Asp Val Ser Thr

                85                  90                  95

Ser Gln Ala Val Leu Pro Asp Asp Phe Pro Arg Tyr Pro Val Gly Lys

            100                 105                 110

Phe Phe Lys Phe Asp Thr Trp Arg Gln Ser Ala Gly Arg Leu Arg Arg

        115                 120                 125

Gly Leu Pro Ala Leu Leu Arg Ala Arg Arg Gly Arg Met Leu Ala Lys

    130                 135                 140

Glu Leu Glu Ala Phe Arg Glu Ala Lys Arg His Arg Pro Leu Ile Val

145                 150                 155                 160

Leu Pro Pro Lys Asp Pro Ala His Gly Gly Ala Ser Ser Glu Met Ser

                165                 170                 175

Ser Asn His Gln

            180

<210> 3

<211> 876

<212> DNA

<1> Rattus norvegius

<220>

<221> CDS

<1> (113)..(454)

<400>3

cggctcctga cctctgttcc tgtgctcccg ccgtcgtcct ccagcgcagg cctccgggct 60

ccagctccgg tgttgggtgc aggcctggtg tggtctccaa agtgactgaa ca atg cag 118

Met Gln

1

aag gac agt ggc cca ctg gtt cct tta cat tat tat ggt ttc ggc tat 166

Lys Asp Ser Gly Pro Leu Val Pro Leu His Tyr Tyr Gly Phe Gly Tyr

        5                   10                  15

gcg gcc ctg gtg gct act ggt ggg att att ggc tat gca aaa gca ggt 214

Ala Ala Leu Val Ala Thr Gly Gly Ile Ile Gly Tyr Ala Lys Ala Gly

    20                  25                  30

agt gtg ccg tcc ctg gct gct gga ctc ttc ttt ggg ggc ctg gca ggc 262

Ser Val Pro Ser Leu Ala Ala Gly Leu Phe Phe Gly Gly Leu Ala Gly

35                  40                  45                  50

ctg ggt gcc tac cag ctg tct cag gac ccc agg aac gtg tgg gtt ttc 310

Leu Gly Ala Tyr Gln Leu Ser Gln Asp Pro Arg Asn Val Trp Val Phe

                55                  60                  65

cta gct acg tct ggg act ttg gct ggc att atg ggg atg aga ttc tac 358

Leu Ala Thr Ser Gly Thr Leu Ala Gly Ile Met Gly Met Arg Phe Tyr

            70                  75                  80

aac tct ggg aaa ttt atg cct gca ggt ttg atc gcg gga gcc agt ttg 406

Asn Ser Gly Lys Phe Met Pro Ala Gly Leu Ile Ala Gly Ala Ser Leu

        85                  90                  95

ctg atg gtt gcc aaa ctt gga ctt agt atg ttg agt tca ccc cat ccg 454

Leu Met Val Ala Lys Leu Gly Leu Ser Met Leu Ser Ser Pro His Pro

    100                 105                 110

tagtagccat agccctgcgt gggctcatga tgagttgaca ctctccagtc ctctacatta 514

ccacgctgaa gagataagaa cagcaaagac ctacactgag cacatggagg cgaagacgtg 574

gttactatag tgaccgttca gagacggcga gtgtctgacc tcagagctca cactgccttc 634

atgcggcttg ttcttgtgtc atgatgtctc gactctctgt actactacat aaaggggtaa 694

aatgttgggt gtcaacactg ggggcccaga gctacacgtc ctgccgggaa gttgtgaaat 754
ctcttggtga catttgtgat gtgggatctt ttgcacaggt ctgctatgaa attatgttac 814
ggcaacatta tcggtgaaaa taaagttttc tattaagaaa aaaaaaaaaa aaaaaaaaaa 874
aa 876
<210> 4
<211> 114
<212> PRT
<213> Rattus norvegicus
<400> 4
Met Gln Lys Asp Ser Gly Pro Leu Val Pro Leu His Tyr Tyr Gly Phe
1               5                   10                  15
Gly Tyr Ala Ala Leu Val Ala Thr Gly Gly Ile Ile Gly Tyr Ala Lys
            20                  25                  30
Ala Gly Ser Val Pro Ser Leu Ala Ala Gly Leu Phe Phe Gly Gly Leu
        35                  40                  45
Ala Gly Leu Gly Ala Tyr Gln Leu Ser Gln Asp Pro Arg Asn Val Trp
    50                  55                  60
Val Phe Leu Ala Thr Ser Gly Thr Leu Ala Gly Ile Met Gly Met Arg
65                  70                  75                  80
Phe Tyr Asn Ser Gly Lys Phe Met Pro Ala Gly Leu Ile Ala Gly Ala
                85                  90                  95
Ser Leu Leu Met Val Ala Lys Leu Gly Leu Ser Met Leu Ser Ser Pro
            100                 105                 110
His Pro
<210> 5
<211> 631
<212> DNA
<213> Rattus norvegius
<220>
<221> CDS

<222> (20)..(628)

<400> 5

actagtgatt taggatcca atg cca act atg tcg agg cct gca ctg gat gtc 52
Met Pro Thr Met Ser Arg Pro Ala Leu Asp Val
1               5                   10

aag ggt ggt acc acc tct gga aag gaa gat gcc aac cag gag atg aac 100
Lys Gly Gly Thr Thr Ser Gly Lys Glu Asp Ala Asn Gln Glu Met Asn
            15                  20                  25

tct ctg gcc tac tct aac ctc ggg gta aaa gat cgc agg gca gtg acc 148
Ser Leu Ala Tyr Ser Asn Leu Gly Val Lys Asp Arg Arg Ala Val Thr
        30                  35                  40

gtc ctg cac tac ccc ggg gtc acc gca aat gga gcc aaa gcc aat gga 196
Val Leu His Tyr Pro Gly Val Thr Ala Asn Gly Ala Lys Ala Asn Gly
    45                  50                  55

gtg ccc act agc ccc tct gga tca tca tct cca aca ggc tct cct act 244
Val Pro Thr Ser Pro Ser Gly Ser Ser Ser Pro Thr Gly Ser Pro Thr
60                  65                  70                  75

gcc acc cct tct acc aaa ccc cca tcc ttc aac ctg cac ccc acc cct 292
Ala Thr Pro Ser Thr Lys Pro Pro Ser Phe Asn Leu His Pro Thr Pro
                80                  85                  90

cac ctg ctg gcc agt atg cag ctt cag aag ctt aat agc cag tac caa 340
His Leu Leu Ala Ser Met Gln Leu Gln Lys Leu Asn Ser Gln Tyr Gln
            95                  100                 105

ggg gct gca gcg act gct gct gct gcc ctg gct gca cca agc caa cca 388
Gly Ala Ala Ala Thr Ala Ala Ala Ala Leu Ala Ala Pro Ser Gln Pro
        110                 115                 120

gga gag gat gag ccc ctg cta aac tgg ggc act ggg atc cag gca gga 436
Gly Glu Asp Glu Pro Leu Leu Asn Trp Gly Thr Gly Ile Gln Ala Gly
    125                 130                 135

gca ggg gga tca ccc gga tca gtc tct cct gct ggt gcc cag agc cct 484

Ala Gly Gly Ser Pro Gly Ser Val Ser Pro Ala Gly Ala Gln Ser Pro
140                 145                 150                 155
gct atc att gat tct gac cca gtg gat gag gag gtg ctg atg tct ctg 532
Ala Ile Ile Asp Ser Asp Pro Val Asp Glu Glu Val Leu Met Ser Leu
                160                 165                 170
gtg gta gaa ttg ggg cta gac cga gcc aat gag ctt ccc gag ctg tgg 580
Val Val Glu Leu Gly Leu Asp Arg Ala Asn Glu Leu Pro Glu Leu Trp
            175                 180                 185
cta ggg cag aat gag ttt gat ttc act gca gat ttt ccc tct ggc tgc 628
Leu Gly Gln Asn Glu Phe Asp Phe Thr Ala Asp Phe Pro Ser Gly Cys
        190                 195                 200
tga 631
<210> 6
<211> 203
<212> PRT
<213> Rattus norvegicus
<400> 6
Met Pro Thr Met Ser Arg Pro Ala Leu Asp Val Lys Gly Gly Thr Thr
1               5                   10                  15
Ser Gly Lys Glu Asp Ala Asn Gln Glu Met Asn Ser Leu Ala Tyr Ser
            20                  25                  30
Asn Leu Gly Val Lys Asp Arg Arg Ala Val Thr Val Leu His Tyr Pro
        35                  40                  45
Gly Val Thr Ala Asn Gly Ala Lys Ala Asn Gly Val Pro Thr Ser Pro
    50                  55                  60
Ser Gly Ser Ser Ser Pro Thr Gly Ser Pro Thr Ala Thr Pro Ser Thr
65                  70                  75                  80
Lys Pro Pro Ser Phe Asn Leu His Pro Thr Pro His Leu Leu Ala Ser
                85                  90                  95
Met Gln Leu Gln Lys Leu Asn Ser Gln Tyr Gln Gly Ala Ala Ala Thr

            100                 105                 110
Ala Ala Ala Ala Leu Ala Ala Pro Ser Gln Pro Gly Glu Asp Glu Pro
        115                 120                 125
Leu Leu Asn Trp Gly Thr Gly Ile Gln Ala Gly Ala Gly Gly Ser Pro
    130                 135                 140
Gly Ser Val Ser Pro Ala Gly Ala Gln Ser Pro Ala Ile Ile Asp Ser
145                 150                 155                 160
Asp Pro Val Asp Glu Glu Val Leu MET Ser Leu Val Val Glu Leu Gly
                165                 170                 175
Leu Asp Arg Ala Asn Glu Leu Pro Glu Leu Trp Leu Gly Gln Asn Glu
            180                 185                 190
Phe Asp Phe Thr Ala Asp Phe Pro Ser Gly Cys
        195                 200
<210> 7
<211> 3653
<212> DNA
<213> Rattus norvegius
<220>
<221> CDS
<222> (361)..(3021)
<400> 7
acccccgtca acagcctgga gaaacattcc tggtatcatg gccctgtatc tcggaatgct 60
gctgagtatc tgctgagcag cggaatcaac ggcagcttct tagtgcggga gagtgagagt 120
agccctggcc agagatccat ctcgctgcgg tatgaaggga gggtgtacca ctacaggatc 180
aacactgcct ctgatggcaa gctgtacgtg tcctccgaga gccgcttcaa cactctggct 240
gagttagttc accatcactc cacggtggct gatggcctca tcaccacact ccactaccca 300
gctcccaagc gcaacaagcc cactatctac ggtgtgtccc ccaactacga caagtgggaa 360
atg gag cgc acc gac atc acc atg aag cac aag ttg ggt gga ggc cag 408
Met Glu Arg Thr Asp Ile Thr Met Lys His Lys Leu Gly Gly Gly Gln
1               5                   10                  15

tac ggg gag gtg tac gag ggc gtt tgg aag aag tac agc ctc act gtg 456
Tyr Gly Glu Val Tyr Glu Gly Val Trp Lys Lys Tyr Ser Leu Thr Val
            20                  25                  30
gcc gtg aag acc ttg aag gag gac acc atg gag gtg gag gag ttc ctg 504
Ala Val Lys Thr Leu Lys Glu Asp Thr Met Glu Val Glu Glu Phe Leu
        35                  40                  45
aag gaa gcg gcg gtg atg aag gag atc aaa cac cct aac ctg gtg cag 552
Lys Glu Ala Ala Val Met Lys Glu Ile Lys His Pro Asn Leu Val Gln
    50                  55                  60
ctg cta ggg gtg tgt acc cgg gaa cca cca ttc tac ata atc act gag 600
Leu Leu Gly Val Cys Thr Arg Glu Pro Pro Phe Tyr Ile Ile Thr Glu
65                  70                  75                  80
ttc atg acc tat ggg aac ctg ctg gac tac ctg agg gag tgt aac cgg 648
Phe Met Thr Tyr Gly Asn Leu Leu Asp Tyr Leu Arg Glu Cys Asn Arg
                85                  90                  95
cag gag gtg agc gcc gtg gta ctg ctc tac atg gcc aca cag atc tca 696
Gln Glu Val Ser Ala Val Val Leu Leu Tyr Met Ala Thr Gln Ile Ser
            100                 105                 110
tca gcc atg gag tac ttg gag aag aag aac ttc atc cac aga gac ctt 744
Ser Ala Met Glu Tyr Leu Glu Lys Lys Asn Phe Ile His Arg Asp Leu
        115                 120                 125
gct gcc cgg aac tgc ctg gta ggg gaa aac cac ttg gtg aag gtg gct 792
Ala Ala Arg Asn Cys Leu Val Gly Glu Asn His Leu Val Lys Val Ala
    130                 135                 140
gat ttt ggc ctg agc agg ttg atg aca ggg gac acc tac acg gcc cat 840
Asp Phe Gly Leu Ser Arg Leu Met Thr Gly Asp Thr Tyr Thr Ala His
145                 150                 155                 160
gct gga gcc aaa ttc ccc atc aaa tgg acc gca cct gag agc ctg gcc 888
Ala Gly Ala Lys Phe Pro Ile Lys Trp Thr Ala Pro Glu Ser Leu Ala
                165                 170                 175

tac aac aag ttc tcc atc aag tcg gac gtg tgg gca ttt gga gta ttg 936
Tyr Asn Lys Phe Ser Ile Lys Ser Asp Val Trp Ala Phe Gly Val Leu
            180                 185                 190
ctc tgg gag att gct acc tat ggc atg tca cct tac ccg gga att gac 984
Leu Trp Glu Ile Ala Thr Tyr Gly Met Ser Pro Tyr Pro Gly Ile Asp
        195                 200                 205
ctg tct cag gtt tat gag ctg ctg gaa aaa gac tac cgc atg gag cgc 1032
Leu Ser Gln Val Tyr Glu Leu Leu Glu Lys Asp Tyr Arg Met Glu Arg
    210                 215                 220
cct gaa ggc tgc ccg gag aag gtc tac gag ctc atg cga gca tgt tgg 1080
Pro Glu Gly Cys Pro Glu Lys Val Tyr Glu Leu Met Arg Ala Cys Trp
225                 230                 235                 240
cag tgg aac ccc tct gac cgg ccc tcc ttt gct gaa atc cac caa gcc 1128
Gln Trp Asn Pro Ser Asp Arg Pro Ser Phe Ala Glu Ile His Gln Ala
                245                 250                 255
ttt gaa acc atg ttc cag gaa tcc agt atc tca gat gag gtg gag aag 1176
Phe Glu Thr Met Phe Gln Glu Ser Ser Ile Ser Asp Glu Val Glu Lys
            260                 265                 270
gag ctg ggg aaa cga ggc acg aga gga ggt gct ggg agt atg ctg cag 1224
Glu Leu Gly Lys Arg Gly Thr Arg Gly Gly Ala Gly Ser Met Leu Gln
        275                 280                 285
gcc cca gag ctg ccc acc aag acc aga acc tgc agg aga gca gct gag 1272
Ala Pro Glu Leu Pro Thr Lys Thr Arg Thr Cys Arg Arg Ala Ala Glu
    290                 295                 300
cag aaa gat gcg cct gac acc cct gag ctg ctc cac acg aag ggc ctg 1320
Gln Lys Asp Ala Pro Asp Thr Pro Glu Leu Leu His Thr Lys Gly Leu
305                 310                 315                 320
gga gaa agc gat gca ctg gac agt gag cct gct gta tcg cca ctg ctt 1368
Gly Glu Ser Asp Ala Leu Asp Ser Glu Pro Ala Val Ser Pro Leu Leu
                325                 330                 335

cct cgg aaa gag cgc ggg ccc cca gac ggc agc cta aat gaa gat gag 1416
Pro Arg Lys Glu Arg Gly Pro Pro Asp Gly Ser Leu Asn Glu Asp Glu
            340                 345                 350
cgc ctt ctc ccc aga gac aga aag acc aac ctg ttc agc gct ttg atc 1464
Arg Leu Leu Pro Arg Asp Arg Lys Thr Asn Leu Phe Ser Ala Leu Ile
        355                 360                 365
aag aag aag aag aaa atg gcg ccg acg ccc cct aag cgc agc agt tcc 1512
Lys Lys Lys Lys Lys Met Ala Pro Thr Pro Pro Lys Arg Ser Ser Ser
    370                 375                 380
ttc cga gag atg gat ggc cag cca gac cgc aga ggg gct agt gag gat 1560
Phe Arg Glu Met Asp Gly Gln Pro Asp Arg Arg Gly Ala Ser Glu Asp
385                 390                 395                 400
gac agc agg gaa ctc tgc aat gga cca cca gct ctc acc tca gac gca 1608
Asp Ser Arg Glu Leu Cys Asn Gly Pro Pro Ala Leu Thr Ser Asp Ala
                405                 410                 415
gca gag cct acc aag tcc cca aag gcc agc aat ggg gct ggc gtc cct 1656
Ala Glu Pro Thr Lys Ser Pro Lys Ala Ser Asn Gly Ala Gly Val Pro
            420                 425                 430
aat gga gcc ttc cgg gag ccg ggc aac tca ggc ttc cgt tct ccc cac 1704
Asn Gly Ala Phe Arg Glu Pro Gly Asn Ser Gly Phe Arg Ser Pro His
        435                 440                 445
atg tgg aaa aag tcc agc aca ctg acc ggg agc cgc ctg gct gct gcc 1752
Met Trp Lys Lys Ser Ser Thr Leu Thr Gly Ser Arg Leu Ala Ala Ala
    450                 455                 460
gaa gag gag agc ggc atg agc tcc agt aag cgc ttc ctg cgt tct tgt 1800
Glu Glu Glu Ser Gly Met Ser Ser Ser Lys Arg Phe Leu Arg Ser Cys
465                 470                 475                 480
tcg gcc tcc tgc atg ccc cat ggg gca agg gac aca gag tgg cgg tcg 1848
Ser Ala Ser Cys Met Pro His Gly Ala Arg Asp Thr Glu Trp Arg Ser
                485                 490                 495

gtc acg ctg cct cga gac ctg ccg tct gct ggc aag cag ttt gac tca 1896
Val Thr Leu Pro Arg Asp Leu Pro Ser Ala Gly Lys Gln Phe Asp Ser
            500                 505                 510
tcc acc ttt gga ggg cac aaa agc gaa aag cca gct ctg cct cgg aaa 1944
Ser Thr Phe Gly Gly His Lys Ser Glu Lys Pro Ala Leu Pro Arg Lys
        515                 520                 525
cgc acc agt gag agc agg tct gag cag gtg gcc aaa agc acg gcg atg 1992
Arg Thr Ser Glu Ser Arg Ser Glu Gln Val Ala Lys Ser Thr Ala Met
    530                 535                 540
ccc cct ccc cgg ctg gtg aag aag aac gag gag gct gct gaa gaa ggc 2040
Pro Pro Pro Arg Leu Val Lys Lys Asn Glu Glu Ala Ala Glu Glu Gly
545                 550                 555                 560
ttc aaa gac aca gaa tcc agc cct ggc tcc agc cct ccc agc ttg act 2088
Phe Lys Asp Thr Glu Ser Ser Pro Gly Ser Ser Pro Pro Ser Leu Thr
                565                 570                 575
ccc aaa ctc ctc cgc agg cag gtc act gcc tct cct tcc tct ggc ctc 2136
Pro Lys Leu Leu Arg Arg Gln Val Thr Ala Ser Pro Ser Ser Gly Leu
            580                 585                 590
tct cac aag gaa gag gcc acc aag ggc agt gcc tca ggc atg ggg act 2184
Ser His Lys Glu Glu Ala Thr Lys Gly Ser Ala Ser Gly Met Gly Thr
        595                 600                 605
ccg gcc act gca gag cca gca ccc ccc agc aac aaa gtg ggc ctc agc 2232
Pro Ala Thr Ala Glu Pro Ala Pro Pro Ser Asn Lys Val Gly Leu Ser
    610                 615                 620
aag gcc tcc tct gag gag atg cgc gta agg agg cac aag cac agc tcg 2280
Lys Ala Ser Ser Glu Glu Met Arg Val Arg Arg His Lys His Ser Ser
625                 630                 635                 640
gag tcc cca ggg aga gac aag ggg cga ctg gct aag ctc aag cct gcc 2328
Glu Ser Pro Gly Arg Asp Lys Gly Arg Leu Ala Lys Leu Lys Pro Ala
                645                 650                 655

ccg ccg cct cct cct gcc tgc aca gga aaa gca ggc aag ccc gca cag 2376
Pro Pro Pro Pro Pro Ala Cys Thr Gly Lys Ala Gly Lys Pro Ala Gln
            660                 665                 670
agc ccc agc caa gag gcc ggg gag gca ggg ggg ccc aca aag aca aaa 2424
Ser Pro Ser Gln Glu Ala Gly Glu Ala Gly Gly Pro Thr Lys Thr Lys
        675                 680                 685
tgc acg agt ctg gct atg gat gct gtg aac act gac ccc acc aag gcc 2472
Cys Thr Ser Leu Ala Met Asp Ala Val Asn Thr Asp Pro Thr Lys Ala
    690                 695                 700
ggc cca cct gga gaa gga ctg aga aag cct gtg ccc cca tct gtg cca 2520
Gly Pro Pro Gly Glu Gly Leu Arg Lys Pro Val Pro Pro Ser Val Pro
705                 710                 715                 720
aag ccc cag tcg acg gct aag cct cca ggg act ccc acc agc ccg gtc 2568
Lys Pro Gln Ser Thr Ala Lys Pro Pro Gly Thr Pro Thr Ser Pro Val
                725                 730                 735
tcc acc ccc tcc aca gca cca gct cct tca ccc ctg gct ggg gac cag 2616
Ser Thr Pro Ser Thr Ala Pro Ala Pro Ser Pro Leu Ala Gly Asp Gln
            740                 745                 750
cag cca tct tct gcc gcc ttc atc ccc ctc ata tca acc cgt gtg tct 2664
Gln Pro Ser Ser Ala Ala Phe Ile Pro Leu Ile Ser Thr Arg Val Ser
        755                 760                 765
ctt agg aag acc cgc cag ccg cca gag cgc att gcc agt ggc acc atc 2712
Leu Arg Lys Thr Arg Gln Pro Pro Glu Arg Ile Ala Ser Gly Thr Ile
    770                 775                 780
acc aag ggt gtg gtt ctg gac agt act gag gcc ctg tgc ctt gcc atc 2760
Thr Lys Gly Val Val Leu Asp Ser Thr Glu Ala Leu Cys Leu Ala Ile
785                 790                 795                 800
tcc cgg aac tca gag cag atg gcc agc cac agt gct gta ctg gag gct 2808
Ser Arg Asn Ser Glu Gln Met Ala Ser His Ser Ala Val Leu Glu Ala
                805                 810                 815

ggc aag aac ctg tac act ttc tgt gtg agc tat gtg gac tct atc cag 2856
Gly Lys Asn Leu Tyr Thr Phe Cys Val Ser Tyr Val Asp Ser Ile Gln
            820                 825                 830
cag atg agg aac aag ttt gcc ttc cgt gag gct atc aac aag ctg gag 2904
Gln Met Arg Asn Lys Phe Ala Phe Arg Glu Ala Ile Asn Lys Leu Glu
        835                 840                 845
agc aac ctc cga gag ctg cag atc tgc cct gcc aca gcc tcc agt ggg 2952
Ser Asn Leu Arg Glu Leu Gln Ile Cys Pro Ala Thr Ala Ser Ser Gly
    850                 855                 860
cca gct gcc acc caa gac ttc agc aag ctg ctc agc tct gtg aag gag 3000
Pro Ala Ala Thr Gln Asp Phe Ser Lys Leu Leu Ser Ser Val Lys Glu
865                 870                 875                 880
atc agc gac att gtc cgg agg tagcagcaac cagtgtatgt cagcaagaga 3051
Ile Ser Asp Ile Val Arg Arg
                885
tgttgcagtt cacagggctc ttgtgcctat aaagatgggg acaggggact ggggagctgg 3111
cgtctttccc caggagcttt aaagagagac aagcagagcc tgagggagac ctggatggag 3171
cctggtggag ttggctcttc ctcctgtgtt gtgcaccagc tgccctgcac ctttcctgtc 3231
cagcccaggc gtcagccacc tctcctcact gcctgtggat gggtctcctg ctctgaagac 3291
tacatctggc ctgcctggcc accaggcttc tcactccccg gtgcctcaga cccagctccc 3351
aggtcagcct ggagtgctct tccctgtcct tgcagaacga cctcctctga tggaccttct 3411
tgtcaccaag gcatgggagc ccctgtgctt actgtacctg cacctttgat gcttacaaac 3471
tgtccccgag agcctgtgct cactgtgttt tcattggaag ggctgtcgct ttaagggtca 3531
tgaggtgcta aagccagggg cccagatggg tgggcactgg aaacaggagc tgggcagtgt 3591
ggtctgtcac ctgctctcag tatcttcagc agtgtgcccg gcagatcttg gacagcaagc 3651
tt 3653
<210> 8
<211> 887
<212> PRT
<213> Rattus norvegicus

<400> 8

Met Glu Arg Thr Asp Ile Thr Met Lys His Lys Leu Gly Gly Gly Gln
1               5                   10                  15

Tyr Gly Glu Val Tyr Glu Gly Val Trp Lys Lys Tyr Ser Leu Thr Val
            20                  25                  30

Ala Val Lys Thr Leu Lys Glu Asp Thr Met Glu Val Glu Glu Phe Leu
        35                  40                  45

Lys Glu Ala Ala Val Met Lys Glu Ile Lys His Pro Asn Leu Val Gln
    50                  55                  60

Leu Leu Gly Val Cys Thr Arg Glu Pro Pro Phe Tyr Ile Ile Thr Glu
65                  70                  75                  80

Phe Met Thr Tyr Gly Asn Leu Leu Asp Tyr Leu Arg Glu Cys Asn Arg
                85                  90                  95

Gln Glu Val Ser Ala Val Val Leu Leu Tyr Met Ala Thr Gln Ile Ser
            100                 105                 110

Ser Ala Met Glu Tyr Leu Glu Lys Lys Asn Phe Ile His Arg Asp Leu
        115                 120                 125

Ala Ala Arg Asn Cys Leu Val Gly Glu Asn His Leu Val Lys Val Ala
    130                 135                 140

Asp Phe Gly Leu Ser Arg Leu Met Thr Gly Asp Thr Tyr Thr Ala His
145                 150                 155                 160

Ala Gly Ala Lys Phe Pro Ile Lys Trp Thr Ala Pro Glu Ser Leu Ala
                165                 170                 175

Tyr Asn Lys Phe Ser Ile Lys Ser Asp Val Trp Ala Phe Gly Val Leu
            180                 185                 190

Leu Trp Glu Ile Ala Thr Tyr Gly Met Ser Pro Tyr Pro Gly Ile Asp
        195                 200                 205

Leu Ser Gln Val Tyr Glu Leu Leu Glu Lys Asp Tyr Arg Met Glu Arg
    210                 215                 220

Pro Glu Gly Cys Pro Glu Lys Val Tyr Glu Leu Met Arg Ala Cys Trp

225                 230                 235                 240
Gln Trp Asn Pro Ser Asp Arg Pro Ser Phe Ala Glu Ile His Gln Ala
                245                 250                 255
Phe Glu Thr Met Phe Gln Glu Ser Ser Ile Ser Asp Glu Val Glu Lys
            260                 265                 270
Glu Leu Gly Lys Arg Gly Thr Arg Gly Gly Ala Gly Ser Met Leu Gln
        275                 280                 285
Ala Pro Glu Leu Pro Thr Lys Thr Arg Thr Cys Arg Arg Ala Ala Glu
    290                 295                 300
Gln Lys Asp Ala Pro Asp Thr Pro Glu Leu Leu His Thr Lys Gly Leu
305                 310                 315                 320
Gly Glu Ser Asp Ala Leu Asp Ser Glu Pro Ala Val Ser Pro Leu Leu
                325                 330                 335
Pro Arg Lys Glu Arg Gly Pro Pro Asp Gly Ser Leu Asn Glu Asp Glu
            340                 345                 350
Arg Leu Leu Pro Arg Asp Arg Lys Thr Asn Leu Phe Ser Ala Leu Ile
        355                 360                 365
Lys Lys Lys Lys Lys Met Ala Pro Thr Pro Pro Lys Arg Ser Ser Ser
    370                 375                 380
Phe Arg Glu Met Asp Gly Gln Pro Asp Arg Arg Gly Ala Ser Glu Asp
385                 390                 395                 400
Asp Ser Arg Glu Leu Cys Asn Gly Pro Pro Ala Leu Thr Ser Asp Ala
                405                 410                 415
Ala Glu Pro Thr Lys Ser Pro Lys Ala Ser Asn Gly Ala Gly Val Pro
            420                 425                 430
Asn Gly Ala Phe Arg Glu Pro Gly Asn Ser Gly Phe Arg Ser Pro His
        435                 440                 445
Met Trp Lys Lys Ser Ser Thr Leu Thr Gly Ser Arg Leu Ala Ala Ala
    450                 455                 460
Glu Glu Glu Ser Gly Met Ser Ser Ser Lys Arg Phe Leu Arg Ser Cys

465                 470                 475                 480
Ser Ala Ser Cys Met Pro His Gly Ala Arg Asp Thr Glu Trp Arg Ser
                485                 490                 495
Val Thr Leu Pro Arg Asp Leu Pro Ser Ala Gly Lys Gln Phe Asp Ser
            500                 505                 510
Ser Thr Phe Gly Gly His Lys Ser Glu Lys Pro Ala Leu Pro Arg Lys
        515                 520                 525
Arg Thr Ser Glu Ser Arg Ser Glu Gln Val Ala Lys Ser Thr Ala Met
    530                 535                 540
Pro Pro Pro Arg Leu Val Lys Lys Asn Glu Glu Ala Ala Glu Glu Gly
545                 550                 555                 560
Phe Lys Asp Thr Glu Ser Ser Pro Gly Ser Ser Pro Pro Ser Leu Thr
                565                 570                 575
Pro Lys Leu Leu Arg Arg Gln Val Thr Ala Ser Pro Ser Ser Gly Leu
            580                 585                 590
Ser His Lys Glu Glu Ala Thr Lys Gly Ser Ala Ser Gly Met Gly Thr
        595                 600                 605
Pro Ala Thr Ala Glu Pro Ala Pro Pro Ser Asn Lys Val Gly Leu Ser
    610                 615                 620
Lys Ala Ser Ser Glu Glu Met Arg Val Arg Arg His Lys His Ser Ser
625                 630                 635                 640
Glu Ser Pro Gly Arg Asp Lys Gly Arg Leu Ala Lys Leu Lys Pro Ala
                645                 650                 655
Pro Pro Pro Pro Pro Ala Cys Thr Gly Lys Ala Gly Lys Pro Ala Gln
            660                 665                 670
Ser Pro Ser Gln Glu Ala Gly Glu Ala Gly Gly Pro Thr Lys Thr Lys
        675                 680                 685
Cys Thr Ser Leu Ala Met Asp Ala Val Asn Thr Asp Pro Thr Lys Ala
    690                 695                 700
Gly Pro Pro Gly Glu Gly Leu Arg Lys Pro Val Pro Pro Ser Val Pro

705                 710                 715                 720
Lys Pro Gln Ser Thr Ala Lys Pro Pro Gly Thr Pro Thr Ser Pro Val
                725                 730                 735
Ser Thr Pro Ser Thr Ala Pro Ala Pro Ser Pro Leu Ala Gly Asp Gln
            740                 745                 750
Gln Pro Ser Ser Ala Ala Phe Ile Pro Leu Ile Ser Thr Arg Val Ser
        755                 760                 765
Leu Arg Lys Thr Arg Gln Pro Pro Glu Arg Ile Ala Ser Gly Thr Ile
    770                 775                 780
Thr Lys Gly Val Val Leu Asp Ser Thr Glu Ala Leu Cys Leu Ala Ile
785                 790                 795                 800
Ser Arg Asn Ser Glu Gln Met Ala Ser His Ser Ala Val Leu Glu Ala
                805                 810                 815
Gly Lys Asn Leu Tyr Thr Phe Cys Val Ser Tyr Val Asp Ser Ile Gln
            820                 825                 830
Gln Met Arg Asn Lys Phe Ala Phe Arg Glu Ala Ile Asn Lys Leu Glu
        835                 840                 845
Ser Asn Leu Arg Glu Leu Gln Ile Cys Pro Ala Thr Ala Ser Ser Gly
    850                 855                 860
Pro Ala Ala Thr Gln Asp Phe Ser Lys Leu Leu Ser Ser Val Lys Glu
865                 870                 875                 880
Ile Ser Asp Ile Val Arg Arg
                885
<210> 9
<211> 1725
<212> DNA
<213> Rattus norvegius
<220>
<221> CDS
<222> (111)..(1412)

<400> 9
agtgctgctc cggtaccgag tcgcgctcac gtttgtcctt aagactccct tcggtgtctc 60
caggaccctc tttccttcct ccgcagcgat cctactgcca gaacttcacc atg tcc 116
Met Ser
1
att ctc aag atc cat gcc aga gag atc ttt gac tcc cgc ggg aat ccc 164
Ile Leu Lys Ile His Ala Arg Glu Ile Phe Asp Ser Arg Gly Asn Pro
        5                   10                  15
acc gtt gag gtg gat ctc tac acc gca aaa ggt ctc ttc cgt gct gcg 212
Thr Val Glu Val Asp Leu Tyr Thr Ala Lys Gly Leu Phe Arg Ala Ala
    20                  25                  30
gtg ccc agc ggt gcg tcc act ggc atc tac gag gcc cta gaa ctc cga 260
Val Pro Ser Gly Ala Ser Thr Gly Ile Tyr Glu Ala Leu Glu Leu Arg
35                  40                  45                  50
gac aat gat aag acc cgc ttc atg ggg aag ggt gtc tca aag gct gtt 308
Asp Asn Asp Lys Thr Arg Phe Met Gly Lys Gly Val Ser Lys Ala Val
                55                  60                  65
gag cac atc aat aaa act att gca cct gct ctg gtt agc aag aaa ctg 356
Glu His Ile Asn Lys Thr Ile Ala Pro Ala Leu Val Ser Lys Lys Leu
            70                  75                  80
aat gtt gtg gag cag gag aag att gac cag ctg atg atc gag atg gac 404
Asn Val Val Glu Gln Glu Lys Ile Asp Gln Leu Met Ile Glu Met Asp
        85                  90                  95
ggc aca gag aat aaa tct aag ttt ggt gca aat gcc atc ctg gga gtg 452
Gly Thr Glu Asn Lys Ser Lys Phe Gly Ala Asn Ala Ile Leu Gly Val
    100                 105                 110
tcc ctg gct gtc tgc aag gct ggt gcc gtg ggg aag ggg gtg ccc ctt 500
Ser Leu Ala Val Cys Lys Ala Gly Ala Val Gly Lys Gly Val Pro Leu
115                 120                 125                 130
tac cgt cac att gcc gac ttg gcc ggc aac cct gaa gtc act ctg ccg 548

Tyr Arg His Ile Ala Asp Leu Ala Gly Asn Pro Glu Val Thr Leu Pro
                135                 140                 145
gtc cca gct ttc aat gtg atc aac ggc ggt tct cat gct ggc aac aag 596
Val Pro Ala Phe Asn Val Ile Asn Gly Gly Ser His Ala Gly Asn Lys
            150                 155                 160
ttg gcc atg caa gag ttc atg atc ctg cct gtg ggg gca tcc tct ttc 644
Leu Ala Met Gln Glu Phe Met Ile Leu Pro Val Gly Ala Ser Ser Phe
        165                 170                 175
cgg gaa gcc atg cgc att gga gca gag gtt tac cac aac ctg aag aac 692
Arg Glu Ala Met Arg Ile Gly Ala Glu Val Tyr His Asn Leu Lys Asn
    180                 185                 190
gtc atc aaa gag aag tac ggg aaa gac gcc acc aat gtg ggt gat gag 740
Val Ile Lys Glu Lys Tyr Gly Lys Asp Ala Thr Asn Val Gly Asp Glu
195                 200                 205                 210
ggt gga ttc gca cct aac atc ctg gag aac aaa gaa gca ctg gag ctg 788
Gly Gly Phe Ala Pro Asn Ile Leu Glu Asn Lys Glu Ala Leu Glu Leu
                215                 220                 225
ctc aag tct gcc att gca aag gcc ggc tac act gac cag gtt gtc atc 836
Leu Lys Ser Ala Ile Ala Lys Ala Gly Tyr Thr Asp Gln Val Val Ile
            230                 235                 240
ggc atg gat gtg gct gcc tcc gag ttc tac agg gct ggc aag tat gac 884
Gly Met Asp Val Ala Ala Ser Glu Phe Tyr Arg Ala Gly Lys Tyr Asp
        245                 250                 255
ctg gac ttc aag tct cca gat gat gcc agc cgg tac atc aca ccc gac 932
Leu Asp Phe Lys Ser Pro Asp Asp Ala Ser Arg Tyr Ile Thr Pro Asp
    260                 265                 270
cag ctg gcc gac ctg tac aag tcc ttc atc aag gac tac cca gtg gtg 980
Gln Leu Ala Asp Leu Tyr Lys Ser Phe Ile Lys Asp Tyr Pro Val Val
275                 280                 285                 290
tcc att gaa gat ccc ttt gac cag gac gac tgg gat gct tgg cag aag 1028

Ser Ile Glu Asp Pro Phe Asp Gln Asp Asp Trp Asp Ala Trp Gln Lys
                295                 300                 305
ttc aca gct act gca ggc atc cag gtg gtg ggg gat gac ctc aca gtg 1076
Phe Thr Ala Thr Ala Gly Ile Gln Val Val Gly Asp Asp Leu Thr Val
            310                 315                 320
acc aac cct aag cgg atc gcc aag gct gca ggc gaa aag tcc tgc aac 1124
Thr Asn Pro Lys Arg Ile Ala Lys Ala Ala Gly Glu Lys Ser Cys Asn
        325                 330                 335
tgc ctc ctg ctc aaa gtg aac cag att ggc tct gtg acc gag tct ctg 1172
Cys Leu Leu Leu Lys Val Asn Gln Ile Gly Ser Val Thr Glu Ser Leu
    340                 345                 350
cag gcg tgt aag ctg gcc cag tcc aat ggc tgg ggt gtc atg gtg tcc 1220
Gln Ala Cys Lys Leu Ala Gln Ser Asn Gly Trp Gly Val Met Val Ser
355                 360                 365                 370
cat cga tct gag gag act gag gac act ttc att gcc gac ctg gtg gtg 1268
His Arg Ser Glu Glu Thr Glu Asp Thr Phe Ile Ala Asp Leu Val Val
                375                 380                 385
ggg ctc tgc act ggg cag atc aag act ggt gcc ccc tgc cga tct gag 1316
Gly Leu Cys Thr Gly Gln Ile Lys Thr Gly Ala Pro Cys Arg Ser Glu
            390                 395                 400
cgc ctg gcc aag tac aat cag atc ctt aga atc gag gag gaa ctg ggc 1364
Arg Leu Ala Lys Tyr Asn Gln Ile Leu Arg Ile Glu Glu Glu Leu Gly
        405                 410                 415
agc aaa gcc aag ttt gcc ggc agg tcc ttc agg aac ccc ctg gcc aag 1412
Ser Lys Ala Lys Phe Ala Gly Arg Ser Phe Arg Asn Pro Leu Ala Lys
    420                 425                 430
taaggcatgg accggagatc cctggagcta ccagatcctc tgtctccgtc atccaggcgg 1472
ctcaaggctg gcccagtgct tgcccctccc atgtcactgc ttccttagat gtccaccccg 1532
accacctgga gccctgctgg agcccccagc tttgtaatca tgtgatcagt ctgaatcatt 1592
gtttctgtca cctgactttc cagctagtgt ctggagccct ctgaactcca gcgtaatctc 1652

tagagtgccc acaccatcaa gactcccccc agtggtttac ttgcaaaaat aaaagctgca 1712
gaagctcaaa aaa 1725
<210> 10
<211> 434
<212> PRT
<213> Rattus norvegicus
<400> 10
Met Ser Ile Leu Lys Ile His Ala Arg Glu Ile Phe Asp Ser Arg Gly
1               5                   10                  15
Asn Pro Thr Val Glu Val Asp Leu Tyr Thr Ala Lys Gly Leu Phe Arg
            20                  25                  30
Ala Ala Val Pro Ser Gly Ala Ser Thr Gly Ile Tyr Glu Ala Leu Glu
        35                  40                  45
Leu Arg Asp Asn Asp Lys Thr Arg Phe Met Gly Lys Gly Val Ser Lys
    50                  55                  60
Ala Val Glu His Ile Asn Lys Thr Ile Ala Pro Ala Leu Val Ser Lys
65                  70                  75                  80
Lys Leu Asn Val Val Glu Gln Glu Lys Ile Asp Gln Leu Met Ile Glu
                85                  90                  95
Met Asp Gly Thr Glu Asn Lys Ser Lys Phe Gly Ala Asn Ala Ile Leu
            100                 105                 110
Gly Val Ser Leu Ala Val Cys Lys Ala Gly Ala Val Gly Lys Gly Val
        115                 120                 125
Pro Leu Tyr Arg His Ile Ala Asp Leu Ala Gly Asn Pro Glu Val Thr
    130                 135                 140
Leu Pro Val Pro Ala Phe Asn Val Ile Asn Gly Gly Ser His Ala Gly
145                 150                 155                 160
Asn Lys Leu Ala Met Gln Glu Phe Met Ile Leu Pro Val Gly Ala Ser
                165                 170                 175
Ser Phe Arg Glu Ala Met Arg Ile Gly Ala Glu Val Tyr His Asn Leu

            180                 185                 190
Lys Asn Val Ile Lys Glu Lys Tyr Gly Lys Asp Ala Thr Asn Val Gly
        195                 200                 205
Asp Glu Gly Gly Phe Ala Pro Asn Ile Leu Glu Asn Lys Glu Ala Leu
    210                 215                 220
Glu Leu Leu Lys Ser Ala Ile Ala Lys Ala Gly Tyr Thr Asp Gln Val
225                 230                 235                 240
Val Ile Gly Met Asp Val Ala Ala Ser Glu Phe Tyr Arg Ala Gly Lys
                245                 250                 255
Tyr Asp Leu Asp Phe Lys Ser Pro Asp Asp Ala Ser Arg Tyr Ile Thr
            260                 265                 270
Pro Asp Gln Leu Ala Asp Leu Tyr Lys Ser Phe Ile Lys Asp Tyr Pro
        275                 280                 285
Val Val Ser Ile Glu Asp Pro Phe Asp Gln Asp Asp Trp Asp Ala Trp
    290                 295                 300
Gln Lys Phe Thr Ala Thr Ala Gly Ile Gln Val Val Gly Asp Asp Leu
305                 310                 315                 320
Thr Val Thr Asn Pro Lys Arg Ile Ala Lys Ala Ala Gly Glu Lys Ser
                325                 330                 335
Cys Asn Cys Leu Leu Leu Lys Val Asn Gln Ile Gly Ser Val Thr Glu
            340                 345                 350
Ser Leu Gln Ala Cys Lys Leu Ala Gln Ser Asn Gly Trp Gly Val Met
        355                 360                 365
Val Ser His Arg Ser Glu Glu Thr Glu Asp Thr Phe Ile Ala Asp Leu
    370                 375                 380
Val Val Gly Leu Cys Thr Gly Gln Ile Lys Thr Gly Ala Pro Cys Arg
385                 390                 395                 400
Ser Glu Arg Leu Ala Lys Tyr Asn Gln Ile Leu Arg Ile Glu Glu Glu
                405                 410                 415

Leu Gly Ser Lys Ala Lys Phe Ala Gly Arg Ser Phe Arg Asn Pro Leu
            420                 425                 430
Ala Lys
<210> 11
<211> 5412
<212> DNA
<213> Rattus norvegius
<220>
<221> CDS
<222> (31)..(4515)
<400> 11
cagggcggag gctggaggcc actgccaagc atg gcg ccc acc tgg aga ccc agc 54
Met Ala Pro Thr Trp Arg Pro Ser
1               5
gtg gtg tct gtg gtg ggt cct gtg ggg ctc ttc ctt gta ctg ctg gcc 102
Val Val Ser Val Val Gly Pro Val Gly Leu Phe Leu Val Leu Leu Ala
    10                  15                  20
aga ggg tgc ttg gct gaa gag cca ccc aga ttt atc aga gag ccc aag 150
Arg Gly Cys Leu Ala Glu Glu Pro Pro Arg Phe Ile Arg Glu Pro Lys
25                  30                  35                  40
gat cag att ggt gtg tca gga ggc gtg gcc tcc ttc gtg tgc cag gcc 198
Asp Gln Ile Gly Val Ser Gly Gly Val Ala Ser Phe Val Cys Gln Ala
                45                  50                  55
aca ggt gac cct aag cca cgg gtg acc tgg aac aag aag ggc aag aaa 246
Thr Gly Asp Pro Lys Pro Arg Val Thr Trp Asn Lys Lys Gly Lys Lys
            60                  65                  70
gtg aac tca cag cgc ttt gag acc att gac ttt gac gag agc tcg ggg 294
Val Asn Ser Gln Arg Phe Glu Thr Ile Asp Phe Asp Glu Ser Ser Gly
        75                  80                  85
gcc gtg ctg agg atc cag cca ctt cgg aca ccc cgg gat gag aac gtg 342

Ala Val Leu Arg Ile Gln Pro Leu Arg Thr Pro Arg Asp Glu Asn Val
    90                  95                  100
tac gag tgt gtg gcc cag aac tcg gtg ggg gag atc aca gtt cat gcg 390
Tyr Glu Cys Val Ala Gln Asn Ser Val Gly Glu Ile Thr Val His Ala
105                 110                 115                 120
aag ctc acc gtc ctg cga gag gac cag ctg cct cct ggc ttc ccc aac 438
Lys Leu Thr Val Leu Arg Glu Asp Gln Leu Pro Pro Gly Phe Pro Asn
                125                 130                 135
att gac atg ggc ccc cag ttg aag gtt gta gag cgc aca cgc aca gcc 486
Ile Asp Met Gly Pro Gln Leu Lys Val Val Glu Arg Thr Arg Thr Ala
            140                 145                 150
acc atg ctc tgt gct gcc agc gga aac cct gac cct gag atc acc tgg 534
Thr Met Leu Cys Ala Ala Ser Gly Asn Pro Asp Pro Glu Ile Thr Trp
        155                 160                 165
ttc aag gac ttc ctg cct gtg gac ccc agt gcc agc aat ggg cgg atc 582
Phe Lys Asp Phe Leu Pro Val Asp Pro Ser Ala Ser Asn Gly Arg Ile
    170                 175                 180
aag cag ctt cgg tca ggt gcc ctg cag att gag agc agc gag gag aca 630
Lys Gln Leu Arg Ser Gly Ala Leu Gln Ile Glu Ser Ser Glu Glu Thr
185                 190                 195                 200
gac cag ggc aag tac gag tgt gtg gcc acc aaa cag gcg ggg gtg cgc 678
Asp Gln Gly Lys Tyr Glu Cys Val Ala Thr Lys Gln Ala Gly Val Arg
                205                 210                 215
tac tca tca cct gcc aac ctc tac gtg cga gtc cgc cgt gtg gcc ccc 726
Tyr Ser Ser Pro Ala Asn Leu Tyr Val Arg Val Arg Arg Val Ala Pro
            220                 225                 230
cgc ttc tcc atc ctg ccc atg agc cac gag atc atg ccc ggt ggg aat 774
Arg Phe Ser Ile Leu Pro Met Ser His Glu Ile Met Pro Gly Gly Asn
        235                 240                 245
gtg aat atc act tgt gtg gct gtg ggc tca ccc atg ccc tac gtg aag 822

Val Asn Ile Thr Cys Val Ala Val Gly Ser Pro Met Pro Tyr Val Lys
    250                 255                 260
tgg atg cag ggg gca gag gac ctg acg cct gag gat gac atg ccc gtg 870
Trp Met Gln Gly Ala Glu Asp Leu Thr Pro Glu Asp Asp Met Pro Val
265                 270                 275                 280
ggt cgg aat gtc ctc gaa ctc acg gat gtc aaa gac tca gcc aac tat 918
Gly Arg Asn Val Leu Glu Leu Thr Asp Val Lys Asp Ser Ala Asn Tyr
                285                 290                 295
cct tgt gtg gcc atg tcc agc ctg gga gtg atc gag gcc gtt gct gac 966
Pro Cys Val Ala Met Ser Ser Leu Gly Val Ile Glu Ala Val Ala Asp
            300                 305                 310
atc act gta aaa tct ctc ccc aaa gcc cct ggg act ccc gtg gtg acg 1014
Ile Thr Val Lys Ser Leu Pro Lys Ala Pro Gly Thr Pro Val Val Thr
        315                 320                 325
gag aac act gct acc agt atc act gtc aca tgg gac gca ggc aat cct 1062
Glu Asn Thr Ala Thr Ser Ile Thr Val Thr Trp Asp Ala Gly Asn Pro
    330                 335                 340
gac cct gtg tcc tac tac gta ttg agt ata atc aaa gcc agg atg ggc 1110
Asp Pro Val Ser Tyr Tyr Val Leu Ser Ile Ile Lys Ala Arg Met Gly
345                 350                 355                 360
cgt atc aga tca aag aag aca tca acc acc acg cgc tac agc atc ggc 1158
Arg Ile Arg Ser Lys Lys Thr Ser Thr Thr Thr Arg Tyr Ser Ile Gly
                365                 370                 375
ggc ctg agc ccc aac tct gag tat gag atc tgg gtg tca gct gtc aac 1206
Gly Leu Ser Pro Asn Ser Glu Tyr Glu Ile Trp Val Ser Ala Val Asn
            380                 385                 390
tcc atc ggc cag gcc ccc agt gag tcg gtg gtg acc cgc aca ggc gag 1254
Ser Ile Gly Gln Ala Pro Ser Glu Ser Val Val Thr Arg Thr Gly Glu
        395                 400                 405
cag gca cca gcc agt gct ccc agg aat gtt cag gcg cgc atg ctc agt 1302

Gln Ala Pro Ala Ser Ala Pro Arg Asn Val Gln Ala Arg Met Leu Ser
    410                 415                 420
gcc acc acc atg att gtg cag tgg gag gag ccc gtg gag ccc aat ggc 1350
Ala Thr Thr Met Ile Val Gln Trp Glu Glu Pro Val Glu Pro Asn Gly
425                 430                 435                 440
ctg atc cgt ggc tac cgc gtc tac tac acc atg gag ccc gag cat ccg 1398
Leu Ile Arg Gly Tyr Arg Val Tyr Tyr Thr Met Glu Pro Glu His Pro
                445                 450                 455
gtg ggc aac tgg cag aag cac aat gtg gac gac agt ctt ctg acc act 1446
Val Gly Asn Trp Gln Lys His Asn Val Asp Asp Ser Leu Leu Thr Thr
            460                 465                 470
gtg ggc agc ctg cta gag gat gag acc tac act gtg aga gtg ctc gcc 1494
Val Gly Ser Leu Leu Glu Asp Glu Thr Tyr Thr Val Arg Val Leu Ala
        475                 480                 485
ttc aca tcg gtg ggc gat ggg cca ctg tca gac ccc atc cag gtc aag 1542
Phe Thr Ser Val Gly Asp Gly Pro Leu Ser Asp Pro Ile Gln Val Lys
    490                 495                 500
acc cag cag gga gtg ccc ggc cag ccc atg aac ttg cgg gct gag gcc 1590
Thr Gln Gln Gly Val Pro Gly Gln Pro Met Asn Leu Arg Ala Glu Ala
505                 510                 515                 520
aag tca gag acc agc att ggg ctc tcg tgg agt gca cca cgg cag gag 1638
Lys Ser Glu Thr Ser Ile Gly Leu Ser Trp Ser Ala Pro Arg Gln Glu
                525                 530                 535
agt gtc att aag tat gaa ctg ctc ttc cgg gag ggc gac cga ggc cga 1686
Ser Val Ile Lys Tyr Glu Leu Leu Phe Arg Glu Gly Asp Arg Gly Arg
            540                 545                 550
gag gtg ggg cga acc ttc gac cca acc aca gcc ttt gtg gtg gag gac 1734
Glu Val Gly Arg Thr Phe Asp Pro Thr Thr Ala Phe Val Val Glu Asp
        555                 560                 565
ctc aag ccc aat acg gag tac gcg ttc cgg ctg gcg gct cgc tcg ccg 1782

Leu Lys Pro Asn Thr Glu Tyr Ala Phe Arg Leu Ala Ala Arg Ser Pro
    570                 575                 580
cag ggc ctg ggc gcc ttc acc gcg gtt gtg cgc cag cgc aca ctg cag 1830
Gln Gly Leu Gly Ala Phe Thr Ala Val Val Arg Gln Arg Thr Leu Gln
585                 590                 595                 600
gcc atc tcc ccc aag aac ttc aag gtg aag atg atc atg aaa act tca 1878
Ala Ile Ser Pro Lys Asn Phe Lys Val Lys Met Ile Met Lys Thr Ser
                605                 610                 615
gtg ctg cta agc tgg gag ttc cct gac aac tat aac tca ccc acg ccc 1926
Val Leu Leu Ser Trp Glu Phe Pro Asp Asn Tyr Asn Ser Pro Thr Pro
            620                 625                 630
tac aag atc cag tac aat gga ctc aca ctg gac gtg gat ggc cgc act 1974
Tyr Lys Ile Gln Tyr Asn Gly Leu Thr Leu Asp Val Asp Gly Arg Thr
        635                 640                 645
acc aag aag ctg atc acg cac ctc aag cca cac acc ttc tat aac ttc 2022
Thr Lys Lys Leu Ile Thr His Leu Lys Pro His Thr Phe Tyr Asn Phe
    650                 655                 660
gtg ctc acc aac cgt ggc agc agc ctg gga ggc ctg cag cag acg gtc 2070
Val Leu Thr Asn Arg Gly Ser Ser Leu Gly Gly Leu Gln Gln Thr Val
665                 670                 675                 680
acc gcc agg acc gcc ttc aac atg ctc agt ggc aag cct agt gtc gcc 2118
Thr Ala Arg Thr Ala Phe Asn Met Leu Ser Gly Lys Pro Ser Val Ala
                685                 690                 695
cca aag cct gac aac gat ggt tcc att gtg gtc tac ctg cct gat ggc 2166
Pro Lys Pro Asp Asn Asp Gly Ser Ile Val Val Tyr Leu Pro Asp Gly
            700                 705                 710
cag agt ccc gtg aca gtg cag aac tac ttc att gtg atg gtc cca ctt 2214
Gln Ser Pro Val Thr Val Gln Asn Tyr Phe Ile Val Met Val Pro Leu
        715                 720                 725
cgg aag tct cgt ggt ggc cag ttc cct atc cta cta cct agt cca gag 2262

Arg Lys Ser Arg Gly Gly Gln Phe Pro Ile Leu Leu Pro Ser Pro Glu
    730                 735                 740
gac atg gat ctg gag gag ctc atc cag gac ctc tcc cgg ctg cag agc 2310
Asp Met Asp Leu Glu Glu Leu Ile Gln Asp Leu Ser Arg Leu Gln Ser
745                 750                 755                 760
agc ctg cgc cac tca aga cag ctg gag gtg cct cgg cct tac atc gcc 2358
Ser Leu Arg His Ser Arg Gln Leu Glu Val Pro Arg Pro Tyr Ile Ala
                765                 770                 775
gct cgg ttc tcc atc ctg cca gct gtc ttc cat cct ggg aac cag aag 2406
Ala Arg Phe Ser Ile Leu Pro Ala Val Phe His Pro Gly Asn Gln Lys
            780                 785                 790
caa tat ggt ggc ttt gac aac agg ggc ttg gag cca ggc cac cgt tat 2454
Gln Tyr Gly Gly Phe Asp Asn Arg Gly Leu Glu Pro Gly His Arg Tyr
        795                 800                 805
gtc ctc ttt gta ctt gct gtg ctg cag aag aat gag cct aca ttt gca 2502
Val Leu Phe Val Leu Ala Val Leu Gln Lys Asn Glu Pro Thr Phe Ala
    810                 815                 820
gcc agt ccc ttc tca gac ccc ttc caa ctg gac aac cca gac ccg cag 2550
Ala Ser Pro Phe Ser Asp Pro Phe Gln Leu Asp Asn Pro Asp Pro Gln
825                 830                 835                 840
ccc att gtg gat ggc gag gag ggc ctc atc tgg gtg atc ggg ccc gtg 2598
Pro Ile Val Asp Gly Glu Glu Gly Leu Ile Trp Val Ile Gly Pro Val
                845                 850                 855
ctg gcc gtg gtc ttc atc atc tgc atc gta att gcc atc ctg ctg tac 2646
Leu Ala Val Val Phe Ile Ile Cys Ile Val Ile Ala Ile Leu Leu Tyr
            860                 865                 870
aag aac aag cct gac agc aaa cgc aag gac tca gag ccc cgc acc aaa 2694
Lys Asn Lys Pro Asp Ser Lys Arg Lys Asp Ser Glu Pro Arg Thr Lys
        875                 880                 885
tgc tta ttg aac aat gca gac ctc gcc ccc cat cac ccc aag gac cct 2742

Cys Leu Leu Asn Asn Ala Asp Leu Ala Pro His His Pro Lys Asp Pro
    890                 895                 900
gtg gaa atg cga cgt atc aac ttc cag acg cca ggt atg ctc agc cac 2790
Val Glu Met Arg Arg Ile Asn Phe Gln Thr Pro Gly Met Leu Ser His
905                 910                 915                 920
ccg ccc att ccc atc aca gac atg gct gaa cac atg gag aga ctc aaa 2838
Pro Pro Ile Pro Ile Thr Asp Met Ala Glu His Met Glu Arg Leu Lys
                925                 930                 935
gcc aac gac agc ctc aag ctc tcc cag gag tat gag tcc atc gac cct 2886
Ala Asn Asp Ser Leu Lys Leu Ser Gln Glu Tyr Glu Ser Ile Asp Pro
            940                 945                 950
ggc cag cag ttc act tgg gaa cat tcg aac ctg gag gcc aac aag cca 2934
Gly Gln Gln Phe Thr Trp Glu His Ser Asn Leu Glu Ala Asn Lys Pro
        955                 960                 965
aag aac cga tac gcc aat gtc atc gcc tat gac cat tca cga gtc atc 2982
Lys Asn Arg Tyr Ala Asn Val Ile Ala Tyr Asp His Ser Arg Val Ile
    970                 975                 980
ctg cag cct tta gaa ggc atc atg ggt agt gat tac atc aat gcc aac 3030
Leu Gln Pro Leu Glu Gly Ile Met Gly Ser Asp Tyr Ile Asn Ala Asn
985                 990                 995                 1000
tat gtt gac ggc tat cgg cgg cag aac gca tac atc gcc acg cag ggg 3078
Tyr Val Asp Gly Tyr Arg Arg Gln Asn Ala Tyr Ile Ala Thr Gln Gly
                1005                1010                1015
ccc ctg cct gag acc ttt ggg gac ttc tgg cgg atg gtg tgg gag cag 3126
Pro Leu Pro Glu Thr Phe Gly Asp Phe Trp Arg Met Val Trp Glu Gln
            1020                1025                1030
cgg tca gcc act gtg gtc atg atg aca cgg ctg gag gag aaa tca cgg 3174
Arg Ser Ala Thr Val Val Met Met Thr Arg Leu Glu Glu Lys Ser Arg
        1035                1040                1045
gtc aaa tgt gac cag tac tgg cct aac cga ggc acc gag aca tac ggc 3222

Val Lys Cys Asp Gln Tyr Trp Pro Asn Arg Gly Thr Glu Thr Tyr Gly
    1050                1055                1060
ttc atc cag gtc acc cta cta gat act atg gag ctg gcc acc ttc tgt 3270
Phe Ile Gln Val Thr Leu Leu Asp Thr Met Glu Leu Ala Thr Phe Cys
1065                1070                1075                1080
gtc agg acc ttt tct cta cac aag aat ggc tct agt gag aag cgt gag 3318
Val Arg Thr Phe Ser Leu His Lys Asn Gly Ser Ser Glu Lys Arg Glu
                1085                1090                1095
gta cga cat ttt cag ttc aca gca tgg cct gac cac ggg gta ccc gag 3366
Val Arg His Phe Gln Phe Thr Ala Trp Pro Asp His Gly Val Pro Glu
            1100                1105                1110
tac ccc aca ccc ttc ctg gcg ttt ctg cgc aga gtc aag acc tgc aac 3414
Tyr Pro Thr Pro Phe Leu Ala Phe Leu Arg Arg Val Lys Thr Cys Asn
        1115                1120                1125
ccg cct gac gct ggc cca gtt gtg gtc cac tgc agc gcg ggt gtg ggg 3462
Pro Pro Asp Ala Gly Pro Val Val Val His Cys Ser Ala Gly Val Gly
    1130                1135                1140
cgt act ggc tgc ttc att gta att gat gcc atg ttg gag cgc atc aga 3510
Arg Thr Gly Cys Phe Ile Val Ile Asp Ala Met Leu Glu Arg Ile Arg
1145                1150                1155                1160
aca gag aag acg gtg gat gtg tac gga cac gtg aca ctc atg cgg tca 3558
Thr Glu Lys Thr Val Asp Val Tyr Gly His Val Thr Leu Met Arg Ser
                1165                1170                1175
cag cgc aac tac atg gtg cag aca gag gat cag tat agc ttc atc cac 3606
Gln Arg Asn Tyr Met Val Gln Thr Glu Asp Gln Tyr Ser Phe Ile His
            1180                1185                1190
gag gca ctg ctg gag gct gtg ggc tgt ggc aat acc gag gtc ccc gcg 3654
Glu Ala Leu Leu Glu Ala Val Gly Cys Gly Asn Thr Glu Val Pro Ala
        1195                1200                1205
cgc agc ctc tac acc tat atc cag aag ctg gcc cag gtg gag cct ggc 3702

Arg Ser Leu Tyr Thr Tyr Ile Gln Lys Leu Ala Gln Val Glu Pro Gly
    1210                1215                1220
gag cat gtc aca gga atg gag ctt gag ttc aag agg ctt gca gct cca 3750
Glu His Val Thr Gly Met Glu Leu Glu Phe Lys Arg Leu Ala Ala Pro
1225                1230                1235                1240
agg cac aca ctt cga gat tca ttc act gcc agc ctg cct tgc aac aag 3798
Arg His Thr Leu Arg Asp Ser Phe Thr Ala Ser Leu Pro Cys Asn Lys
                1245                1250                1255
ttt aag aac cgc ctg gtg aac atc ctg ccg tac gag agc tcg cgt gtc 3846
Phe Lys Asn Arg Leu Val Asn Ile Leu Pro Tyr Glu Ser Ser Arg Val
            1260                1265                1270
tgc ctg cag ccc att cgt ggt gtc gag ggc tct gac tac atc aat gcc 3894
Cys Leu Gln Pro Ile Arg Gly Val Glu Gly Ser Asp Tyr Ile Asn Ala
        1275                1280                1285
agc ttc atc gac ggc tac aga cag cag aaa gcc tac att gca acg cag 3942
Ser Phe Ile Asp Gly Tyr Arg Gln Gln Lys Ala Tyr Ile Ala Thr Gln
    1290                1295                1300
ggt cca ctg gca gag acc aca gag gac ttc tgg cgt gcc ctg tgg gag 3990
Gly Pro Leu Ala Glu Thr Thr Glu Asp Phe Trp Arg Ala Leu Trp Glu
1305                1310                1315                1320
aac aac tcc act att gtg gta atg ctc acc aag ctc cgc gag atg ggc 4038
Asn Asn Ser Thr Ile Val Val Met Leu Thr Lys Leu Arg Glu Met Gly
                1325                1330                1335
cgg gag aag tgc cac cag tac tgg cca gct gag cgc tct gcc cgc tac 4086
Arg Glu Lys Cys His Gln Tyr Trp Pro Ala Glu Arg Ser Ala Arg Tyr
            1340                1345                1350
cag tac ttt gtg gtt gac ccg atg gca gag tat aac atg cca gag tac 4134
Gln Tyr Phe Val Val Asp Pro Met Ala Glu Tyr Asn Met Pro Glu Tyr
        1355                1360                1365
att ctg cgt gag ttt aag gtc aca gat gcc cgg gat ggc cag tcc cgg 4182

Ile Leu Arg Glu Phe Lys Val Thr Asp Ala Arg Asp Gly Gln Ser Arg
    1370                1375                1380
acc gtc cga cag ttc acg gac tgg cca gag cag ggt gca ccc aag tca 4230
Thr Val Arg Gln Phe Thr Asp Trp Pro Glu Gln Gly Ala Pro Lys Ser
1385                1390                1395                1400
ggg gaa ggc ttc att gac ttc atc ggc caa gtg cat aag acc aag gag 4278
Gly Glu Gly Phe Ile Asp Phe Ile Gly Gln Val His Lys Thr Lys Glu
                1405                1410                1415
cag ttt ggc cag gat ggc ccc atc tcg gtg cac tgt agt gct gga gtg 4326
Gln Phe Gly Gln Asp Gly Pro Ile Ser Val His Cys Ser Ala Gly Val
            1420                1425                1430
ggc agg acc gga gta ttc atc act ctg agc atc gtg ctg gag cga atg 4374
Gly Arg Thr Gly Val Phe Ile Thr Leu Ser Ile Val Leu Glu Arg Met
        1435                1440                1445
cgc tac gag ggg gtg gtg gac att ttc cag aca gtg aag gtg ctt cgg 4422
Arg Tyr Glu Gly Val Val Asp Ile Phe Gln Thr Val Lys Val Leu Arg
    1450                1455                1460
acc cag cgg cct gcc atg gtg cag aca gag gat gag tac cag ttc tgc 4470
Thr Gln Arg Pro Ala Met Val Gln Thr Glu Asp Glu Tyr Gln Phe Cys
1465                1470                1475                1480
ttc cag gcg gcg ttg gaa ttg ggc agc ttt gat cat tat gca aca taagcc4521
Phe Gln Ala Ala Leu Glu Leu Gly Ser Phe Asp His Tyr Ala Thr
                1485                1490                1495
atgggccccg caacgcctcg acccagctcc aagtgccctg catgtgagcc cagccctcgg 4581
tgctggtggg aggcggccca gggaggaaac ctcctctccc tggagacagc actgccttct 4641
aagggcacat tcctcattcc ttctgactcc aaaacgaggt tccagggtgg ggggtagggt 4701
ggagagtaga ggagccactg ctcccatagc tggggtcaca agggacagaa ctctgctccc 4761
acacttccct gcctgcctgc ctgtcagcaa cattcttttt tttcattttt ttaactgtag 4821
tgtattttc ttcatcttct tttttttttt aagaaaaaaa aaacaatgcg cagtcaaatt 4881
ttgaaaacaa cgagacacgt tggctctgtt tgtcgctctg tggagggcca actttcata 4941

gtaagtgtgt cgtgtggcgg ctctgtgcaa caactttgat ggcttctgtg tgcattcttc 5001
ccacatgtcc ccgtgtgaat ggctcacgta ggtttctttt ttaccctttt tacttttttt 5061
ttaaatcaat cttcagacat atcagatgtg aaggggtgat ggctggagca cctgggccag 5121
gctgcaggac atggccacca ggacacagtg gctggcctca ctgcccagtc cctgccgcac 5181
cagagagggt ctttgtcctc tcctgactca tgccccgcat ggaggacccc cgggactacg 5241
gacacttggg gacacgcagc cccctagagc aagtgaggtc tctctttgta ggagagtggg 5301
tcagcactcg tccccgcttg tttttgggc agaagcgggt gacagccctg tatgtagata 5361
aaccaagttt gtattaataa agattcgtcc gacctaaaaa aaaaaaaaaa a 5412
<210> 12
<211> 1495
<212> PRT
<213> Rattus norvegicus
<400> 12
Met Ala Pro Thr Trp Arg Pro Ser Val Val Ser Val Val Gly Pro Val
1               5                   10                  15
Gly Leu Phe Leu Val Leu Leu Ala Arg Gly Cys Leu Ala Glu Glu Pro
            20                  25                  30
Pro Arg Phe Ile Arg Glu Pro Lys Asp Gln Ile Gly Val Ser Gly Gly
        35                  40                  45
Val Ala Ser Phe Val Cys Gln Ala Thr Gly Asp Pro Lys Pro Arg Val
    50                  55                  60
Thr Trp Asn Lys Lys Gly Lys Lys Val Asn Ser Gln Arg Phe Glu Thr
65                  70                  75                  80
Ile Asp Phe Asp Glu Ser Ser Gly Ala Val Leu Arg Ile Gln Pro Leu
                85                  90                  95
Arg Thr Pro Arg Asp Glu Asn Val Tyr Glu Cys Val Ala Gln Asn Ser
            100                 105                 110
Val Gly Glu Ile Thr Val His Ala Lys Leu Thr Val Leu Arg Glu Asp
        115                 120                 125
Gln Leu Pro Pro Gly Phe Pro Asn Ile Asp Met Gly Pro Gln Leu Lys

    130                 135                 140
Val Val Glu Arg Thr Arg Thr Ala Thr Met Leu Cys Ala Ala Ser Gly
145                 150                 155                 160
Asn Pro Asp Pro Glu Ile Thr Trp Phe Lys Asp Phe Leu Pro Val Asp
                165                 170                 175
Pro Ser Ala Ser Asn Gly Arg Ile Lys Gln Leu Arg Ser Gly Ala Leu
            180                 185                 190
Gln Ile Glu Ser Ser Glu Glu Thr Asp Gln Gly Lys Tyr Glu Cys Val
        195                 200                 205
Ala Thr Lys Gln Ala Gly Val Arg Tyr Ser Ser Pro Ala Asn Leu Tyr
    210                 215                 220
Val Arg Val Arg Arg Val Ala Pro Arg Phe Ser Ile Leu Pro Met Ser
225                 230                 235                 240
His Glu Ile Met Pro Gly Gly Asn Val Asn Ile Thr Cys Val Ala Val
                245                 250                 255
Gly Ser Pro Met Pro Tyr Val Lys Trp Met Gln Gly Ala Glu Asp Leu
            260                 265                 270
Thr Pro Glu Asp Asp Met Pro Val Gly Arg Asn Val Leu Glu Leu Thr
        275                 280                 285
Asp Val Lys Asp Ser Ala Asn Tyr Pro Cys Val Ala Met Ser Ser Leu
    290                 295                 300
Gly Val Ile Glu Ala Val Ala Asp Ile Thr Val Lys Ser Leu Pro Lys
305                 310                 315                 320
Ala Pro Gly Thr Pro Val Val Thr Glu Asn Thr Ala Thr Ser Ile Thr
                325                 330                 335
Val Thr Trp Asp Ala Gly Asn Pro Asp Pro Val Ser Tyr Tyr Val Leu
            340                 345                 350
Ser Ile Ile Lys Ala Arg Met Gly Arg Ile Arg Ser Lys Lys Thr Ser
        355                 360                 365
Thr Thr Thr Arg Tyr Ser Ile Gly Gly Leu Ser Pro Asn Ser Glu Tyr

    370                 375                 380
Glu Ile Trp Val Ser Ala Val Asn Ser Ile Gly Gln Ala Pro Ser Glu
385                 390                 395                 400
Ser Val Val Thr Arg Thr Gly Glu Gln Ala Pro Ala Ser Ala Pro Arg
                405                 410                 415
Asn Val Gln Ala Arg MET Leu Ser Ala Thr Thr Met Ile Val Gln Trp
            420                 425                 430
Glu Glu Pro Val Glu Pro Asn Gly Leu Ile Arg Gly Tyr Arg Val Tyr
        435                 440                 445
Tyr Thr Met Glu Pro Glu His Pro Val Gly Asn Trp Gln Lys His Asn
    450                 455                 460
Val Asp Asp Ser Leu Leu Thr Thr Val Gly Ser Leu Leu Glu Asp Glu
465                 470                 475                 480
Thr Tyr Thr Val Arg Val Leu Ala Phe Thr Ser Val Gly Asp Gly Pro
                485                 490                 495
Leu Ser Asp Pro Ile Gln Val Lys Thr Gln Gln Gly Val Pro Gly Gln
            500                 505                 510
Pro Met Asn Leu Arg Ala Glu Ala Lys Ser Glu Thr Ser Ile Gly Leu
        515                 520                 525
Ser Trp Ser Ala Pro Arg Gln Glu Ser Val Ile Lys Tyr Glu Leu Leu
    530                 535                 540
Phe Arg Glu Gly Asp Arg Gly Arg Glu Val Gly Arg Thr Phe Asp Pro
545                 550                 555                 560
Thr Thr Ala Phe Val Val Glu Asp Leu Lys Pro Asn Thr Glu Tyr Ala
                565                 570                 575
Phe Arg Leu Ala Ala Arg Ser Pro Gln Gly Leu Gly Ala Phe Thr Ala
            580                 585                 590
Val Val Arg Gln Arg Thr Leu Gln Ala Ile Ser Pro Lys Asn Phe Lys
        595                 600                 605
Val Lys MET Ile MET Lys Thr Ser Val Leu Leu Ser Trp Glu Phe Pro

610                 615                 620
Asp Asn Tyr Asn Ser Pro Thr Pro Tyr Lys Ile Gln Tyr Asn Gly Leu
625                 630                 635                 640
Thr Leu Asp Val Asp Gly Arg Thr Thr Lys Lys Leu Ile Thr His Leu
                645                 650                 655
Lys Pro His Thr Phe Tyr Asn Phe Val Leu Thr Asn Arg Gly Ser Ser
            660                 665                 670
Leu Gly Gly Leu Gln Gln Thr Val Thr Ala Arg Thr Ala Phe Asn Met
        675                 680                 685
Leu Ser Gly Lys Pro Ser Val Ala Pro Lys Pro Asp Asn Asp Gly Ser
    690                 695                 700
Ile Val Val Tyr Leu Pro Asp Gly Gln Ser Pro Val Thr Val Gln Asn
705                 710                 715                 720
Tyr Phe Ile Val Met Val Pro Leu Arg Lys Ser Arg Gly Gly Gln Phe
                725                 730                 735
Pro Ile Leu Leu Pro Ser Pro Glu Asp Met Asp Leu Glu Glu Leu Ile
            740                 745                 750
Gln Asp Leu Ser Arg Leu Gln Ser Ser Leu Arg His Ser Arg Gln Leu
        755                 760                 765
Glu Val Pro Arg Pro Tyr Ile Ala Ala Arg Phe Ser Ile Leu Pro Ala
    770                 775                 780
Val Phe His Pro Gly Asn Gln Lys Gln Tyr Gly Gly Phe Asp Asn Arg
785                 790                 795                 800
Gly Leu Glu Pro Gly His Arg Tyr Val Leu Phe Val Leu Ala Val Leu
                805                 810                 815
Gln Lys Asn Glu Pro Thr Phe Ala Ala Ser Pro Phe Ser Asp Pro Phe
            820                 825                 830
Gln Leu Asp Asn Pro Asp Pro Gln Pro Ile Val Asp Gly Glu Glu Gly
        835                 840                 845
Leu Ile Trp Val Ile Gly Pro Val Leu Ala Val Val Phe Ile Ile Cys

850                 855                 860
Ile Val Ile Ala Ile Leu Leu Tyr Lys Asn Lys Pro Asp Ser Lys Arg
865                 870                 875                 880
Lys Asp Ser Glu Pro Arg Thr Lys Cys Leu Leu Asn Asn Ala Asp Leu
                885                 890                 895
Ala Pro His His Pro Lys Asp Pro Val Glu Met Arg Arg Ile Asn Phe
            900                 905                 910
Gln Thr Pro Gly Met Leu Ser His Pro Pro Ile Pro Ile Thr Asp Met
        915                 920                 925
Ala Glu His Met Glu Arg Leu Lys Ala Asn Asp Ser Leu Lys Leu Ser
    930                 935                 940
Gln Glu Tyr Glu Ser Ile Asp Pro Gly Gln Gln Phe Thr Trp Glu His
945                 950                 955                 960
Ser Asn Leu Glu Ala Asn Lys Pro Lys Asn Arg Tyr Ala Asn Val Ile
                965                 970                 975
Ala Tyr Asp His Ser Arg Val Ile Leu Gln Pro Leu Glu Gly Ile Met
            980                 985                 990
Gly Ser Asp Tyr Ile Asn Ala Asn Tyr Val Asp Gly Tyr Arg Arg Gln
        995                 1000                1005
Asn Ala Tyr Ile Ala Thr Gln Gly Pro Leu Pro Glu Thr Phe Gly Asp
    1010                1015                1020
Phe Trp Arg Met Val Trp Glu Gln Arg Ser Ala Thr Val Val Met Met
1025                1030                1035                1040
Thr Arg Leu Glu Glu Lys Ser Arg Val Lys Cys Asp Gln Tyr Trp Pro
                1045                1050                1055
Asn Arg Gly Thr Glu Thr Tyr Gly Phe Ile Gln Val Thr Leu Leu Asp
            1060                1065                1070
Thr Met Glu Leu Ala Thr Phe Cys Val Arg Thr Phe Ser Leu His Lys
        1075                1080                1085
Asn Gly Ser Ser Glu Lys Arg Glu Val Arg His Phe Gln Phe Thr Ala

1090                1095                1100
Trp Pro Asp His Gly Val Pro Glu Tyr Pro Thr Pro Phe Leu Ala Phe
1105                1110                1115                1120
Leu Arg Arg Val Lys Thr Cys Asn Pro Pro Asp Ala Gly Pro Val Val
                1125                1130                1135
Val His Cys Ser Ala Gly Val Gly Arg Thr Gly Cys Phe Ile Val Ile
            1140                1145                1150
Asp Ala Met Leu Glu Arg Ile Arg Thr Glu Lys Thr Val Asp Val Tyr
        1155                1160                1165
Gly His Val Thr Leu Met Arg Ser Gln Arg Asn Tyr Met Val Gln Thr
    1170                1175                1180
Glu Asp Gln Tyr Ser Phe Ile His Glu Ala Leu Leu Glu Ala Val Gly
1185                1190                1195                1200
Cys Gly Asn Thr Glu Val Pro Ala Arg Ser Leu Tyr Thr Tyr Ile Gln
                1205                1210                1215
Lys Leu Ala Gln Val Glu Pro Gly Glu His Val Thr Gly Met Glu Leu
            1220                1225                1230
Glu Phe Lys Arg Leu Ala Ala Pro Arg His Thr Leu Arg Asp Ser Phe
        1235                1240                1245
Thr Ala Ser Leu Pro Cys Asn Lys Phe Lys Asn Arg Leu Val Asn Ile
    1250                1255                1260
Leu Pro Tyr Glu Ser Ser Arg Val Cys Leu Gln Pro Ile Arg Gly Val
1265                1270                1275                1280
Glu Gly Ser Asp Tyr Ile Asn Ala Ser Phe Ile Asp Gly Tyr Arg Gln
                1285                1290                1295
Gln Lys Ala Tyr Ile Ala Thr Gln Gly Pro Leu Ala Glu Thr Thr Glu
            1300                1305                1310
Asp Phe Trp Arg Ala Leu Trp Glu Asn Asn Ser Thr Ile Val Val Met
        1315                1320                1325
Leu Thr Lys Leu Arg Glu Met Gly Arg Glu Lys Cys His Gln Tyr Trp

1330                1335                1340

Pro Ala Glu Arg Ser Ala Arg Tyr Gln Tyr Phe Val Val Asp Pro Met

1345                1350                1355                1360

Ala Glu Tyr Asn Met Pro Glu Tyr Ile Leu Arg Glu Phe Lys Val Thr

                1365                1370                1375

Asp Ala Arg Asp Gly Gln Ser Arg Thr Val Arg Gln Phe Thr Asp Trp

            1380                1385                1390

Pro Glu Gln Gly Ala Pro Lys Ser Gly Glu Gly Phe Ile Asp Phe Ile

        1395                1400                1405

Gly Gln Val His Lys Thr Lys Glu Gln Phe Gly Gln Asp Gly Pro Ile

    1410                1415                1420

Ser Val His Cys Ser Ala Gly Val Gly Arg Thr Gly Val Phe Ile Thr

1425                1430                1435                1440

Leu Ser Ile Val Leu Glu Arg Met Arg Tyr Glu Gly Val Val Asp Ile

                1445                1450                1455

Phe Gln Thr Val Lys Val Leu Arg Thr Gln Arg Pro Ala Met Val Gln

            1460                1465                1470

Thr Glu Asp Glu Tyr Gln Phe Cys Phe Gln Ala Ala Leu Glu Leu Gly

        1475                1480                1485

Ser Phe Asp His Tyr Ala Thr

    1490                1495

<210> 13

<211> 1666

<212> DNA

<213> Rattus norvegius

<220>

<221> CDS

<222> (180)..(608)

<400> 13

cggcagccga gtcggattga gctgctgcag acgccaggcc actccagcca gcactgccgt 6

tttcacgccc cggctgcaga cagctaggag gctttatcta gtttgaacca ggctgctgga 120
gctcgctcct tccctctctt tttttccacg aggctgtttt tttatttggc tgcaattgc 179
atg aaa tcc caa tgg tgt aga cca gtg gcg atg gat cta gga gtt tac 227
Met Lys Ser Gln Trp Cys Arg Pro Val Ala Met Asp Leu Gly Val Tyr
1               5                   10                  15
caa ctg aga cat ttt tca att tct ttc ttg tcg tct ttg ctg gga act 275
Gln Leu Arg His Phe Ser Ile Ser Phe Leu Ser Ser Leu Leu Gly Thr
            20                  25                  30
gaa aac gct tcc gtg aga ctt gac aat agc tct ggt gca agt gtg gta 323
Glu Asn Ala Ser Val Arg Leu Asp Asn Ser Ser Gly Ala Ser Val Val
        35                  40                  45
gct atc gac aac aaa ata gag caa gct atg gat ctg gtg aaa agc cat 371
Ala Ile Asp Asn Lys Ile Glu Gln Ala Met Asp Leu Val Lys Ser His
    50                  55                  60
ttg atg tat gca gtt aga gag gaa gtg gag gtt ctg aag gag cag atc 419
Leu Met Tyr Ala Val Arg Glu Glu Val Glu Val Leu Lys Glu Gln Ile
65                  70                  75                  80
aaa gaa cta ata gag aaa aac tcc caa ctg gag cag gag aac aat ctg 467
Lys Glu Leu Ile Glu Lys Asn Ser Gln Leu Glu Gln Glu Asn Asn Leu
                85                  90                  95
ttg aag aca ctg gcc agt ccg gag cag ctc gcc cag ttt cag gcc cag 515
Leu Lys Thr Leu Ala Ser Pro Glu Gln Leu Ala Gln Phe Gln Ala Gln
            100                 105                 110
ctg cag act ggc tcc cct ccg gct acc acg cag cca cag ggg acc aca 563
Leu Gln Thr Gly Ser Pro Pro Ala Thr Thr Gln Pro Gln Gly Thr Thr
        115                 120                 125
cag ccc cct gca cag cca gcg tcc cag ggc tca gga tca acc gca tagcct614
Gln Pro Pro Ala Gln Pro Ala Ser Gln Gly Ser Gly Ser Thr Ala
    130                 135                 140
gctatgcccc aacagaactg gctgctgctg tctgaactga acagaccgaa gagatgtgct 674

agtgagaagc cgcctccagt cacccatttc attgctgtct gcgaaagaga cgtgagactc 734
acacatgctg ttctcgcttt ctccccagta ttaagcactc atatgctttt ggcttgaaga 794
aatatactag ttgagtgaat taaaggttaa acagagagtg agcatggatg taccctgtgc 854
aacgtggcag atgtctgagg aatggtttga ttgacgctga ggaggagctc tgtgcctttt 914
caaccctccc cagccgccca ctctactccc aagctctggg gctcgcctgc atggggctca 974
gaaggtgggc tgctcctgga ttttgtgttc tcctctcctt cccttcaaag aatttgagag 1034
gccagaaacg agactgcaaa gggggggatg cagtcctttt acaaaaccga caactgtcac 1094
caaagcttat aaaacaggac agtactgtcc ctcttttctg aaacatcaga agacacaaaa 1154
ctgttagtga cacaacggtg acaggtagct gggacctagg ctatcttatt atgaaggttg 1214
ttttgcttgt tgtatatttg tgtatgtagt gtaacgaatt tgtacaatag aggaccgtaa 1274
ctactgttag gttgtacaga ttgaagttta gatgttccat tggctgtctg aaaaggtgtg 1334
gattgtcctt cctagagaga tctacttaaa aactgcttcg tgacaaaaac cacacctgaa 1394
gaaattttaa gaatttggca cagttagtca ctttgtgtca cccggaatct agctgctgag 1454
tcttgcaaag taaacccccct gttgactgat gtcagttgag ctagtgaatg aatagatgga 1514
gaaacgtcag tcagttgctg aggaagtgga tttcccagta ggggtttctg cagctcacct 1574
gtatagtcct gcgcatgttc cccacacaga acccactgta tttacctgtt ctacttgtca 1634
cctttcaata aagcatatca aatgttgata cc 1666
<210> 14
<211> 143
<212> PRT
<213> Rattus norvegicus
<400> 14
Met Lys Ser Gln Trp Cys Arg Pro Val Ala Met Asp Leu Gly Val Tyr
1               5                   10                  15
Gln Leu Arg His Phe Ser Ile Ser Phe Leu Ser Ser Leu Leu Gly Thr
            20                  25                  30
Glu Asn Ala Ser Val Arg Leu Asp Asn Ser Ser Gly Ala Ser Val Val
        35                  40                  45
Ala Ile Asp Asn Lys Ile Glu Gln Ala Met Asp Leu Val Lys Ser His
    50                  55                  60

Leu Met Tyr Ala Val Arg Glu Glu Val Glu Val Leu Lys Glu Gln Ile
65                  70                  75                  80
Lys Glu Leu Ile Glu Lys Asn Ser Gln Leu Glu Gln Glu Asn Asn Leu
                85                  90                  95
Leu Lys Thr Leu Ala Ser Pro Glu Gln Leu Ala Gln Phe Gln Ala Gln
            100                 105                 110
Leu Gln Thr Gly Ser Pro Pro Ala Thr Thr Gln Pro Gln Gly Thr Thr
        115                 120                 125
Gln Pro Pro Ala Gln Pro Ala Ser Gln Gly Ser Gly Ser Thr Ala
    130                 135                 140
<210> 15
<211> 2010
<212> DNA
<213> Rattus norvegius
<220>
<221> CDS
<222> (139)..(1242)
<400> 15
tcgacccacg cgtccgcgca gagctggcaa gttgatttgg cggtggcggc gcctaccccc 60
gcgcggagga acgaaatcgg ttcggcgacg ccggcggaaa ccttagttcg agcgggaagc 120
ctgacagtgg caggcggc atg tcg gtg gcg ggg ctg aag aag cag ttc tac    171
                    Met Ser Val Ala Gly Leu Lys Lys Gln Phe Tyr
                    1               5                   10
aag gcg agc cag ctg gtc agc gag aaa gtt ggt ggg gcc gaa ggg acc    219
Lys Ala Ser Gln Leu Val Ser Glu Lys Val Gly Gly Ala Glu Gly Thr
            15                  20                  25
aaa ctg gat gat gac ttc aga gag atg gaa aag aaa gtg gat atc acc    267
Lys Leu Asp Asp Asp Phe Arg Glu Met Glu Lys Lys Val Asp Ile Thr
        30                  35                  40
agt aag gcc gtg gca gag gtg ctg gtc aga acc ata gaa tat ctg caa    315

Ser Lys Ala Val Ala Glu Val Leu Val Arg Thr Ile Glu Tyr Leu Gln
    45                  50                  55
cct aac cca gcc tcg agg gcc aag ctg act atg ctg aat act gta tcc 363
Pro Asn Pro Ala Ser Arg Ala Lys Leu Thr Met Leu Asn Thr Val Ser
60                  65                  70                  75
aag atc cgg ggc caa gtg aag aat cct ggc tac cca cag tca gag ggt 411
Lys Ile Arg Gly Gln Val Lys Asn Pro Gly Tyr Pro Gln Ser Glu Gly
                80                  85                  90
ctg ctg gga gag tgc atg gtc cgc cat ggc aag gaa cta ggc gga gag 459
Leu Leu Gly Glu Cys Met Val Arg His Gly Lys Glu Leu Gly Gly Glu
            95                  100                 105
tcc aac ttt ggc gat gct ctg cta gat gca ggt gag tct atg aag cgc 507
Ser Asn Phe Gly Asp Ala Leu Leu Asp Ala Gly Glu Ser Met Lys Arg
        110                 115                 120
ctg gct gag gtg aag gac tca ctg gac atc gag gtc aag cag aac ttc 555
Leu Ala Glu Val Lys Asp Ser Leu Asp Ile Glu Val Lys Gln Asn Phe
    125                 130                 135
atc gac cca ctg cag aac ctg tgt gac aag gat ctg aag gag atc cag 603
Ile Asp Pro Leu Gln Asn Leu Cys Asp Lys Asp Leu Lys Glu Ile Gln
140                 145                 150                 155
cac cac ctg aag aag ttg gag ggc cgc cgc ctt gac ttt gac tac aag 651
His His Leu Lys Lys Leu Glu Gly Arg Arg Leu Asp Phe Asp Tyr Lys
                160                 165                 170
aag aag cgc cag ggc aag atc cct gat gag gag ctg cgt cag gcc cta 699
Lys Lys Arg Gln Gly Lys Ile Pro Asp Glu Glu Leu Arg Gln Ala Leu
            175                 180                 185
gag aag ttt gag gag tcc aag gag gtg gcg gag acc agt atg cac aac 747
Glu Lys Phe Glu Glu Ser Lys Glu Val Ala Glu Thr Ser Met His Asn
        190                 195                 200
ctc ctg gag act gat att gag cag gtg agc cag ctc tca gcc cta gtg 795

Leu Leu Glu Thr Asp Ile Glu Gln Val Ser Gln Leu Ser Ala Leu Val
    205                 210                 215
gat gcc cag ctg gac tac cac cgg cag gca gtg cag atc ctg gag gag 843
Asp Ala Gln Leu Asp Tyr His Arg Gln Ala Val Gln Ile Leu Glu Glu
220                 225                 230                 235
ctg gct gat aag ctg aag cgc agg gtg aga gaa gcc tcc tca cgc ccc 891
Leu Ala Asp Lys Leu Lys Arg Arg Val Arg Glu Ala Ser Ser Arg Pro
                240                 245                 250
agg agg gag ttc aag ccc agg ccc cag gag ccc ttt gag ctt gga gag 939
Arg Arg Glu Phe Lys Pro Arg Pro Gln Glu Pro Phe Glu Leu Gly Glu
            255                 260                 265
ctg gag cag ccc aat ggg gga ttc ccc tgt gcc tca gca ccc aag atc 987
Leu Glu Gln Pro Asn Gly Gly Phe Pro Cys Ala Ser Ala Pro Lys Ile
        270                 275                 280
aca gct tcg tca tca ttt aga tca ggg gac aag ccc acc agg acg ccc 1035
Thr Ala Ser Ser Ser Phe Arg Ser Gly Asp Lys Pro Thr Arg Thr Pro
    285                 290                 295
agc aag agt atg cca ccc ctg gac cag cca agc tgc aag gcg ctg tat 1083
Ser Lys Ser Met Pro Pro Leu Asp Gln Pro Ser Cys Lys Ala Leu Tyr
300                 305                 310                 315
gac ttt gag cca gag aac gat ggc gag ctg ggc ttc cga gag ggt gac 1131
Asp Phe Glu Pro Glu Asn Asp Gly Glu Leu Gly Phe Arg Glu Gly Asp
                320                 325                 330
ctc atc acg ctt acc aac cag atc gat gag aac tgg tat gag ggg atg 1179
Leu Ile Thr Leu Thr Asn Gln Ile Asp Glu Asn Trp Tyr Glu Gly Met
            335                 340                 345
ctg cat ggc cag tca ggc ttc ttc cca ctt agc tat gtg cag gtg ctg 1227
Leu His Gly Gln Ser Gly Phe Phe Pro Leu Ser Tyr Val Gln Val Leu
        350                 355                 360
gtg cct ctg cct cag tgactgcgct tgcacactga ctagcgcctg cacacgctgc 1282

Val Pro Leu Pro Gln
    365
cagtcacagt gtggcagtag tctaatgcca aggtgctcta taaacactaa tgttcctcca 1342
ggggagacct cttccctcct ccctcagccc tggggccccc ccatcctaag actcagaaag 1402
gcccaccctg aggttctatt acctttctgg tggtggcagc tcccacctat ttcaaccctt 1462
cccagcccgt tgctggcgat gggcccatgc ccctctccag gctccctagg ggaggcaggt 1522
ccttgggatc cccagcctgc aagcacagcc agctcagcat atggagacac ctggcacctg 1582
ctgctcattc agaagtgcac aaggcatgaa cgtgtacact tcccatggga ccacagaccc 1642
agctcagccc tgttgaagac caagcacaaa ggccctgaag agtggacatt cccaggtccc 1702
tggcaccttc ccctgagcca gctccactgc tacctgctca tgtgactcta cagctggcca 1762
caggcagttg gcaggtccct tttcaaccag catgcgaggc tggccacagc cgcggctctg 1822
catcatgagg gaggctttgg ctggactcag ttacactctt ctctacagct gccccacaac 1882
ccgtggcttg tccctggtac gtggggccac acccatgccc cctagatggg caacactgtc 1942
ctccagcctg tgaagtggac tttactccta attttttttt tttaaaagta ttaaatatct 2002
ctttctat 2010
<210> 16
<211> 368
<212> PRT
<213> Rattus norvegicus
<400> 16
Met Ser Val Ala Gly Leu Lys Lys Gln Phe Tyr Lys Ala Ser Gln Leu
1               5                   10                  15
Val Ser Glu Lys Val Gly Gly Ala Glu Gly Thr Lys Leu Asp Asp Asp
            20                  25                  30
Phe Arg Glu Met Glu Lys Lys Val Asp Ile Thr Ser Lys Ala Val Ala
        35                  40                  45
Glu Val Leu Val Arg Thr Ile Glu Tyr Leu Gln Pro Asn Pro Ala Ser
    50                  55                  60
Arg Ala Lys Leu Thr Met Leu Asn Thr Val Ser Lys Ile Arg Gly Gln
65                  70                  75                  80

Val Lys Asn Pro Gly Tyr Pro Gln Ser Glu Gly Leu Leu Gly Glu Cys
                85                  90                  95
Met Val Arg His Gly Lys Glu Leu Gly Gly Glu Ser Asn Phe Gly Asp
            100                 105                 110
Ala Leu Leu Asp Ala Gly Glu Ser Met Lys Arg Leu Ala Glu Val Lys
        115                 120                 125
Asp Ser Leu Asp Ile Glu Val Lys Gln Asn Phe Ile Asp Pro Leu Gln
    130                 135                 140
Asn Leu Cys Asp Lys Asp Leu Lys Glu Ile Gln His His Leu Lys Lys
145                 150                 155                 160
Leu Glu Gly Arg Arg Leu Asp Phe Asp Tyr Lys Lys Lys Arg Gln Gly
                165                 170                 175
Lys Ile Pro Asp Glu Glu Leu Arg Gln Ala Leu Glu Lys Phe Glu Glu
            180                 185                 190
Ser Lys Glu Val Ala Glu Thr Ser Met His Asn Leu Leu Glu Thr Asp
        195                 200                 205
Ile Glu Gln Val Ser Gln Leu Ser Ala Leu Val Asp Ala Gln Leu Asp
    210                 215                 220
Tyr His Arg Gln Ala Val Gln Ile Leu Glu Glu Leu Ala Asp Lys Leu
225                 230                 235                 240
Lys Arg Arg Val Arg Glu Ala Ser Ser Arg Pro Arg Arg Glu Phe Lys
                245                 250                 255
Pro Arg Pro Gln Glu Pro Phe Glu Leu Gly Glu Leu Glu Gln Pro Asn
            260                 265                 270
Gly Gly Phe Pro Cys Ala Ser Ala Pro Lys Ile Thr Ala Ser Ser Ser
        275                 280                 285
Phe Arg Ser Gly Asp Lys Pro Thr Arg Thr Pro Ser Lys Ser Met Pro
    290                 295                 300
Pro Leu Asp Gln Pro Ser Cys Lys Ala Leu Tyr Asp Phe Glu Pro Glu
305                 310                 315                 320

Asn Asp Gly Glu Leu Gly Phe Arg Glu Gly Asp Leu Ile Thr Leu Thr
                325                 330                 335
Asn Gln Ile Asp Glu Asn Trp Tyr Glu Gly Met Leu His Gly Gln Ser
            340                 345                 350
Gly Phe Phe Pro Leu Ser Tyr Val Gln Val Leu Val Pro Leu Pro Gln
        355                 360                 365
<210> 17
<211> 731
<212> DNA
<213> Rattus norvegius
<220>
<221> CDS
<222> (23)..(442)
<400> 17
atcgagccgg cccgtgagcg ag atg cag cac cga agt ttc ttc ctt cta gcc 52
Met Gln His Arg Ser Phe Phe Leu Leu Ala
1               5                   10
ctt gtt gcc ctc ttg gct gtc acg acc gcg gtg gcc aaa aag aaa gac 100
Leu Val Ala Leu Leu Ala Val Thr Thr Ala Val Ala Lys Lys Lys Asp
                15                  20                  25
aag gtg aag aag ggc agc gag tgt tcg gag tgg acc tgg ggg ccc tgc 148
Lys Val Lys Lys Gly Ser Glu Cys Ser Glu Trp Thr Trp Gly Pro Cys
            30                  35                  40
acc ccc agc agc aag gac tgc ggc atg ggt ttc cgc gag ggt acc tgt 196
Thr Pro Ser Ser Lys Asp Cys Gly Met Gly Phe Arg Glu Gly Thr Cys
        45                  50                  55
ggg gcc cag acc cag cgc atc cat tgc aag gtg ccc tgc aac tgg aag 244
Gly Ala Gln Thr Gln Arg Ile His Cys Lys Val Pro Cys Asn Trp Lys
    60                  65                  70
aag gag ttt gga gcc gac tgc aaa tac aag ttt gag agc tgg ggg gcg 292

Lys Glu Phe Gly Ala Asp Cys Lys Tyr Lys Phe Glu Ser Trp Gly Ala
75                  80                  85                  90
tgt gat ggg agc act ggc acc aaa gcc cgc caa ggg acc ctg aag aag 340
Cys Asp Gly Ser Thr Gly Thr Lys Ala Arg Gln Gly Thr Leu Lys Lys
                95                  100                 105
gct cgg tac aat gcc cag tgc cag gag acc atc cgc gtg acc aag ccc 388
Ala Arg Tyr Asn Ala Gln Cys Gln Glu Thr Ile Arg Val Thr Lys Pro
            110                 115                 120
tgc acc tcc aag acc aag tca aag gcc aaa gcc aag aaa gga aaa gga 436
Cys Thr Ser Lys Thr Lys Ser Lys Ala Lys Ala Lys Lys Gly Lys Gly
        125                 130                 135
aag gac tgagtcagga ggccagagag tttctggcct gggacctgaa cggagccctc 492
Lys Asp
    140
ctctcccaca ggcccaagat gtaacccacc agtgcctttt gtcttcctgt cagctttgtc 552
aatcacaccc ttttactcct gccccctctt gctacaccta gtacccaaag tggggaggga 612
caagggattc tgggaagtga gcctccccat aacccctttt gtttccccca ccctgatacc 672
tgttatcgag aaatgaataa aatgaactca ctttttttcc aaaaaaaaaa aaaaaaaaa 731
<210> 18
<211> 140
<212> PRT
<213> Rattus norvegicus
<400> 18
Met Gln His Arg Ser Phe Phe Leu Leu Ala Leu Val Ala Leu Leu Ala
1               5                   10                  15
Val Thr Thr Ala Val Ala Lys Lys Lys Asp Lys Val Lys Lys Gly Ser
            20                  25                  30
Glu Cys Ser Glu Trp Thr Trp Gly Pro Cys Thr Pro Ser Ser Lys Asp
        35                  40                  45
Cys Gly Met Gly Phe Arg Glu Gly Thr Cys Gly Ala Gln Thr Gln Arg

    50                  55                  60

Ile His Cys Lys Val Pro Cys Asn Trp Lys Lys Glu Phe Gly Ala Asp

65                  70                  75                  80

Cys Lys Tyr Lys Phe Glu Ser Trp Gly Ala Cys Asp Gly Ser Thr Gly

                85                  90                  95

Thr Lys Ala Arg Gln Gly Thr Leu Lys Lys Ala Arg Tyr Asn Ala Gln

            100                 105                 110

Cys Gln Glu Thr Ile Arg Val Thr Lys Pro Cys Thr Ser Lys Thr Lys

        115                 120                 125

Ser Lys Ala Lys Ala Lys Lys Gly Lys Gly Lys Asp

130 135

<210> 19

<211> 1158

<212> DNA

<213> Rattus norvegius

<220>

<221> CDS

<222> (1)..(369)

<400> 19

ctt gga ttc acc gcc acg gac tcc aca ctg gag ggt gag act gcc aga 48

Leu Gly Phe Thr Ala Thr Asp Ser Thr Leu Glu Gly Glu Thr Ala Arg

1               5                   10                  15

gcc tac atg ccc aga cac atg tgc aaa gga acg cag aga caa tct gaa 96

Ala Tyr Met Pro Arg His Met Cys Lys Gly Thr Gln Arg Gln Ser Glu

            20                  25                  30

gcc acg atg tcc ctc tgc agg gac tca ggc ccc aga gtt tgc tca ctg 144

Ala Thr Met Ser Leu Cys Arg Asp Ser Gly Pro Arg Val Cys Ser Leu

        35                  40                  45

cat gag agg gct cag gtc ccc cgg gtt cac ctc agt agc ata gga gcc 192

His Glu Arg Ala Gln Val Pro Arg Val His Leu Ser Ser Ile Gly Ala

    50                  55                  60
cag aca agg gca ggg cac aag cga ctg aca ccg aaa gaa cag tca tgg 240
Gln Thr Arg Ala Gly His Lys Arg Leu Thr Pro Lys Glu Gln Ser Trp
65                  70                  75                  80
tgt cac ggg gac aga aag gca gct ggt ccc aga agg gaa aca ctg gtg 288
Cys His Gly Asp Arg Lys Ala Ala Gly Pro Arg Arg Glu Thr Leu Val
                85                  90                  95
tct gca tgg gag aac act ctc gga agt ccc cag ctt ctg tca gac tgc 336
Ser Ala Trp Glu Asn Thr Leu Gly Ser Pro Gln Leu Leu Ser Asp Cys
            100                 105                 110
ttt cac cca gca gca aag gac aac cta cga gtt taaggcaagt 379
Phe His Pro Ala Ala Lys Asp Asn Leu Arg Val
        115                 120
cccctgaggg gagctgtgtg tgctgacata atactaagcc cacaccaagg gtccactgac 439
tgaagtgcca tgggtaaaga aatagtcaca tgtccccact cttgatagct caggcaccgg 499
gctgtctcag cctgcagcac ctctccaaag gggcccaagg cggcttctca tgctgagctc 559
cccacagccc ctgccccaca gtgctgggtc tcagcacagg gctaccactt tccttgttga 619
gagaatgttg cagtggctag tttggctgga acaacaaaac ctcagctcca tgccctcaaa 679
cactaagttt tcagtgaaat aaaagcagga ggccgggccc atgcgcaggc atgcctgttc 739
tttggatctg gacactggag gatacattca taggaggcca gcaagggcca gcagtgcgtc 799
ccacttccct gaaacactgg agtctgaaag cagcttgtct accacacgcg tgcttgaaca 859
acactgctga ttctgacaca tgctcacgca cacacaccac acagacacac agatgagcac 919
acacagactt gctggaaggg tcaaaggatg cccgttctgg gcagcatgga gtgtctggaa 979
gccgctttga ctgttcagtg ctaggtgtca gagcctccaa gccagagtct ctgtgggaac 1039
ccttccagta ggagtgcgtt gggatgggca gcaggaccca gcaggtgctg tgctggttct 1099
tcatgaccag agaacggact gtgtttggtg tcgagcacgg gcatctggtc agatgtcag 1158
<210> 20
<211> 123
<212> PRT
<213> Rattus norvegicus

<400> 20

Leu Gly Phe Thr Ala Thr Asp Ser Thr Leu Glu Gly Glu Thr Ala Arg

1               5                   10                  15

Ala Tyr Met Pro Arg His Met Cys Lys Gly Thr Gln Arg Gln Ser Glu

            20                  25                  30

Ala Thr Met Ser Leu Cys Arg Asp Ser Gly Pro Arg Val Cys Ser Leu

        35                  40                  45

His Glu Arg Ala Gln Val Pro Arg Val His Leu Ser Ser Ile Gly Ala

    50                  55                  60

Gln Thr Arg Ala Gly His Lys Arg Leu Thr Pro Lys Glu Gln Ser Trp

65                  70                  75                  80

Cys His Gly Asp Arg Lys Ala Ala Gly Pro Arg Arg Glu Thr Leu Val

                85                  90                  95

Ser Ala Trp Glu Asn Thr Leu Gly Ser Pro Gln Leu Leu Ser Asp Cys

            100                 105                 110

Phe His Pro Ala Ala Lys Asp Asn Leu Arg Val

        115                 120

<210> 21

<211> 762

<212> DNA

<213> Rattus norvegius

<220>

<221> CDS

<222> (255)..(581)

<400> 21
   cgtgaccaat gctctgtgct gtgaatataa tagcaaaccc ttcccctggt tcagcatagt 60
   tagtcgtagg ggtttgctat gcctttcacg tacctctagg ctcttaagtc cctactgttt 120
   cacaagcttt aatcagagca gtagtggtca caggagaagg gctggcttcc agaagtagcc 180
   caggtcagcc actgtcagtc tctggaaagg gcatagtgtc tctgctcatt tacctggagc 240
   agcacgacag tcgc atg cac cta cag gaa gca gtc acc aca gag agc aga 290
   Met His Leu Gln Glu Ala Val Thr Thr Glu Ser Arg
   1               5                   10
   tgt gag tcc agc ccc aca ttt tca gac tgc agc cag agt cct tct cac    338
   Cys Glu Ser Ser Pro Thr Phe Ser Asp Cys Ser Gln Ser Pro Ser His
           15                  20                  25
   gtc aca cgc agc cag agc tac gct gct ctt cag ctt cca ccc ggt cct    386
   Val Thr Arg Ser Gln Ser Tyr Ala Ala Leu Gln Leu Pro Pro Gly Pro
       30                  35                  40
   ggg tcc tgc atg ctg cac ttg ctt gct atg ttg gcg cag agc cca gcc    434
   Gly Ser Cys Met Leu His Leu Leu Ala Met Leu Ala Gln Ser Pro Ala
   45                  50                  55                  60
   agt aca aag ccc tct cct ctc ctc ttt tct tct ctt cca gca ttc ccc    482
   Ser Thr Lys Pro Ser Pro Leu Leu Phe Ser Ser Leu Pro Ala Phe Pro
                   65                  70                  75
   tct cta aaa tgt caa cca aag aga gcc tcc cct ccc ccg cac ccc acc    530
   Ser Leu Lys Cys Gln Pro Lys Arg Ala Ser Pro Pro Pro His Pro Thr
               80                  85                  90
   cct gct ctc agc ctc ttt aga cag tct cct ctc cat ctc gtg gca tgt    578
   Pro Ala Leu Ser Leu Phe Arg Gln Ser Pro Leu His Leu Val Ala Cys
           95                  100                 105
   ctg tgaccctaga gaattcattt acagtgccat acggaaccct gtattttaca    631
   Leu
   cacacagcaa gcgatgttta ggtttattta tgatacttga tgctgtaaat gaaaataaat 691
   atggttcttt ataaaaaaaa aaaaaaaaaa aaaaaaaaaa aaaaaaaaaa aaaaaaaaaa 751
   aaaaaaaaaa a                                                    762
<210> 22
<211> 109
<212> PRT
<213> Rattus norvegicus
<400> 22

Met His Leu Gln Glu Ala Val Thr Thr Glu Ser Arg Cys Glu Ser Ser
1               5                   10                  15
Pro Thr Phe Ser Asp Cys Ser Gln Ser Pro Ser His Val Thr Arg Ser
            20                  25                  30
Gln Ser Tyr Ala Ala Leu Gln Leu Pro Pro Gly Pro Gly Ser Cys Met
        35                  40                  45
Leu His Leu Leu Ala Met Leu Ala Gln Ser Pro Ala Ser Thr Lys Pro
    50                  55                  60
Ser Pro Leu Leu Phe Ser Ser Leu Pro Ala Phe Pro Ser Leu Lys Cys
65                  70                  75                  80
Gln Pro Lys Arg Ala Ser Pro Pro Pro His Pro Thr Pro Ala Leu Ser
                85                  90                  95
Leu Phe Arg Gln Ser Pro Leu His Leu Val Ala Cys Leu
            100                 105
<210> 23
<211> 1312
<212> DNA
<213> Rattus norvegius
<220>
<221> CDS
<222> (164)..(1291)
<400> 23
   ctgattgact ggccccggta cccaggctat agagtggaac ctgcggcggt gtgaacgcgc 60
   gcgaactttg tgtcgccgcg gtctaacttc gactcggctt gctgcagctt caggcaggat 120
   cctggcttcc actatccccc ctccatccaa ccactcggga act atg gag gta gcc   175
                                                   Met Glu Val Ala
                                                   1
   gag gcc aac agc ccc act gag gag gag gaa gag gaa gag gag gaa gaa   223
   Glu Ala Asn Ser Pro Thr Glu Glu Glu Glu Glu Glu Glu Glu Glu Glu
   5                   10                  15                  20
   gga gag gag ccg att tca gag ccc agg cct cac act cgc tcc aat cct 271
   Gly Glu Glu Pro Ile Ser Glu Pro Arg Pro His Thr Arg Ser Asn Pro
   25 30 35
   gag ggg gct gag gac cgg gcc atc ggg gct cag gcc aat gtg ggt agc 319
   Glu Gly Ala Glu Asp Arg Ala Ile Gly Ala Gln Ala Asn Val Gly Ser
   40 45 50
   cgc agc gag ggc gaa ggt gag gca gcc act gca gat gat ggg gcg gcc 367
   Arg Ser Glu Gly Glu Gly Glu Ala Ala Thr Ala Asp Asp Gly Ala Ala
   55 60 65
   agt gtc ccg gga gct gtg ccc aag ccc tgg cag gta cca gca cca gca 415
   Ser Val Pro Gly Ala Val Pro Lys Pro Trp Gln Val Pro Ala Pro Ala
   70 75 80
   tct gag gtc cag att cga aca cca agg gtc aac tgt cca gag aaa gtg 463
   Ser Glu Val Gln Ile Arg Thr Pro Arg Val Asn Cys Pro Glu Lys Val
   85 90 95 100
   atc atc tgt ctg gat ctt tca gag gag atg tct gtg cca aag ctg gag 511
   Ile Ile Cys Leu Asp Leu Ser Glu Glu Met Ser Val Pro Lys Leu Glu
   105 110 115
   tcc ttt aat ggg tcc aga aca aac gcc ctg aat gtg tct cag aag atg 559
   Ser Phe Asn Gly Ser Arg Thr Asn Ala Leu Asn Val Ser Gln Lys Met
   120 125 130
   gtt gag atg ttt gtg cgc acg aag cac aag att gac aag agc cac gag 607
   Val Glu Met Phe Val Arg Thr Lys His Lys Ile Asp Lys Ser His Glu
   135 140 145
   ttt gcc ttg gtc gta gtg aac gac gac tct gcc tgg ttg tct ggc ctg 655
   Phe Ala Leu Val Val Val Asn Asp Asp Ser Ala Trp Leu Ser Gly Leu
   150 155 160
   acc tct gac cca cgt gaa ctc tgc agc tgc ctg tac gac cta gag acg 703
   Thr Ser Asp Pro Arg Glu Leu Cys Ser Cys Leu Tyr Asp Leu Glu Thr
   165 170 175 180
   gca tcc tgc tcc aca ttc aat ttg gaa ggc ctc ttc agc ctc atc cag 751
   Ala Ser Cys Ser Thr Phe Asn Leu Glu Gly Leu Phe Ser Leu Ile Gln
   185 190 195
   cag aag act gag ctg cca gtc aca gag aat gtg caa acc atc cca ccc 799
   Gln Lys Thr Glu Leu Pro Val Thr Glu Asn Val Gln Thr Ile Pro Pro
   200 205 210
   ccc tac gtc gtg cgc acc atc ctg gtc tac agc cgc cca ccc tgc cag 847
   Pro Tyr Val Val Arg Thr Ile Leu Val Tyr Ser Arg Pro Pro Cys Gln
   215 220 225
   ccc cag ttc tcc ttg act gag ccc atg aag aag atg ttc caa tgt ccc 895
   Pro Gln Phe Ser Leu Thr Glu Pro Met Lys Lys Met Phe Gln Cys Pro
   230 235 240
   tac ttc ttc ttc gac atc att tac atc cac agt ggc cct gag gaa aag 943
   Tyr Phe Phe Phe Asp Ile Ile Tyr Ile His Ser Gly Pro Glu Glu Lys
   245 250 255 260
   gaa gac gat atg agc tgg aag gac atg ttc gcc ttc atg ggc agt ctg 991
   Glu Asp Asp Met Ser Trp Lys Asp Met Phe Ala Phe Met Gly Ser Leu
   265 270 275
   gac acc aag ggc acc agc tac aag tat gca gta gca ctt gct ggc ccc 1039
   Asp Thr Lys Gly Thr Ser Tyr Lys Tyr Ala Val Ala Leu Ala Gly Pro
   280 285 290
   gcc ctg gag ctg cac aac tgc gtg gcc aag ttg ctg gcc cac ccg ctg 1087
   Ala Leu Glu Leu His Asn Cys Val Ala Lys Leu Leu Ala His Pro Leu
   295 300 305
   cag agg ccc tgc cag agc cac gcg agc tat agc ctg ctg gaa gag gac 1135
   Gln Arg Pro Cys Gln Ser His Ala Ser Tyr Ser Leu Leu Glu Glu Asp
   310 315 320
   gaa gag gcc ggt gag ggg gga ggc cac tgt gtg aca act cca cac cgt 1183
   Glu Glu Ala Gly Glu Gly Gly Gly His Cys Val Thr Thr Pro His Arg
   325 330 335 340
   ccc atg agg aga gaa ccc aca ccc tcg tca ctg gca cat gct caa tct 1231
   Pro Met Arg Arg Glu Pro Thr Pro Ser Ser Leu Ala His Ala Gln Ser
   345 350 355
   cac act gtc cac tgt aaa gtc att ctt cct ggg acc att ttt gtc atc 1279
   His Thr Val His Cys Lys Val Ile Leu Pro Gly Thr Ile Phe Val Ile
   360 365 370
   gga ata aaa gtc tgaggcccca gaaaaaaaaa a 1312
   Gly Ile Lys Val
   375
<210> 24
<211> 376
<212> PRT
<213> Rattus norvegicus
<400> 24
Met Glu Val Ala Glu Ala Asn Ser Pro Thr Glu Glu Glu Glu Glu Glu
1               5                   10                  15
Glu Glu Glu Glu Gly Glu Glu Pro Ile Ser Glu Pro Arg Pro His Thr
            20                  25                  30
Arg Ser Asn Pro Glu Gly Ala Glu Asp Arg Ala Ile Gly Ala Gln Ala
        35                  40                  45
Asn Val Gly Ser Arg Ser Glu Gly Glu Gly Glu Ala Ala Thr Ala Asp
    50                  55                  60
Asp Gly Ala Ala Ser Val Pro Gly Ala Val Pro Lys Pro Trp Gln Val
65                  70                  75                  80
Pro Ala Pro Ala Ser Glu Val Gln Ile Arg Thr Pro Arg Val Asn Cys
                85                  90                  95
Pro Glu Lys Val Ile Ile Cys Leu Asp Leu Ser Glu Glu Met Ser Val
            100                 105                 110
Pro Lys Leu Glu Ser Phe Asn Gly Ser Arg Thr Asn Ala Leu Asn Val
        115                 120                 125

Ser Gln Lys Met Val Glu Met Phe Val Arg Thr Lys His Lys Ile Asp
    130                 135                 140
Lys Ser His Glu Phe Ala Leu Val Val Val Asn Asp Asp Ser Ala Trp
145                 150                 155                 160
Leu Ser Gly Leu Thr Ser Asp Pro Arg Glu Leu Cys Ser Cys Leu Tyr
                165                 170                 175
Asp Leu Glu Thr Ala Ser Cys Ser Thr Phe Asn Leu Glu Gly Leu Phe
            180                 185                 190
Ser Leu Ile Gln Gln Lys Thr Glu Leu Pro Val Thr Glu Asn Val Gln
        195                 200                 205
Thr Ile Pro Pro Pro Tyr Val Val Arg Thr Ile Leu Val Tyr Ser Arg
    210                 215                 220
Pro Pro Cys Gln Pro Gln Phe Ser Leu Thr Glu Pro Met Lys Lys Met
225                 230                 235                 240
Phe Gln Cys Pro Tyr Phe Phe Phe Asp Ile Ile Tyr Ile His Ser Gly
                245                 250                 255
Pro Glu Glu Lys Glu Asp Asp Met Ser Trp Lys Asp Met Phe Ala Phe
            260                 265                 270
Met Gly Ser Leu Asp Thr Lys Gly Thr Ser Tyr Lys Tyr Ala Val Ala
        275                 280                 285
Leu Ala Gly Pro Ala Leu Glu Leu His Asn Cys Val Ala Lys Leu Leu
    290                 295                 300
Ala His Pro Leu Gln Arg Pro Cys Gln Ser His Ala Ser Tyr Ser Leu
305                 310                 315                 320
Leu Glu Glu Asp Glu Glu Ala Gly Glu Gly Gly Gly His Cys Val Thr
                325                 330                 335
Thr Pro His Arg Pro Met Arg Arg Glu Pro Thr Pro Ser Ser Leu Ala
            340                 345                 350
His Ala Gln Ser His Thr Val His Cys Lys Val Ile Leu Pro Gly Thr
        355                 360                 365

Ile Phe Val Ile Gly Ile Lys Val
370                 375
<210> 25
<211> 1670
<212> DNA
<213> Rattus norvegius
<220>
<221> CDS
<222> (19)..(1557)
<400> 25
gcgggcgggc gggcggac atg gcg gcc aac atg tac cgg gtc gga gat tat     51
                    Met Ala Ala Asn Met Tyr Arg Val Gly Asp Tyr
                    1               5                   10
gtt tac ttt gag aat tcg tcc agc aac cca tac cta atc aga agg ata     99
Val Tyr Phe Glu Asn Ser Ser Ser Asn Pro Tyr Leu Ile Arg Arg Ile
            15                  20                  25
gag gaa ctc aac aag act gca agc ggc aat gtg gaa gcc aaa gta gtc     147
Glu Glu Leu Asn Lys Thr Ala Ser Gly Asn Val Glu Ala Lys Val Val
        30                  35                  40
tgc ttt tat aga aga cgg gac atc tcc aac acg ctg ata atg ctt gcc     195
Cys Phe Tyr Arg Arg Arg Asp Ile Ser Asn Thr Leu Ile Met Leu Ala
    45                  50                  55
gat aag cat gct aaa gaa act gag gaa gaa tca gag acg acg gtt gag     243
Asp Lys His Ala Lys Glu Thr Glu Glu Glu Ser Glu Thr Thr Val Glu
60                  65                  70                  75
gct gac ttg acg gag aag cag aag cac cag ctg aaa cac agg gag ctc     291
Ala Asp Leu Thr Glu Lys Gln Lys His Gln Leu Lys His Arg Glu Leu
                80                  85                  90
ttt ctg tcc cgc cag tat gag tcc ctg cct gca aca cat atc agg ggg     339
Phe Leu Ser Arg Gln Tyr Glu Ser Leu Pro Ala Thr His Ile Arg Gly

            95                  100                 105

aag tgc agc gtg gcc ctg ctg aac gag aca gaa tca gtg ctg tca tac 387

Lys Cys Ser Val Ala Leu Leu Asn Glu Thr Glu Ser Val Leu Ser Tyr

        110                 115                 120

ctt gac aaa gag gat acc ttc ttc tac tca ttg gtg tat gac cct tcc 435

Leu Asp Lys Glu Asp Thr Phe Phe Tyr Ser Leu Val Tyr Asp Pro Ser

    125                 130                 135

gtg aaa aca tta ttg gct gac aaa ggt gaa atc aga gtg ggc cca aag 483

Val Lys Thr Leu Leu Ala Asp Lys Gly Glu Ile Arg Val Gly Pro Lys

140                 145                 150                 155

tac caa gcc gac att cca gac gtg ctg ccg gaa ggc gac tca gat gag 531

Tyr Gln Ala Asp Ile Pro Asp Val Leu Pro Glu Gly Asp Ser Asp Glu

                160                 165                 170

agg gaa caa tca aaa ttg gaa gtt aag gtt tgg gac ccc aat agt ccg 579

Arg Glu Gln Ser Lys Leu Glu Val Lys Val Trp Asp Pro Asn Ser Pro

            175                 180                 185

ctt acg gat cga cag att gac cag ttt tta gtt gta gcc cgt gcc gtg 627

Leu Thr Asp Arg Gln Ile Asp Gln Phe Leu Val Val Ala Arg Ala Val

        190                 195                 200

gga aca ttt gcc cgt gcc ctg gat tgc agc agc tct gtg agg cag ccc 675

Gly Thr Phe Ala Arg Ala Leu Asp Cys Ser Ser Ser Val Arg Gln Pro

    205                 210                 215

agc ctg cat atg agc gcg gct gcg gcc tcc cga gac atc acc ttg ttc 723

Ser Leu His Met Ser Ala Ala Ala Ala Ser Arg Asp Ile Thr Leu Phe

220                 225                 230                 235

cat gcc atg gac acg ctg tat agg cac ggc tat gac ctc agc agt gcc 771

His Ala Met Asp Thr Leu Tyr Arg His Gly Tyr Asp Leu Ser Ser Ala

                240                 245                 250

atc agt gtg ctg gtg cca ctc gga ggg ccg gtc ctg tgc agg gac gag 819

Ile Ser Val Leu Val Pro Leu Gly Gly Pro Val Leu Cys Arg Asp Glu

            255                 260                 265
atg gag gag tgg tct gcc tct gaa gcc agc tta ttc gaa gaa gca ctg 867
Met Glu Glu Trp Ser Ala Ser Glu Ala Ser Leu Phe Glu Glu Ala Leu
        270                 275                 280
gaa aaa tat ggc aaa gat ttc aat gac atc cgt cag gac ttt ctc cca 915
Glu Lys Tyr Gly Lys Asp Phe Asn Asp Ile Arg Gln Asp Phe Leu Pro
    285                 290                 295
tgg aag tcc ttg act agc atc att gaa tat tat tac atg tgg aaa act 963
Trp Lys Ser Leu Thr Ser Ile Ile Glu Tyr Tyr Tyr Met Trp Lys Thr
300                 305                 310                 315
act gac aga tac gtt caa cag aag cgc cta aaa gct gcg gaa gcc gag 1011
Thr Asp Arg Tyr Val Gln Gln Lys Arg Leu Lys Ala Ala Glu Ala Glu
                320                 325                 330
agc aaa ctg aaa caa gtg tac atc cca act tac aaa cca aat ccc aac 1059
Ser Lys Leu Lys Gln Val Tyr Ile Pro Thr Tyr Lys Pro Asn Pro Asn
            335                 340                 345
caa atc tcc agc agc aac ggc aag gct ggc act gtg aat gga gct gtg 1107
Gln Ile Ser Ser Ser Asn Gly Lys Ala Gly Thr Val Asn Gly Ala Val
        350                 355                 360
ggg acc ccg ttc cag ccc cag agc gca ctc cta gga cga gcc tgt gag 1155
Gly Thr Pro Phe Gln Pro Gln Ser Ala Leu Leu Gly Arg Ala Cys Glu
    365                 370                 375
agc tgc tat gcc aca cag tct cac cag tgg tat tcc tgg ggc cca cct 1203
Ser Cys Tyr Ala Thr Gln Ser His Gln Trp Tyr Ser Trp Gly Pro Pro
380                 385                 390                 395
aat atg cag tgt aga ctc tgt gcg acc tgc tgg ctg tat tgg aaa aag 1251
Asn Met Gln Cys Arg Leu Cys Ala Thr Cys Trp Leu Tyr Trp Lys Lys
                400                 405                 410
tac gga ggt ctg aaa atg ccc acg cag acg gac gag gag aag gct ccc 1299
Tyr Gly Gly Leu Lys Met Pro Thr Gln Thr Asp Glu Glu Lys Ala Pro

            415                 420                 425
agc cct gcc gca gag gac ccg cgc gtg aga agc cac ctg tcc cgg cag 1347
Ser Pro Ala Ala Glu Asp Pro Arg Val Arg Ser His Leu Ser Arg Gln
        430                 435                 440
gcc ttg cag ggc atg ccg gtc cgg aac acc ggg agc ccc aag tcg gcc 1395
Ala Leu Gln Gly Met Pro Val Arg Asn Thr Gly Ser Pro Lys Ser Ala
    445                 450                 455
gtg aag acc cgc caa gct ttc ttc ctc cat act acg tat ttc aca aaa 1443
Val Lys Thr Arg Gln Ala Phe Phe Leu His Thr Thr Tyr Phe Thr Lys
460                 465                 470                 475
att gct cgt cag gtc tgc aaa aac acc ctg cgg ctg cgg cag gca gcg 1491
Ile Ala Arg Gln Val Cys Lys Asn Thr Leu Arg Leu Arg Gln Ala Ala
                480                 485                 490
aga cgg ccg ttt gtt gct att aac tat gct gcc att agg gca gaa tgt 1539
Arg Arg Pro Phe Val Ala Ile Asn Tyr Ala Ala Ile Arg Ala Glu Cys
            495                 500                 505
aag acg ctt ttc aat tct taaccttaca cgttccgctc ctcgccatcc 1587
Lys Thr Leu Phe Asn Ser
        510
tctctctctc cctcgctctc tctttttgtt tgtttgtttg caataaacat aagttcttgt 1647
gtaaaaaaaa aaaaaaaaaa aaa 1670
<210> 26
<211> 513
<212> PRT
<213> Rattus norvegicus
<400> 26
Met Ala Ala Asn Met Tyr Arg Val Gly Asp Tyr Val Tyr Phe Glu Asn
1               5                   10                  15
Ser Ser Ser Asn Pro Tyr Leu Ile Arg Arg Ile Glu Glu Leu Asn Lys
            20                  25                  30

Thr Ala Ser Gly Asn Val Glu Ala Lys Val Val Cys Phe Tyr Arg Arg
        35                  40                  45
Arg Asp Ile Ser Asn Thr Leu Ile Met Leu Ala Asp Lys His Ala Lys
    50                  55                  60
Glu Thr Glu Glu Glu Ser Glu Thr Thr Val Glu Ala Asp Leu Thr Glu
65                  70                  75                  80
Lys Gln Lys His Gln Leu Lys His Arg Glu Leu Phe Leu Ser Arg Gln
                85                  90                  95
Tyr Glu Ser Leu Pro Ala Thr His Ile Arg Gly Lys Cys Ser Val Ala
            100                 105                 110
Leu Leu Asn Glu Thr Glu Ser Val Leu Ser Tyr Leu Asp Lys Glu Asp
        115                 120                 125
Thr Phe Phe Tyr Ser Leu Val Tyr Asp Pro Ser Val Lys Thr Leu Leu
    130                 135                 140
Ala Asp Lys Gly Glu Ile Arg Val Gly Pro Lys Tyr Gln Ala Asp Ile
145                 150                 155                 160
Pro Asp Val Leu Pro Glu Gly Asp Ser Asp Glu Arg Glu Gln Ser Lys
                165                 170                 175
Leu Glu Val Lys Val Trp Asp Pro Asn Ser Pro Leu Thr Asp Arg Gln
            180                 185                 190
Ile Asp Gln Phe Leu Val Val Ala Arg Ala Val Gly Thr Phe Ala Arg
        195                 200                 205
Ala Leu Asp Cys Ser Ser Ser Val Arg Gln Pro Ser Leu His Met Ser
    210                 215                 220
Ala Ala Ala Ala Ser Arg Asp Ile Thr Leu Phe His Ala Met Asp Thr
225                 230                 235                 240
Leu Tyr Arg His Gly Tyr Asp Leu Ser Ser Ala Ile Ser Val Leu Val
                245                 250                 255
Pro Leu Gly Gly Pro Val Leu Cys Arg Asp Glu Met Glu Glu Trp Ser
            260                 265                 270

Ala Ser Glu Ala Ser Leu Phe Glu Glu Ala Leu Glu Lys Tyr Gly Lys
        275                 280                 285
Asp Phe Asn Asp Ile Arg Gln Asp Phe Leu Pro Trp Lys Ser Leu Thr
    290                 295                 300
Ser Ile Ile Glu Tyr Tyr Tyr Met Trp Lys Thr Thr Asp Arg Tyr Val
305                 310                 315                 320
Gln Gln Lys Arg Leu Lys Ala Ala Glu Ala Glu Ser Lys Leu Lys Gln
                325                 330                 335
Val Tyr Ile Pro Thr Tyr Lys Pro Asn Pro Asn Gln Ile Ser Ser Ser
            340                 345                 350
Asn Gly Lys Ala Gly Thr Val Asn Gly Ala Val Gly Thr Pro Phe Gln
        355                 360                 365
Pro Gln Ser Ala Leu Leu Gly Arg Ala Cys Glu Ser Cys Tyr Ala Thr
    370                 375                 380
Gln Ser His Gln Trp Tyr Ser Trp Gly Pro Pro Asn Met Gln Cys Arg
385                 390                 395                 400
Leu Cys Ala Thr Cys Trp Leu Tyr Trp Lys Lys Tyr Gly Gly Leu Lys
                405                 410                 415
Met Pro Thr Gln Thr Asp Glu Glu Lys Ala Pro Ser Pro Ala Ala Glu
            420                 425                 430
Asp Pro Arg Val Arg Ser His Leu Ser Arg Gln Ala Leu Gln Gly Met
        435                 440                 445
Pro Val Arg Asn Thr Gly Ser Pro Lys Ser Ala Val Lys Thr Arg Gln
    450                 455                 460
Ala Phe Phe Leu His Thr Thr Tyr Phe Thr Lys Ile Ala Arg Gln Val
465                 470                 475                 480
Cys Lys Asn Thr Leu Arg Leu Arg Gln Ala Ala Arg Arg Pro Phe Val
                485                 490                 495
Ala Ile Asn Tyr Ala Ala Ile Arg Ala Glu Cys Lys Thr Leu Phe Asn
            500                 505                 510

Ser

<210> 27

<211> 994

<212> DNA

<213> Rattus norvegius

<220>

<221> CDS

<222> (271)..(744)

<400> 27

aaaaaagtcg ttttggatgg aagcatttca ctaattgctt tatttaagca tacaagggaa 60

aagtcttatc tgaccaatta ggttgaaggg ttttattacc tttaagaagc cattagctgg 120

gatgaaacgc tgcaagcact tgtgtcaaaa caaataaata atgctctctc aattagcaga 180

gaaagtggta catctgttaa attagacaca cattgcttca cacagtggcc cagccagcac 240

cccaatgtcc ccccaccccc aacaaacaaa atg aca gac agc aac ttt aag cta 294

Met Thr Asp Ser Asn Phe Lys Leu

1               5

gtc tta gga aag atc ggg gcc ccg aag acc aac agc tct ttc cct ctc 342

Val Leu Gly Lys Ile Gly Ala Pro Lys Thr Asn Ser Ser Phe Pro Leu

    10                  15                  20

tcc ttc cac ccc aac tct aca aga gcc agc agg gca gca gag gga ggt 390

Ser Phe His Pro Asn Ser Thr Arg Ala Ser Arg Ala Ala Glu Gly Gly

25                  30                  35                  40

ggt gtc cag agg ctg ggc ttc ctc ccc aag gtg ctt ccc agg cac agg 438

Gly Val Gln Arg Leu Gly Phe Leu Pro Lys Val Leu Pro Arg His Arg

                45                  50                  55

gag aga cag gtc tgt gtg act gtc agc agg tac gga gag tcc tct ttc 486

Glu Arg Gln Val Cys Val Thr Val Ser Arg Tyr Gly Glu Ser Ser Phe

            60                  65                  70

aaa agc aat tac cct gag gta tta ccg tac cag gac atg gac act aaa 534

Lys Ser Asn Tyr Pro Glu Val Leu Pro Tyr Gln Asp Met Asp Thr Lys

75                    80                    85
act tct caa agg ctc ctc atc ttc ccc aca aga aga ggc tca aca gaa    582
Thr Ser Gln Arg Leu Leu Ile Phe Pro Thr Arg Arg Gly Ser Thr Glu
    90                    95                    100
aag ggc agt ggg act cca tcg agt ggg cgc ata aag ctc ggg agc aag    630
Lys Gly Ser Gly Thr Pro Ser Ser Gly Arg Ile Lys Leu Gly Ser Lys
105                    110                    115                    120
gta cag gcc gag aat cca tgc ctt cag gac acc cac cag ccc aca ccg    678
Val Gln Ala Glu Asn Pro Cys Leu Gln Asp Thr His Gln Pro Thr Pro
                125                    130                    135
cct tca ggc aga agg gga cag tcc ctc tcc cca cag gtc ctt ggg aca    726
Pro Ser Gly Arg Arg Gly Gln Ser Leu Ser Pro Gln Val Leu Gly Thr
            140                    145                    150
att tct aac cag gtg agt tagaggaaaa tcaaccccca cctctccaaa          774
Ile Ser Asn Gln Val Ser
        155
aaaatctatt cagggaaata aattataaat aaatagtgct ccttttctta aaaggtcact 834
ttcttcaagg ctttcacaac tcctcagcat gatctccaca gattgcttgg agcaagtgcc 894
ctaatttggc cagtccgccc aggagcagcc taacctcaga ggctgagagg aggctgccga 954
gtctcctccc ccagttctga agtggtaggg tgggtgccgc                       994
<210> 28
<211> 158
<212> PRT
<213> Rattus norvegicus
<400> 28
Met Thr Asp Ser Asn Phe Lys Leu Val Leu Gly Lys Ile Gly Ala Pro
1               5                   10                  15
Lys Thr Asn Ser Ser Phe Pro Leu Ser Phe His Pro Asn Ser Thr Arg
            20                  25                  30
Ala Ser Arg Ala Ala Glu Gly Gly Gly Val Gln Arg Leu Gly Phe Leu

        35                  40                  45
Pro Lys Val Leu Pro Arg His Arg Glu Arg Gln Val Cys Val Thr Val
    50                  55                  60
Ser Arg Tyr Gly Glu Ser Ser Phe Lys Ser Asn Tyr Pro Glu Val Leu
65                  70                  75                  80
Pro Tyr Gln Asp Met Asp Thr Lys Thr Ser Gln Arg Leu Leu Ile Phe
                85                  90                  95
Pro Thr Arg Arg Gly Ser Thr Glu Lys Gly Ser Gly Thr Pro Ser Ser
            100                 105                 110
Gly Arg Ile Lys Leu Gly Ser Lys Val Gln Ala Glu Asn Pro Cys Leu
        115                 120                 125
Gln Asp Thr His Gln Pro Thr Pro Pro Ser Gly Arg Arg Gly Gln Ser
    130                 135                 140
Leu Ser Pro Gln Val Leu Gly Thr Ile Ser Asn Gln Val Ser
145                 150                 155
<210> 29
<211> 716
<212> DNA
<213> Rattus norvegius
<400> 29
   ttccagcaga cactcttttg ccccgggggc ctgcggctgg tgggcagcac gtctgacaac 60
   gggacactgc cctggcagcc agtcaccctg ccagaccctg aggagtttct gacagacagg 120
   cttgtgaggg agtatgtgag gcaggtactc aaggggctgg gcaaggggct ggtgctgtgg 180
   cgggcagggc agtgcctctg ggcccagcgc ctaggccact cgcattcctt ctgggccctg 240
   ggtgaggagc tgcttccaga cagtgggaga gggcctgatg gagaggtccc caaggacaag 300
   aacggagtcg tgttcgacct caggcccttt gtggcagatc tcattgcctt catggaagga 360
   agcagacatt ccccacgata cactctgtgg ttctgtgtgg gggaatcgtg gccccaggac 420
   cagccgtggg tcaagaggct tgtgatggtc aaggttgttc ctacatgtct taaggagctg 480
   ttagagatgg cccgggaagg gggagcctca tcactgaaaa ccgtggactt gcacatctcc 540
   aacagccagc cgatctccct tacctctgac cagtacaagg cctgcctcca ggacttggtg 600
   gaagacatgg acttccaggc cactggagaa acctgagccc agctcagctg ctccaataaa 660
   gcaatttatg ccaccatcac aaaaaaaaaa aaaaaaaaaa aaaaaaaaaa aaaaaa 716
<210> 30
<211> 787
<212> DNA
<213> Rattus norvegius
<220>
<221> CDS
<222> (423)..(704)
<400> 30
   ggcaaaagca gtctgtctga tcaccagagt gggagttcga ggtgactgcc ttctaccgag 60
   gccgccaggt cttccagcag acactctttt gccccggggg cctgcggctg gtgggcagca 120
   cgtctgacaa cgggacactg ccctggcagc cagtcaccct gccagaccct gaggagtttc 180
   tgacagacag gcttgtgagg gagtatgtga ggcaggtact caaggggctg ggcaaggggc 240
   tggtgctgtg gcgggcaggg cagtgcctct gggcccagcg cctaggccac tcgcattcct 300
   tctgggccct gggtgaggag ctgcttccag acagtgggag agggcctgat ggagaggtcc 360
   ccaaggacaa gaacggagtc gtgttcgacc tcaggccctt tgtggcagat ctcattgcct 420
   tc atg gaa gga agc aga cat tcc cca cga tac act ctg tgg ttc tgt 467
   Met Glu Gly Ser Arg His Ser Pro Arg Tyr Thr Leu Trp Phe Cys
   1 5 10 15
   gtg ggg gaa tcg tgg ccc cag gac cag ccg tgg gtc aag agg ctt gtg 515
   Val Gly Glu Ser Trp Pro Gln Asp Gln Pro Trp Val Lys Arg Leu Val
   20 25 30
   atg gtc aag gtt gtt cct aca tgt ctt aag gag ctg tta gag atg gcc 563
   Met Val Lys Val Val Pro Thr Cys Leu Lys Glu Leu Leu Glu Met Ala
   35 40 45
   cgg gaa ggg gga gcc tca tca ctg aaa acc gtg gac ttg cac atc tcc 611
   Arg Glu Gly Gly Ala Ser Ser Leu Lys Thr Val Asp Leu His Ile Ser
   50 55 60
   aac agc cag ccg atc tcc ctt acc tct gac cag tac aag gcc tgc ctc 659
   Asn Ser Gln Pro Ile Ser Leu Thr Ser Asp Gln Tyr Lys Ala Cys Leu
   65 70 75
   cag gac ttg gtg gaa gac atg gac ttc cag gcc act gga gaa acc tgagcc710
   Gln Asp Leu Val Glu Asp Met Asp Phe Gln Ala Thr Gly Glu Thr
   80 85 90
   cagctcagct gctccaataa agcaatttat gccaccatca caaaaaaaaa aaaaaaaaaa 770
   aaaaaaaaaa aaaaaaa 787
<210> 31
<211> 94
<212> PRT
<213> Rattus norvegicus
<400> 31
Met Glu Gly Ser Arg His Ser Pro Arg Tyr Thr Leu Trp Phe Cys Val
1               5                   10                  15
Gly Glu Ser Trp Pro Gln Asp Gln Pro Trp Val Lys Arg Leu Val Met
            20                  25                  30
Val Lys Val Val Pro Thr Cys Leu Lys Glu Leu Leu Glu Met Ala Arg
        35                  40                  45
Glu Gly Gly Ala Ser Ser Leu Lys Thr Val Asp Leu His Ile Ser Asn
    50                  55                  60
Ser Gln Pro Ile Ser Leu Thr Ser Asp Gln Tyr Lys Ala Cys Leu Gln
65                  70                  75                  80
Asp Leu Val Glu Asp Met Asp Phe Gln Ala Thr Gly Glu Thr
                85                  90
<210> 32
<211> 1570
<212> DNA
<213> Rattus norvegius
<400> 32
ctgacttgaa catatggtag gtgtgcctct gtaagaatcc aggctcattc tgagttgatg 60

aaaagtctta tttggacata tgcagggttt ggctccctga acactcttgg ggggggggtc 120
atctgacaca gtaccattta gtgactttcc caagataagc atgagttgtg tccttgagtg 180
aaacattggc catgcatggc tgtsgtgggc cattgctttt gctgctttag agcaccacgg 240
acctggctgg tgctgagctg gtccctacag ctaatgctcg gtaaactgtc accttctggc 300
ctcctcatgg caccgggtcc ttcgtcttca tgtgcatgca tgacttgcag tagagcctac 360
tcactgcatg ggagtccctg gctagagtct gctgcctggc ggcttgcagc gacggctcag 420
tcaaggctga aaggtcttts gccacacagt gaaaagtgaa ccatagatgg agcttgctga 480
ccccactgac actacctgtg tttggtttgc tgggctcttg gtgctgggag tgcagcaagc 540
acaggaggta ccctcgggaa ggcggaggcc ttgcctttcc ggactcacct aggtcttgtc 600
tttcaacttg ttgtagaagt tacaaattat tctcaaagat gtctgtctca tcatgaggga 660
aaaggaactt tcttttgttc acattcagga ctttgagggt atagtgccat aaaatgtagc 720
aaagggcccc gtgggccaga tcagtgttac attgattctg aggttacagt gtctcccacc 780
aaacatctgc tgtaggccat gcggtgtatt tgaagagggc tttgggagaa ggaagctttc 840
ctcttgcttt aaaccagtat gtgcatcaca tgcttacggc agacatttgc taccagtgga 900
gttctgtgga gcctggcttt gggcagctca ttcttgagga ggtggcaaaa cttggggaag 960
ttaggtgtga gtgttgtcaa ggcctgaagc cctatgatgc actgtggccc tagaaaagga 1020
agatgcagcc tctgctgacc actgtgtcct ctcagacttg gccagctgca ggagcccaac 1080
ctgatgttcc ctccttatcc tctgtgtagt cattccttcc gccccacaaa gggagcgcaa 1140
acatttctca ctgtgctgct gatgctctga cctggtgtgt ctgacagata catcaggctt 1200
tgcagggcaa aggtgattca cctaagtgtt cacacacagc aacactgaat cctacagacc 1260
aaaacccact tgtcagcagg agccgtggtc caggcccagc acctgtgtct cctaaccgag 1320
gccagctgtg ttgggtgaag agagccatgc caaggtccca ggctgcttta gtccatgtgc 1380
cctacccatt tggggacaga tggtttttct tgtaaacttg tggacttttg aaacctgttg 1440
actaaacagt aattaattta tatttgtgaa aaatgccact gtcctggtga tttctgatgt 1500
aaataatgtt gtttatatag tatgtattaa attttcctac cattgtaaaa aaaaaaaaaa 1560
aaaaaaaaaa 1570
<210> 33
<211> 1089
<212> DNA
<213> Rattus norvegius

<220>

<221> CDS

<222> (97)..(1047)

<400> 33

ggcacgaggg gtcgcggtgg cagccgtgcg gtgcttggct ccctaagcta tccggtgcca 60

tccttgtcgc tgcggcgact cgcaacatct gacgcc atg acc gag caa atg acc 114

Met Thr Glu Gln Met Thr

1               5

ctt cgt ggg acc ctc aag ggc cat aat gga tgg gtt aca cag atc gcc 162

Leu Arg Gly Thr Leu Lys Gly His Asn Gly Trp Val Thr Gln Ile Ala

            10                  15                  20

acc act ccg cag ttc ccg gac atg atc ctg tcg gcg tct cga gac aag 210

Thr Thr Pro Gln Phe Pro Asp Met Ile Leu Ser Ala Ser Arg Asp Lys

        25                  30                  35

acc atc atc atg tgg aag ctg acc agg gat gag acc aac tac ggc ata 258

Thr Ile Ile Met Trp Lys Leu Thr Arg Asp Glu Thr Asn Tyr Gly Ile

    40                  45                  50

cca caa cgt gct ctt cga ggt cac tcc cac ttt gtt agc gat gtt gtc 306

Pro Gln Arg Ala Leu Arg Gly His Ser His Phe Val Ser Asp Val Val

55                  60                  65                  70

atc tcc tct gat ggc cag ttt gcc ctc tca ggc tcc tgg gat gga acc 354

Ile Ser Ser Asp Gly Gln Phe Ala Leu Ser Gly Ser Trp Asp Gly Thr

                75                  80                  85

cta cgc ctc tgg gat ctc aca acg ggc act acc acg aga cga ttt gtc 402

Leu Arg Leu Trp Asp Leu Thr Thr Gly Thr Thr Thr Arg Arg Phe Val

            90                  95                  100

ggc cac acc aag gat gtg ctg agc gtg gct ttc tcc tct gac aac cgg 450

Gly His Thr Lys Asp Val Leu Ser Val Ala Phe Ser Ser Asp Asn Arg

        105                 110                 115

cag att gtc tct ggg tcc cga gac aag acc att aag tta tgg aat act 498

Gln Ile Val Ser Gly Ser Arg Asp Lys Thr Ile Lys Leu Trp Asn Thr
    120                 125                 130
ctg ggt gtc tgc aag tac act gtc cag gat gag agt cat tca gaa tgg 546
Leu Gly Val Cys Lys Tyr Thr Val Gln Asp Glu Ser His Ser Glu Trp
135                 140                 145                 150
gtg tct tgt gtc cgc ttc tcc ccg aac agc agc aac cct atc atc gtc 594
Val Ser Cys Val Arg Phe Ser Pro Asn Ser Ser Asn Pro Ile Ile Val
                155                 160                 165
tcc tgc gga tgg gac aag ctg gtc aag gtg tgg aat ctg gct aac tgc 642
Ser Cys Gly Trp Asp Lys Leu Val Lys Val Trp Asn Leu Ala Asn Cys
            170                 175                 180
aag cta aag acc aac cac att ggc cac act ggc tat ctg aac aca gtg 690
Lys Leu Lys Thr Asn His Ile Gly His Thr Gly Tyr Leu Asn Thr Val
        185                 190                 195
act gtc tct cca gat gga tcc ctc tgt gct tct gga ggc aag gat ggc 738
Thr Val Ser Pro Asp Gly Ser Leu Cys Ala Ser Gly Gly Lys Asp Gly
    200                 205                 210
cag gct atg ctg tgg gat ctc aat gaa ggc aag cac ctt tac aca tta 786
Gln Ala Met Leu Trp Asp Leu Asn Glu Gly Lys His Leu Tyr Thr Leu
215                 220                 225                 230
gat ggt gga gac atc atc aat gcc ttg tgc ttc agc ccc aac cgc tac 834
Asp Gly Gly Asp Ile Ile Asn Ala Leu Cys Phe Ser Pro Asn Arg Tyr
                235                 240                 245
tgg ctc tgt gct gcc act ggc ccc agt atc aag atc tgg gac ttg gag 882
Trp Leu Cys Ala Ala Thr Gly Pro Ser Ile Lys Ile Trp Asp Leu Glu
            250                 255                 260
ggc aag atc atg gta gat gaa ctg aag caa gaa gtt atc agc acc agc 930
Gly Lys Ile Met Val Asp Glu Leu Lys Gln Glu Val Ile Ser Thr Ser
        265                 270                 275
agc aag gca gag cca ccc cag tgt acc tct ttg gct tgg tct gct gat 978

Ser Lys Ala Glu Pro Pro Gln Cys Thr Ser Leu Ala Trp Ser Ala Asp
    280                 285                 290
ggc cag act ctg ttt gct ggc tat acc gac aac ttg gtg cgt gta tgg     1026
Gly Gln Thr Leu Phe Ala Gly Tyr Thr Asp Asn Leu Val Arg Val Trp
295                 300                 305                 310
cag gtg act att ggt acc cgc taaaagttta tgacagactc ttagaaataa     1077
Gln Val Thr Ile Gly Thr Arg
                315
actggctttc tg                                                       1089
<210> 34
<211> 317
<212> PRT
<213> Rattus norvegicus
<400> 34
Met Thr Glu Gln Met Thr Leu Arg Gly Thr Leu Lys Gly His Asn Gly
  1               5                  10                  15
Trp Val Thr Gln Ile Ala Thr Thr Pro Gln Phe Pro Asp Met Ile Leu
             20                  25                  30
Ser Ala Ser Arg Asp Lys Thr Ile Ile Met Trp Lys Leu Thr Arg Asp
         35                  40                  45
Glu Thr Asn Tyr Gly Ile Pro Gln Arg Ala Leu Arg Gly His Ser His
     50                  55                  60
Phe Val Ser Asp Val Val Ile Ser Ser Asp Gly Gln Phe Ala Leu Ser
 65                  70                  75                  80
Gly Ser Trp Asp Gly Thr Leu Arg Leu Trp Asp Leu Thr Thr Gly Thr
                 85                  90                  95
Thr Thr Arg Arg Phe Val Gly His Thr Lys Asp Val Leu Ser Val Ala
            100                 105                 110
Phe Ser Ser Asp Asn Arg Gln Ile Val Ser Gly Ser Arg Asp Lys Thr
        115                 120                 125

Ile Lys Leu Trp Asn Thr Leu Gly Val Cys Lys Tyr Thr Val Gln Asp
    130                 135                 140
Glu Ser His Ser Glu Trp Val Ser Cys Val Arg Phe Ser Pro Asn Ser
145                 150                 155                 160
Ser Asn Pro Ile Ile Val Ser Cys Gly Trp Asp Lys Leu Val Lys Val
                165                 170                 175
Trp Asn Leu Ala Asn Cys Lys Leu Lys Thr Asn His Ile Gly His Thr
            180                 185                 190
Gly Tyr Leu Asn Thr Val Thr Val Ser Pro Asp Gly Ser Leu Cys Ala
        195                 200                 205
Ser Gly Gly Lys Asp Gly Gln Ala Met Leu Trp Asp Leu Asn Glu Gly
    210                 215                 220
Lys His Leu Tyr Thr Leu Asp Gly Gly Asp Ile Ile Asn Ala Leu Cys
225                 230                 235                 240
Phe Ser Pro Asn Arg Tyr Trp Leu Cys Ala Ala Thr Gly Pro Ser Ile
                245                 250                 255
Lys Ile Trp Asp Leu Glu Gly Lys Ile Met Val Asp Glu Leu Lys Gln
            260                 265                 270
Glu Val Ile Ser Thr Ser Ser Lys Ala Glu Pro Pro Gln Cys Thr Ser
        275                 280                 285
Leu Ala Trp Ser Ala Asp Gly Gln Thr Leu Phe Ala Gly Tyr Thr Asp
    290                 295                 300
Asn Leu Val Arg Val Trp Gln Val Thr Ile Gly Thr Arg
305                 310                 315
<210> 35
<211> 1317
<212> DNA
<213> Rattus norvegius
<220>
<221> CDS

<222> (2)..(1256)

<400> 35

g atg cag acc ctg gtg cta ctc ctc tgg act gga gcc ctg ctt ggg cac 49
Met Gln Thr Leu Val Leu Leu Leu Trp Thr Gly Ala Leu Leu Gly His
1               5                   10                  15

ggc agc agc cag aat gtc cct gac agc tct cag gat tcc cca gcc cct 97
Gly Ser Ser Gln Asn Val Pro Asp Ser Ser Gln Asp Ser Pro Ala Pro
            20                  25                  30

gac agc acc ggg gag ccc gta gtg gag gag gat gac ccc ttc ttc aag 145
Asp Ser Thr Gly Glu Pro Val Val Glu Glu Asp Asp Pro Phe Phe Lys
        35                  40                  45

gcc ccc gtg aac aag ttg gca gca gct gtt tcc aac ttc ggc tac gat 193
Ala Pro Val Asn Lys Leu Ala Ala Ala Val Ser Asn Phe Gly Tyr Asp
    50                  55                  60

ctc tac cgc ctg aga tcc ggt gct gtc tca acc ggc aac att ctg ctg 241
Leu Tyr Arg Leu Arg Ser Gly Ala Val Ser Thr Gly Asn Ile Leu Leu
65                  70                  75                  80

tct cct ctc agc gtg gcc acg gcc ctc tcg gcc ctt tcc ctg gga gct 289
Ser Pro Leu Ser Val Ala Thr Ala Leu Ser Ala Leu Ser Leu Gly Ala
                85                  90                  95

gaa cag cga aca gag tct gtc att cac cgg gct ctc tac tac gac ttg 337
Glu Gln Arg Thr Glu Ser Val Ile His Arg Ala Leu Tyr Tyr Asp Leu
            100                 105                 110

atc aac aat cct gac atc cac agc acc tac aag gag ctc ctt gcc tct 385
Ile Asn Asn Pro Asp Ile His Ser Thr Tyr Lys Glu Leu Leu Ala Ser
        115                 120                 125

gtt act gcc cct gag aag aac ttc aag agt gcc tcc aga att gtg ttt 433
Val Thr Ala Pro Glu Lys Asn Phe Lys Ser Ala Ser Arg Ile Val Phe
    130                 135                 140

gag agg aaa ctt cga gta aaa tcc agc ttt gtt gct cct ctg gag aag 481

Glu Arg Lys Leu Arg Val Lys Ser Ser Phe Val Ala Pro Leu Glu Lys
145                 150                 155                 160
tca tat ggg acc agg ccc cga atc ctc act ggc aac cct cgc ata gac 529
Ser Tyr Gly Thr Arg Pro Arg Ile Leu Thr Gly Asn Pro Arg Ile Asp
                165                 170                 175
ctt cag gag att aac aac tgg gtg cag gcc cag atg aaa ggg aaa att 577
Leu Gln Glu Ile Asn Asn Trp Val Gln Ala Gln Met Lys Gly Lys Ile
            180                 185                 190
gcc cgg tct aca agg gaa atg ccc agt gcc ctc agc atc ctc ctc ctt 625
Ala Arg Ser Thr Arg Glu Met Pro Ser Ala Leu Ser Ile Leu Leu Leu
        195                 200                 205
ggc gtg gct tac ttc aag ggg cag tgg gca acc aag ttt gac tcg aga 673
Gly Val Ala Tyr Phe Lys Gly Gln Trp Ala Thr Lys Phe Asp Ser Arg
    210                 215                 220
aag acg acc ctc cag gat ttt cac ttg gac gag gac agg act gtg aga 721
Lys Thr Thr Leu Gln Asp Phe His Leu Asp Glu Asp Arg Thr Val Arg
225                 230                 235                 240
gtc ccc atg atg tca gac cct aag gcc atc tta cga tat ggc ttg gac 769
Val Pro Met Met Ser Asp Pro Lys Ala Ile Leu Arg Tyr Gly Leu Asp
                245                 250                 255
tct gat ctc aac tgc aag att gcc cag ctg cct ttg aca gga agc atg 817
Ser Asp Leu Asn Cys Lys Ile Ala Gln Leu Pro Leu Thr Gly Ser Met
            260                 265                 270
agt atc atc ttc ttc ctg ccc ctg acg gtg acc cag aac ttg acc atg 865
Ser Ile Ile Phe Phe Leu Pro Leu Thr Val Thr Gln Asn Leu Thr Met
        275                 280                 285
ata gag gag agc ctc acc tct gag ttc att cat gac att gac cga gaa 913
Ile Glu Glu Ser Leu Thr Ser Glu Phe Ile His Asp Ile Asp Arg Glu
    290                 295                 300
ctg aag act atc caa gct gtg ctg act gtc ccc aag ctg aag ctg agc 961

Leu Lys Thr Ile Gln Ala Val Leu Thr Val Pro Lys Leu Lys Leu Ser
305                 310                 315                 320
tat gaa ggc gac gtt acc aac tct ttg cag gac atg aag cta cag tcc     1009
Tyr Glu Gly Asp Val Thr Asn Ser Leu Gln Asp Met Lys Leu Gln Ser
                325                 330                 335
ttg ttt gag tcc cct gac ttc agc aag att acc ggc aaa cct gtg aag     1057
Leu Phe Glu Ser Pro Asp Phe Ser Lys Ile Thr Gly Lys Pro Val Lys
            340                 345                 350
ctc acc caa gtg gaa cac agg gca gct ttt gag tgg aat gag gag ggg     1105
Leu Thr Gln Val Glu His Arg Ala Ala Phe Glu Trp Asn Glu Glu Gly
        355                 360                 365
gca ggg acc agc tct aac cca gac ctc cag cct gtc cgc ctc acc ttc     1153
Ala Gly Thr Ser Ser Asn Pro Asp Leu Gln Pro Val Arg Leu Thr Phe
    370                 375                 380
ccg ctc gac tat cac ctt aac cga ccg ttc atc ttt gtt ctg agg gac     1201
Pro Leu Asp Tyr His Leu Asn Arg Pro Phe Ile Phe Val Leu Arg Asp
385                 390                 395                 400
acg gac acg ggg gcc ctc ctc ttc ata ggc aga atc ctg gac ccc agc     1249
Thr Asp Thr Gly Ala Leu Leu Phe Ile Gly Arg Ile Leu Asp Pro Ser
                405                 410                 415
agc act taattgtccc agtgcgctac agaaaacccc agagggaggg aggactgatt     1305
Ser Thr
acacattcca gg                                                       1317
<210> 36
<211> 418
<212> PRT
<213> Rattus norvegicus
<400> 36
Met Gln Thr Leu Val Leu Leu Leu Trp Thr Gly Ala Leu Leu Gly His
  1               5                  10                  15

Gly Ser Ser Gln Asn Val Pro Asp Ser Ser Gln Asp Ser Pro Ala Pro
            20                  25                  30
Asp Ser Thr Gly Glu Pro Val Val Glu Glu Asp Asp Pro Phe Phe Lys
        35                  40                  45
Ala Pro Val Asn Lys Leu Ala Ala Ala Val Ser Asn Phe Gly Tyr Asp
    50                  55                  60
Leu Tyr Arg Leu Arg Ser Gly Ala Val Ser Thr Gly Asn Ile Leu Leu
65                  70                  75                  80
Ser Pro Leu Ser Val Ala Thr Ala Leu Ser Ala Leu Ser Leu Gly Ala
                85                  90                  95
Glu Gln Arg Thr Glu Ser Val Ile His Arg Ala Leu Tyr Tyr Asp Leu
            100                 105                 110
Ile Asn Asn Pro Asp Ile His Ser Thr Tyr Lys Glu Leu Leu Ala Ser
        115                 120                 125
Val Thr Ala Pro Glu Lys Asn Phe Lys Ser Ala Ser Arg Ile Val Phe
    130                 135                 140
Glu Arg Lys Leu Arg Val Lys Ser Ser Phe Val Ala Pro Leu Glu Lys
145                 150                 155                 160
Ser Tyr Gly Thr Arg Pro Arg Ile Leu Thr Gly Asn Pro Arg Ile Asp
                165                 170                 175
Leu Gln Glu Ile Asn Asn Trp Val Gln Ala Gln Met Lys Gly Lys Ile
            180                 185                 190
Ala Arg Ser Thr Arg Glu Met Pro Ser Ala Leu Ser Ile Leu Leu Leu
        195                 200                 205
Gly Val Ala Tyr Phe Lys Gly Gln Trp Ala Thr Lys Phe Asp Ser Arg
    210                 215                 220
Lys Thr Thr Leu Gln Asp Phe His Leu Asp Glu Asp Arg Thr Val Arg
225                 230                 235                 240
Val Pro Met Met Ser Asp Pro Lys Ala Ile Leu Arg Tyr Gly Leu Asp
                245                 250                 255

Ser Asp Leu Asn Cys Lys Ile Ala Gln Leu Pro Leu Thr Gly Ser Met
            260                 265                 270
Ser Ile Ile Phe Phe Leu Pro Leu Thr Val Thr Gln Asn Leu Thr Met
        275                 280                 285
Ile Glu Glu Ser Leu Thr Ser Glu Phe Ile His Asp Ile Asp Arg Glu
    290                 295                 300
Leu Lys Thr Ile Gln Ala Val Leu Thr Val Pro Lys Leu Lys Leu Ser
305                 310                 315                 320
Tyr Glu Gly Asp Val Thr Asn Ser Leu Gln Asp Met Lys Leu Gln Ser
                325                 330                 335
Leu Phe Glu Ser Pro Asp Phe Ser Lys Ile Thr Gly Lys Pro Val Lys
            340                 345                 350
Leu Thr Gln Val Glu His Arg Ala Ala Phe Glu Trp Asn Glu Glu Gly
        355                 360                 365
Ala Gly Thr Ser Ser Asn Pro Asp Leu Gln Pro Val Arg Leu Thr Phe
    370                 375                 380
Pro Leu Asp Tyr His Leu Asn Arg Pro Phe Ile Phe Val Leu Arg Asp
385                 390                 395                 400
Thr Asp Thr Gly Ala Leu Leu Phe Ile Gly Arg Ile Leu Asp Pro Ser
                405                 410                 415
Ser Thr
<210> 37
<211> 1462
<212> DNA
<213> Rattus norvegius
<220>
<221> CDS
<222> (1)..(852)
<400> 37
ttt gct gtc ttt tcc att gct gtc tcc tca gat gga cga gaa gta cta 48

Phe Ala Val Phe Ser Ile Ala Val Ser Ser Asp Gly Arg Glu Val Leu
1               5                   10                  15
gga gga gcc aat gat ggc tgc ctt tat gtc ttt gac cgt gaa caa aac     96
Gly Gly Ala Asn Asp Gly Cys Leu Tyr Val Phe Asp Arg Glu Gln Asn
            20                  25                  30
cgg cgt act ctt cag att gag tct cat gag gat gat gtg aat gca gtg     144
Arg Arg Thr Leu Gln Ile Glu Ser His Glu Asp Asp Val Asn Ala Val
        35                  40                  45
gcc ttt gct gac ata agc tcc cag atc ctg ttc tct ggg gga gac gat     192
Ala Phe Ala Asp Ile Ser Ser Gln Ile Leu Phe Ser Gly Gly Asp Asp
    50                  55                  60
gcc atc tgc aaa gtg tgg gac cga cgc acc atg cgg gag gat gac ccc     240
Ala Ile Cys Lys Val Trp Asp Arg Arg Thr Met Arg Glu Asp Asp Pro
65                  70                  75                  80
aag cct gtg ggg gca ctg gct ggc cac cag gat ggc atc acc ttc att     288
Lys Pro Val Gly Ala Leu Ala Gly His Gln Asp Gly Ile Thr Phe Ile
                85                  90                  95
gac agc aag ggt gat gcc cgg tat ctc atc tcc aac tcc aaa gat cag     336
Asp Ser Lys Gly Asp Ala Arg Tyr Leu Ile Ser Asn Ser Lys Asp Gln
            100                 105                 110
acc att aag ctc tgg gat atc aga cgc ttt tcc agc cga gaa ggc atg     384
Thr Ile Lys Leu Trp Asp Ile Arg Arg Phe Ser Ser Arg Glu Gly Met
        115                 120                 125
gaa gcg tca cga ctg gct gcc aca cag cag aac tgg gac tat cgc tgg     432
Glu Ala Ser Arg Leu Ala Ala Thr Gln Gln Asn Trp Asp Tyr Arg Trp
    130                 135                 140
cag cag gtg ccc aag aaa gcc tgg aag aag ttg aag ctc cca ggt gac     480
Gln Gln Val Pro Lys Lys Ala Trp Lys Lys Leu Lys Leu Pro Gly Asp
145                 150                 155                 160
agc tcc ttg atg acc tac aga ggc cat gga gtg ctt cac act ctg atc     528

Ser Ser Leu Met Thr Tyr Arg Gly His Gly Val Leu His Thr Leu Ile
                165                 170                 175
cga tgc cga ttc tcc cca gcc cac agc acg ggc cag cag ttc atc tac 576
Arg Cys Arg Phe Ser Pro Ala His Ser Thr Gly Gln Gln Phe Ile Tyr
            180                 185                 190
agc ggc tgt tct act ggc aaa gtg gtt gta tat gac ctc tta agc ggc 624
Ser Gly Cys Ser Thr Gly Lys Val Val Val Tyr Asp Leu Leu Ser Gly
        195                 200                 205
cac att gtg aag aag ctg acc aat cac aag gcc tgt gtg cgt gat gtc 672
His Ile Val Lys Lys Leu Thr Asn His Lys Ala Cys Val Arg Asp Val
    210                 215                 220
agt tgg cat ccc ttt gaa gaa aag att gtc agc agt tcg tgg gat ggg 720
Ser Trp His Pro Phe Glu Glu Lys Ile Val Ser Ser Ser Trp Asp Gly
225                 230                 235                 240
agc cta cgc ctg tgg cag tac cgc caa gct gag tac ttc cag gat gac 768
Ser Leu Arg Leu Trp Gln Tyr Arg Gln Ala Glu Tyr Phe Gln Asp Asp
                245                 250                 255
atg acc gag tct gac agg aac aga gtc tgt tcc agt ggc cct gct ccg 816
Met Thr Glu Ser Asp Arg Asn Arg Val Cys Ser Ser Gly Pro Ala Pro
            260                 265                 270
gtg ccc tgc cca tct gtg gcc ttt tcc tca cct cag tagatctgac 862
Val Pro Cys Pro Ser Val Ala Phe Ser Ser Pro Gln
        275                 280
ctccagttct gtgtagggtg aattctcagc atattctctg cctcctcctt cctttcccca 922
tttggggagt aagtggaaga actgctgaca ctgggtagtg agagacagaa acccagagtc 982
tgggcccagg ctgagcctgg actacccgtc cccaaactgg gccaagtggt ttcctcatgc 1042
attcataccc agtctgcttg gatttctatc tctagccaga atgtggccgg acatccatta 1102
tctggggtgc agcttctgcc aacaagagtc ttgagtgttt taatcatgtt ctgatgttag 1162
atatggctca tagtgtagac acgaggtcta aatagaccca ttagcctttt gcccaggtct 1222
tcacaccaag agttagattg accaaccagg gcccagtata ctggcctttc tttctgagat 1282

ctttgaggga ggacagggtg ggcagggacg tgtttcctca gcaccctctg gggtaggaac 1342
tgtgtctgct ctcatacttg gcctttgaag tgaagagact ggtctgatgt gtgggtctca 1402
gaactccgca gggccctact tgccattgga tcctgtcttt gctggtggcc agcagcttca 1462
<210> 38
<211> 284
<212> PRT
<213> Rattus norvegicus
<400> 38
Phe Ala Val Phe Ser Ile Ala Val Ser Ser Asp Gly Arg Glu Val Leu
1               5                   10                  15
Gly Gly Ala Asn Asp Gly Cys Leu Tyr Val Phe Asp Arg Glu Gln Asn
            20                  25                  30
Arg Arg Thr Leu Gln Ile Glu Ser His Glu Asp Asp Val Asn Ala Val
        35                  40                  45
Ala Phe Ala Asp Ile Ser Ser Gln Ile Leu Phe Ser Gly Gly Asp Asp
    50                  55                  60
Ala Ile Cys Lys Val Trp Asp Arg Arg Thr Met Arg Glu Asp Asp Pro
65                  70                  75                  80
Lys Pro Val Gly Ala Leu Ala Gly His Gln Asp Gly Ile Thr Phe Ile
                85                  90                  95
Asp Ser Lys Gly Asp Ala Arg Tyr Leu Ile Ser Asn Ser Lys Asp Gln
            100                 105                 110
Thr Ile Lys Leu Trp Asp Ile Arg Arg Phe Ser Ser Arg Glu Gly Met
        115                 120                 125
Glu Ala Ser Arg Leu Ala Ala Thr Gln Gln Asn Trp Asp Tyr Arg Trp
    130                 135                 140
Gln Gln Val Pro Lys Lys Ala Trp Lys Lys Leu Lys Leu Pro Gly Asp
145                 150                 155                 160
Ser Ser Leu Met Thr Tyr Arg Gly His Gly Val Leu His Thr Leu Ile
                165                 170                 175

Arg Cys Arg Phe Ser Pro Ala His Ser Thr Gly Gln Gln Phe Ile Tyr
            180                 185                 190
Ser Gly Cys Ser Thr Gly Lys Val Val Val Tyr Asp Leu Leu Ser Gly
        195                 200                 205
His Ile Val Lys Lys Leu Thr Asn His Lys Ala Cys Val Arg Asp Val
    210                 215                 220
Ser Trp His Pro Phe Glu Glu Lys Ile Val Ser Ser Ser Trp Asp Gly
225                 230                 235                 240
Ser Leu Arg Leu Trp Gln Tyr Arg Gln Ala Glu Tyr Phe Gln Asp Asp
                245                 250                 255
Met Thr Glu Ser Asp Arg Asn Arg Val Cys Ser Ser Gly Pro Ala Pro
            260                 265                 270
Val Pro Cys Pro Ser Val Ala Phe Ser Ser Pro Gln
        275                 280
<210> 39
<211> 23
<212> DNA
<213> Artificial Sequence
<400> 39
aattaaccct cactaaaggg aac 23
<210> 40
<211> 22
<212> DNA
<213> Artificial Sequence
<400> 40
gtaatacgac tcactatagg gc 22
<210> 41
<211> 22
<212> DNA
<213> Artificial Sequence

<400> 41

tcacatactc cctcacaagc ct 22

<210> 42

<211> 17

<212> DNA

<213> Artificial Sequence

<400> 42

gtaaaacgac ggccagt 17